# EUROPÄISCHE PATENTSCHRIFT

(11) **EP 2 700 715 B1**
(45) Veröffentlichungstag und Bekanntmachung des Hinweises auf die Patenterteilung: **25.07.2018**
(21) Anmeldenummer: 12181028.7
(22) Anmeldetag: 20.08.2012
(51) Int. Cl.: C12P 13/12, C12N 15/70, C07K 14/245, C12N 1/20

(54) **Verfahren zur fermentativen Herstellung von L-Aminosäuren unter Verwendung von verbesserten Stämmen der Familie Enterobacteriaceae**
Method for manufacturing L-amino acids using improved strains of the enterobacteriaceae family by means of fermentation
Procédé de fabrication fermentative d'acides aminés L en utilisant des souches améliorées de la famille des enterobacteriaceae

(43) Veröffentlichungstag der Anmeldung: 26.02.2014
(73) Patentinhaber: Evonik Degussa GmbH, 45128 Essen (DE)
(72) Erfinder: Bathe, Brigitte, Dr., 33154 Salzkotten (DE); Molck, Stella, 33602 Bielefeld (DE); Priefert, Horst, Dr., 48346 Ostbevern (DE)

(56) Entgegenhaltungen:
- EP-A1- 2 479 279
- WO-A1-2005/085463
- WO-A1-2008/127240
- MELLIES J ET AL: "TWO DIFFERENT ESCHERICHIA COLI PROP PROMOTERS RESPOND TO OSMOTIC AND GROWTH PHASE SIGNALS", JOURNAL OF BACTERIOLOGY, AMERICAN SOCIETY FOR MICROBIOLOGY, WASHINGTON, DC; US, Bd. 177, Nr. 1, 1. Januar 1995 (1995-01-01), Seiten 144-151, XP000578578, ISSN: 0021-9193
- ELISE R. HONDORP ET AL: "Oxidative Stress Inactivates Cobalamin-Independent Methionine Synthase (MetE) in Escherichia coli", PROTEIN ENGINEERING FOR THERAPEUTICS, PART B, Bd. 290, Nr. 11, 1. Januar 2004 (2004-01-01), Seite 59, XP055050137, ISSN: 0076-6879, DOI: 10.1371/journal.pbio.0020336 ISBN: 978-0-12-396962-0
- OLD I G ET AL: "Regulation of methionine biosynthesis in the enterobacteriaceae", PROGRESS IN BIOPHYSICS AND MOLECULAR BIOLOGY, PERGAMON PRESS, OXFORD, GB, Bd. 56, Nr. 3, 1. Januar 1991 (1991-01-01) , Seiten 145-185, XP025215159, ISSN: 0079-6107, DOI: 10.1016/0079-6107(91)90012-H [gefunden am 1991-01-01]
- DATABASE WPI Week 200656 Thomson Scientific, London, GB; AN 2006-546018 XP002697400, -& KR 2005 0079338 A (CJ CORP) 10. August 2005 (2005-08-10)

## Beschreibung

Die vorliegende Erfindung betrifft ein Verfahren zur fermentativen Herstellung von L-Aminosäuren unter Verwendung von Mikroorganismen der Familie der Enterobacteriaceae, die ein abgeschwächtes *proP-Gen* enthalten, die für die Produktion geeigneten Mikroorganismen sowie Polynukleotide, die für Varianten des Transporters ProP kodieren.

Organisch-chemische Verbindungen, insbesondere schwefelhaltige L-Aminosäuren, haben eine große wirtschaftliche Bedeutung. L-Cystein wird als Lebensmittelzusatz, als Ausgangsstoff für pharmakologische Wirkstoffe (z.B. N-Acetylcystein) und für Kosmetika verwendet. Die Aminosäure L-Methionin spielt eine herausragende Rolle in der Tierernährung. Sie gehört zu den essentiellen Aminosäuren, die im Stoffwechsel der Wirbeltiere nicht durch Biosynthese hergestellt werden kann. Demzufolge muss bei der Tierzucht sichergestellt werden, dass die jeweilige Aminosäure in ausreichenden Mengen mit der Nahrung aufgenommen wird. Da zum Beispiel L-Methionin aber in klassischen Futtermittelpflanzen (wie Soja oder Getreide) insbesondere für Schweine und Geflügel häufig in zu geringen Mengen vorhanden ist, um eine optimale Tierernährung zu gewährleisten, ist es vorteilhaft, Methionin als Zusatzstoff dem Tierfutter beizumischen. D-Methionin kann von Wirbeltieren in biologisch aktives L-Methionin überführt werden. Daher wird dem Tierfutter meist ein Racemat aus D- und L-Methionin zugesetzt. L-Homocystein kann von Tieren durch Transmethylierung zu L-Methionin umgesetzt werden und dieses somit ersetzen.

Werden im Folgenden organisch-chemische Verbindungen erwähnt, sind damit eine oder mehrere Verbindungen ausgewählt aus der Gruppe der L-Aminosäuren, vorzugsweise schwefelhaltige L-Aminosäuren, insbesondere L-Methionin, L-Cystein, L-Cystin, L-Homocystein und L-Homocystin gemeint. Bevorzugt ist L-Methionin.

Im Stand der Technik werden Aminosäuren wie Methionin durch chemische Synthese hergestellt. Dabei wird zunächst Acrolein und Methylmercaptan zu 3-Methylthiopropionaldehyd umgesetzt, der wiederum mit Cyanid, Ammoniak und Kohlenmonoxid zum Hydantoin führt. Dieses kann letztendlich zum Razemat, einem äquimolaren Gemisch der beiden Stereoisomeren D-bzw. L-Methionin hydrolysiert werden. Da die biologisch aktive Form des Moleküls ausschließlich die L-Form repräsentiert, muss die im Futter enthaltene D-Form im Stoffwechsel erst durch Des- und Transaminierung in die aktive L-Form überführt werden.

Im Gegensatz zu Methionin werden die meisten anderen natürlichen, proteinogenen Aminosäuren wie zum Beispiel L-Threonin vorwiegend durch Fermentation von Mikroorganismen hergestellt. Dabei wird ausgenutzt, dass Mikroorganismen über entsprechende Biosynthesewege zur Synthese der natürlichen Aminosäuren verfügen. Zudem erreichen viele Fermentationsverfahren mit kostengünstigen Edukten wie Glucose und Mineralsalzen sehr günstige Herstellkosten und liefern zudem die biologisch aktive L-Form der jeweiligen Aminosäure.

Es ist bekannt, dass organisch-chemischen Verbindungen durch Fermentation von Stämmen der Enterobacteriaceae, insbesondere Escherichia coli (E. coli) und Serratia marcescens, hergestellt werden können. Wegen der großen Bedeutung wird ständig an der Verbesserung der Herstellverfahren gearbeitet. Verfahrensverbesserungen können fermentationstechnische Maßnahmen, wie z.B. Rühren und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

Biosynthesewege von Aminosäuren unterliegen in WildtypStämmen einer strengen metabolischen Kontrolle, die gewährleistet, dass die Aminosäuren nur zum Eigenbedarf der Zelle hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch gesteigerte Produktionsleistung über den Eigenbedarf hinaus (Überproduktion) für die Herstellung der gewünschten Aminosäure aufweisen.

Solche Aminosäure-überproduzierenden Mikroorganismen können durch klassische Mutations-/Selektionsverfahren und/oder durch moderne, gezielte, rekombinante Techniken ("metabolic engineering") erzeugt werden. Bei letzterem werden zunächst Gene oder Allele identifiziert, die durch ihre Veränderung, Aktivierung oder Inaktivierung eine Aminosäure-Überproduktion bewirken. Diese Gene/Allele werden dann durch molekularbiologische Techniken in einen Mikroorganismenstamm eingebracht oder inaktiviert, so dass eine optimale Überproduktion erzielt wird. Häufig führt jedoch erst die Kombination mehrerer, verschiedener Maßnahmen zu einer wirklich effizienten Produktion.

L-Methionin leitet sich zusammen mit Lysin und Threonin vom Aspartat ab. Schwefel wird in Form von L-Cystein (über Cystathionin als Zwischenprodukt) durch Transsulfurierung in L-Methionin eingebracht. Die CH₃-Gruppe des L-Methionins stammt aus dem C1-Stoffwechsel und sie wird von den Methionin-Synthasen MetE oder MetH auf L-Homocystein übertragen (Review: Greene RC (1996) in Neidhardt FC et al. (Hrsg.) "Escherichia coli and Salmonella", 2. Ausgabe, S. 542-560). Stämme und Verfahren zur fermentativen Produktion von L-Methionin wurden für *E. coli* z.B. in der WO2006/001616 oder WO2009/043803 beschrieben.

Hondrop et al. beschreiben das Wachstum von E. coli in einem Methionin enthaltenden Medium (Protein Engineering for Therapeutics, Part B, Bd. 290, Nr. 11, S. 59). In EP2479279A1 wird die fermentative Produktion schwefelhaltiger Aminosäuren beschrieben, wobei die verwendeten Mikroorganismen erhöhte oder abgeschwächte Aktivitäten von beteiligten Enzymen und/oder Transportern bzw. Methionin-Exportern aufweisen.

ProP ist der Protonen/kompatible Solute Symporter aus E. coli, und somit einer der unter hyperosmotischen Bedingungen aktiven Transporter in E. coli ((Grothe et al., Journal of Bacteriology 166, 253-259 (1986); Racher et al., Biochemistry 38, 1676-1684 (1999); Wood, Methods in Enzymology 428, 77-107 (2007)). Wenn die Osmolalität des die Zelle umgebenden Mediums steigt, sinkt dessen Wasserpotential und Wassermoleküle diffundieren entlang des osmotischen Gradienten aus der Zelle heraus. Der Zellturgor sinkt und in der Folge wird den cytoplasmatischen Proteinen ihre funktionsrelevante Hydrathülle entzogen. Aufgrund dieser Dehydratation kommen Zellstoffwechsel und Zellteilung zum Erliegen.

Um dieses zu verhindern, haben Mikroorganismen im Laufe der Evolution unterschiedliche Strategien entwickelt, z.B. über die Synthese und/oder Aufnahme so genannter kompatibler Solute. Bei externer Verfügbarkeit dieser natürlich vorkommenden Schutzsubstanzen, wie z.B. Prolin oder Glycinbetain, wird deren schnellere und zudem energetisch günstigere Aufnahme gegenüber der Eigensynthese bevorzugt (Wood, Microbiology and Molecular Biology Reviews 63, 230-262 (1999)). Der Symporter ProP gehört zur MFS-Familie (major facilitator superfamily) und katalysiert unter hyperosmotischen Bedingungen die Aufnahme von Prolin, Glycinbetain, Prolinbetain, Ectoin und weiteren strukturähnlichen Substraten im Symport mit Protonen in die Zelle. ProP war der erste Transporter, dem im rekonstituierten System osmosensorische und osmoregulatorische Eigenschaften nachgewiesen werden konnten. Die C-terminale Domäne ist in der Lage, ein sogenanntes coiled coil-Motiv auszubilden. Die Ausbildung dieser Domäne ist wichtig für die Osmoregulation und möglicherweise für die Wahrnehmung osmotischer Stimuli (Culham et al., Journal of Molecular Recognition 13, 309-322 (2000)). ProP wird durch interne Konzentrationserhöhungen von Kaliumionen und durch macromolecular crowding, das in Proteoliposomen durch die Verwendung von Polyethylenglycol (PEG) unterschiedlicher Kettenlänge imitiert wurde, aktiviert (Racher et al., Biochemistry 40, 7324-7333 (2001); Culham et al., Biochemistry 42, 410-420 (2003)). Zwar ist ProP ohne zusätzliche Faktoren in der Lage osmotische Stresssituationen wahrzunehmen, allerdings ist zur vollen Aktivität des Carriers in vivo die Anwesenheit des cytoplasmatischen Proteins ProQ notwendig (Kunte et al., Journal of Bacteriology 181, 1537-43 (1999)).

Mellies et al. beschreiben die Ausschaltung des proP-Gens in E. coli, um die Rolle des Gens bei osmotischem Schock zu untersuchen (Journal of Bacteriology, Bd. 177, Nr. 1, S. 144-151).

Somit war der Transporter ProP aus E. coli schon seit längerem in seiner Funktion bei der Osmoregulation bekannt.

Überraschenderweise wurde erfindungsgemäß nun gefunden, dass die L-Aminosäureproduktion von Mikroorganismen der Familie der Enterobacteriaceae durch Abschwächung des proP-Gens gesteigert werden kann.

Aufgabe der vorliegenden Erfindung war es, ein Verfahren und Mikroorganismen zur Verfügung zu stellen, die eine höhere Überproduktion von schwefelhaltigen Aminosäuren, insbesondere von L-Methionin, ermöglichen.

### Beschreibung der Erfindung

Ein erster Gegenstand der vorliegenden Erfindung ist ein Verfahren zur Herstellung von L-Aminosäuren oder von L-Aminosäure enthaltenden Futtermitteladditiven durch Fermentation eines Mikroorganismus der Familie der Enterobacteriaceae, dadurch gekennzeichnet, dass man einen Mikroorganismus einsetzt, in dem das *proP*-Gen abgeschwächt ist, so dass die Aktivität oder Konzentration des Proteins ProP auf 0 bis 75 % der Aktivität oder Konzentration des Proteins im für das entsprechende Protein nicht rekombinanten Mikroorganismus herabgesenkt wurde.

Der Mikroorganismus produziert die L-Aminosäure und sezerniert sie vorzugsweise ins umgebende Medium.

Der Mikroorganismus reichert die L-Aminosäure weiterhin vorzugsweise im Medium und/oder im Zellinnern an (Akkumulation), wobei eine Anreicherung im Medium besonders bevorzugt ist.

Weiterer Gegenstand der vorliegenden Erfindung ist ein Mikroorganismus aus der Familie der Enterobacteriaceae, der ein abgeschwächtes *proP*-Gen enthält, dadurch gekennzeichnet, dass er L-Methionin überproduziert und vorzugsweise ins Medium sezerniert, wobei er vorzugsweise die L-Aminosäuren im Medium und/oder im Zellinnern, vorzugsweise im Medium, anreichert, dadurch gekennzeichnet, dass er eines oder mehrere der Merkmale ausgewählt aus der folgenden Gruppe besitzt:
a) überexprimiertes Polynukleotid, das für eine oder meh-rere Komponenten des Thiosulfat-/Sulfat-Transportsystems CysPUWA (EC 3.6.3.25) kodiert,
b) überexprimiertes Polynukleotid, das für eine 3'-Phosphoadenosin 5'-Phosphosulfat Reduktase CysH (EC 1.8.4.8) kodiert,
c) überexprimiertes Polynukleotid, das für eine oder mehrere Komponenten der Sulfit Reduktase CysJI (EC 1.8.1.2) kodiert,
d) überexprimiertes Polynukleotid, das für eine Cystein Synthase A CysK (EC 2.5.1.47) kodiert,
e) überexprimiertes Polynukleotid, das für eine Cystein Synthase B CysM (EC 2.5.1.47) kodiert,
f) überexprimiertes Polynukleotid, das für eine Serin Acetyltransferase CysE (EC 2.3.1.30) kodiert,
g) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
h) überexprimiertes Polynukleotid, das für eine Lipoyl Synthase LipA (EC 2.8.1.8) kodiert,
i) überexprimiertes Polynukleotid, das für eine Lipoyl-Protein Ligase LipB (EC 2.3.1.181) kodiert,
j) überexprimiertes Polynukleotid, das für eine Phosphoglycerat Dehydrogenase SerA (EC 1.1.1.95) kodiert,
k) überexprimiertes Polynukleotid, das für eine 3-Phosphoserin Phosphatase SerB (EC 3.1.3.3) kodiert,
l) überexprimiertes Polynukleotid, das für eine 3-Phosphoserin/Phosphohydroxythreonin Aminotransferase SerC (EC 2.6.1.52) kodiert,
m) überexprimiertes Polynukleotid, das für eine Serin Hydroxymethyltransferase GlyA (EC 2.1.2.1) kodiert,
n) überexprimiertes Polynukleotid, das für eine Aspartokinase I und Homoserin Dehydrogenase I ThrA (EC 2.7.2.4, EC 1.1.1.3) kodiert,
o) verstärkte Enzymaktivität der Aspartatkinase, insbesondere ein überexprimiertes Polynukleotid, das für eine Aspartatkinase LysC (EC 2.7.2.4) kodiert,
p) überexprimiertes Polynukleotid, das für eine Homose-rin-Dehydrogenase Hom (EC 1.1.1.3) kodiert,
q) überexprimiertes Polynukleotid, das für eine Homoserin O-Acetyltransferase MetX (EC 2.3.1.31) kodiert,
r) überexprimiertes Polynukleotid, das für eine Homoserin O-Succinlytransferase MetA (EC 2.3.1.46) kodiert,
s) überexprimiertes Polynukleotid, das für eine Cystathioningamma-Synthase MetB (EC 2.5.1.48) kodiert,
t) überexprimiertes Polynukleotid, das für eine β-C-S-Lyase AecD (EC 4.4.1.8, auch als beta-Lyase bezeichnet) kodiert,
u) überexprimiertes Polynukleotid, das für eine Cystathionin beta-Lyase MetC (EC 4.4.1.8) kodiert,
v) überexprimiertes Polynukleotid, das für eine B12 unabhängige Homocystein S-Methyltransferase MetE (EC 2.1.1.14) kodiert,
w) überexprimiertes Polynukleotid, das für eine B12 abhängige Homocystein S-Methyltransferase MetH (EC 2.1.1.13) kodiert,
x) überexprimiertes Polynukleotid, das für eine Methylentetrahydrofolat Reduktase MetF (EC 1.5.1.20) kodiert,
y) überexprimiertes Polynukleotid, das für eine oder mehrere Komponenten des L-Methionin Exporters BrnFE aus Co-rynebacterium glutamicum kodiert,
z) überexprimiertes Polynukleotid, das für eine oder mehrere Komponenten des Valin Exporter YgaZH aus E-scherichia coli (b2682, b2683) kodiert,
aa) überexprimiertes Polynukleotid, das für den putativen Transporter YjeH aus Escherichia coli (b4141) kodiert,
bb) überexprimiertes Polynukleotid, das für eine oder mehrere Komponenten der Pyridin Nucleotid Transhydrogenase PntAB (EC 1.6.1.2) kodiert,
cc) überexprimiertes Polynukleotid, das für eine O-Succinylhomoserine Sulfhydrylase MetZ (EC 2.5.1.48) kodiert,
dd) überexprimiertes Polynukleotid, das für eine Phosphoenolpyruvate Carboxylase Pyc (EC 4.1.1.31) kodiert,
ee) überexprimiertes Polynukleotid, das für eine Thiosulfat-Sulfurtransferase RDL2 (EC 2.8.1.1) kodiert,
ff) überexprimiertes Polynukleotid, das für eine Thiosulfat-Thiol Sulfurtransferase (EC 2.8.1.3) kodiert,
gg) überexprimiertes Polynukleotid, das für eine Thiosulfat-Dithiol Sulfurtransferase (EC 2.8.1.5) kodiert,
hh) erhöhte Aktivität der Homoserin O-Succinyltransferase MetA (EC 2.3.1.46),
ii) erhöhte Aktivität der Serin Acetyltransferase CysE (EC 2.3.1.30),
jj) Abschwächung des für den Transkriptionsregulators der L-Methioninbiosynthese (MetJ) kodierenden Gens metJ (b3938, ECK3930),
kk) Abschwächung des für die Glucose-6-Phosphat-Isomerase (Pgi, EC Nr. 5.3.1.9) kodierenden Gens pgi (b4025, ECK4017),
ll) Abschwächung des für die Homoserinkinase (ThrB, EC 2.7.1.39) kodierenden Gens thrB (b0003, ECK0003),
mm) Abschwächung des für die S-Adenosylmethionin Synthase (MetK, EC Nr. 2.5.1.6) kodierenden Gens metK (b2942, ECK2937),
nn) Abschwächung des für die Dihydrodipicolinat Synthase (DapA, EC Nr. 4.2.1.52) kodierenden Gens dapA (b2478, ECK2474),
oo) Abschwächung des für die Phosphoenolpyruvat Carboxykinase (Pck, EC Nr. 4.1.1.49) kodierenden Gens pck (b3403, ECK3390),
pp) Abschwächung des für die Formyltetrahydrofolat Hydrolase (PurU, EC Nr. 3.5.1.10) kodierenden Gens purU (b1232, ECK1227),
qq) Abschwächung des für die Pyruvatkinase II (PykA, EC Nr. 2.7.1.40) kodierenden Gens pykA (b1854, ECK1855)
rr) Abschwächung des für die Pyruvatkinase I (PykF, EC 2.7.1.40) kodierenden Gens pykF (b1676, ECK1672),
ss) Abschwächung des für eine Untereinheit des L-Methionintransporters (MetQNI) kodierenden Gens metQ (b0197, ECK0197),
tt) Abschwächung des für eine Untereinheit des L-Methionintransporters (MetQNI) kodierenden Gens metI (b0198, ECK0198),
uu) Abschwächung des für eine Untereinheit des L-Methionintransporters (MetQNI) kodierenden Gens metN (b0199, ECK0199),
vv) Abschwächung des für die Deoxycytidin 5'-Triphosphat Deaminase (Dcd, EC Nr. 3.5.4.13) kodierenden Gens dcd (b2065, ECK2059),
ww) Abschwächung des für die putative N-Acyltransferase (YncA, Metabo-lic Explorer WO2010/020681) kodierenden Gens yncA (b1448, ECK1442),
xx) Abschwächung des für den Sigma Faktor RpoS kodierenden Gens rpoS (b2741, ECK2736),
yy) Abschwächung des Regulatorproteins MetJ,
zz) Abschwächung der S-Adenosylmethionin Synthase MetK;
wobei unter "Abschwächung" zu verstehen ist, dass die Aktivität oder Konzentration des entsprechenden Proteins auf 0 bis 75 % der Aktivität oder Konzentration des Proteins im für das entsprechende Protein nicht rekombinanten Mikroorganismus herabgesenkt wurde und wobei unter "Überexpression", "Verstärkung" oder "erhöhte Aktivität" zu verstehen ist, dass die Aktivität des entsprechenden Proteins gegenüber dem Wildtypstamm um einen Faktor von mindestens 2 gesteigert ist.

Der Mikroorganismus zeigt hierbei vorzugsweise eine im Vergleich zum eingesetzten Ausgangsstamm beziehungsweise Elternstamm ohne abgeschwächtes proP-Gen erhöhte Produktion und vorzugsweise Ausscheidung der gewünschten L-Aminosäure in einem Fermentationsprozess.

Unter "abgeschwächt" ist erfindungsgemäß zu verstehen, dass das proP-Gen entweder auf niedrigem Niveau exprimiert wird oder völlig ausgeschaltet ist.

Der Begriff "Abschwächung" beschreibt in diesem Sinne erfindungsgemäß die Verringerung oder Ausschaltung der intrazellulären Aktivität oder Konzentration eines oder mehrerer Enzyme bzw. Proteine, vorliegend insbesondere des ProP-Transporters, in einem Mikroorganismus, die durch die entsprechende DNA, vorliegend insbesondere durch das *proP*-Gen, kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Insbesondere in Bezug auf das *proP*-Gen ist unter "Abschwächung" und "Expression auf niedrigem Niveau" zu verstehen, dass die Aktivität und/oder Konzentration des *ProP-*Transporters durch die zuvor geschilderten Maßnahmen auf 0 bis 75%, 0 bis 50%, 0 bis 25%, 0 bis 10% oder 0 bis 5% der Aktivität und/oder Konzentration des Wildtyp-Proteins, beziehungsweise der Aktivität und/oder Konzentration des Proteins im für den ProP-Transporter nicht rekombinanten Mikroorganismus oder Elternstamm, herabgesenkt wird.

Die L-Aminosäusre wird erfindungsgemäß vorzugsweise durch den Mikroorganismus überproduziert. Unter "Überproduktion" ist erfindungsgemäß zu verstehen, dass hinsichtlich der L-Aminosäure eine drastisch gesteigerte Produktionsleistung über den Eigenbedarf hinaus besteht.

Bei der L-Aminosäure handelt es sich erfindungsgemäß vorzugsweise um eine schwefelhaltige Aminosäure, insbesondere um L-Methionin, L-Cystein, L-Cystin, L-Homocystein oder L-Homocystin, besonders bevorzugt um L-Methionin.

Die erfindungsgemäßen und in erfindungsgemäßen Verfahren eingesetzten Mikroorganismen zeichnen sich vorzugsweise dadurch aus, dass sie eine erhöhte Methionin-Toleranz im Vergleich zu den Mikroorganismen ohne abgeschwächtes proP-Gen aufweisen. Vorzugsweise sind sie hierbei in der Lage, noch noch bei einer L-Methionin-Konzentration von 50 oder 60 Gramm pro Liter, besonders bevorzugt noch bei einer L-Methionin-Konzentration von 70, 75, 80, 90 oder 100 Gramm pro Liter, zu wachsen, da sie gegenüber solchen Methionin-Konzentrationen resistent sind. Die Toleranzangaben hinsichtlich des L-Methionins beziehen sich hierbei vorzugsweise auf einen Minimalagar, der entsprechende L-Methionin-Konzentrationen aufweist.

Derartige Methionin-resistente Stämme können ausgehend vdn einem bereits L-Methionin produzierendem *E. coli* Stamm durch Selektion auf Methionin-haltigem Minimalagar isoliert werden.

Zur Erzeugung der erfindungsgemäßen gegen Methionin resistenten Bakterien werden vorzugsweise im Stand der Technik beschriebene Selektionsmethoden verwendet, diese können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA) nachgelesen werden.

Um gezielt Mutationen im *proP*-Gen vorzunehmen, können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Zur Konstruktion von Mutationen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene *proP*-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden. Auch eine de novo Gensynthese (z.B. durch die Firma GENEART AG (Regensburg, Deutschland)) der Nukleotidsequenzen kann zur Herstellung von Mutationen im *proP*-Gen verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Die erzeugten Allele können beispielsweise durch Transformation und das Verfahren des Gen- bzw. Allelaustausches in das Chromosom geeigneter Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Eine weitere gebräuchliche Methode besteht darin, ein durch PCR oder Gensynthese erzeugtes DNA-Fragment über kurze, homologe Flankensequenzen direkt mit Hilfe der Lambda Red-Rekombinase in das Chromosom einzubauen, bzw. einen Austausch vorzunehmen (Proc Natl Acad Sci U S A.97(12), 6640-6645 (2000); Nature Genetics 20, 123 - 128 (1998)).

Es ist ebenfalls möglich die erzeugten Allele durch Konjugation oder Transduktion in verschiedene Stämme zu überführen.

Die Nukleinsäuresequenzen von *proP* können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Zur Erläuterung sind die bekannte Sequenz des proP-Gens aus Escherichia coli unter der SEQ ID NO: 1 und die bekannten Sequenzen der proP-Gene aus Salmonella enterica und Shigella sonnei, die ebenfalls zur Familie der Enterobacteriaceae gehören, unter SEQ ID NO: 3 und SEQ ID NO: 5 dargestellt. Die von diesen Leserahmen kodierten Proteine sind als SEQ ID NO: 2, SEQ ID NO: 4 und SEQ ID NO: 6 dargestellt. Weitere Nukleotidsequenzen für das *proP*-Gen findet man beispielsweise aus folgenden Enterobacteriaceae: Shigella boydii (Accession NO:: NC_007613); Shigella flexneri (Accession NO: NC_004741); Shigella dysenteriae (Accession NO:: NC_007606); Citrobacter rodentium (Accession NO:: NC_013716); Erwinia pyrifoliae (Accession NO:: NC_012214); Klebsiella pneumoniae (Accession NO:: NC_011283).
Das durch das *proP*-Gen aus Escherichia coli K12 kodierte Protein wird auch als Osmosensorischer MFS Transporter ProP bzw. als Protonen/kompatible Solute Symporter bezeichnet (Accession NO:: 11612 (Region: 4328525-4330027); alternative Gennamen: b4111, ECK4104); Grothe et al., Journal of Bacteriology 166, 253-259 (1986); Racher et al., Biochemistry 38, 1676-1684 (1999); Wood, Methods in Enzymology 428, 77-107 (2007)).

In einer erfindungsgemäß bevorzugten Ausführungsform handelt es sich bei dem abzuschwächenden *proP*-Gen um ein Gen mit einer Sequenzidentität von mindestens 80 %, vorzugsweise von mindestens 90 %, insbesondere von mindestens 95 %, vor allem von mindestens 98, 99 oder 100 %, in Bezug auf vorzugsweise die kompletten Polynukleotidesequenzen gemäß SEQ ID NO: 1, 3, 5 oder 7, besonders bevorzugt in Bezug auf die komplette Polynukleotidsequenz gemäß SEQ ID NO: 1.

Literaturhinweise zu den *proP*-Genen und offenen Leserahmen dieser *proP*-Gene wurden bereits zuvor angegeben. Diese können erfindungsgemäß entsprechend verwendet werden, um das *proP*-Gen abzuschwächen. Weiterhin können auch Allele der Gene oder offene Leserahmen verwendet werden, die sich aus der Degeneriertheit des genetischen Codes oder durch funktionsneutrale Sinnmutationen ("sense mutations") ergeben, um ein abgeschwächtes *proP*-Gen herzustellen. Die Verwendung endogener Gene bzw. endogener offener Leserahmen wird bevorzugt.

Zu den Allelen des *proP*-Gens, welche funktionsneutrale Sinnmutationen enthalten, zählen unter anderem solche, die zu höchstens 40 oder zu höchstens 30 oder zu höchstens 20, bevorzugt zu höchstens 10 oder zu höchstens 5, ganz besonders bevorzugt zu höchstens 3 oder zu höchstens 2 oder zu genau einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 10, 5, 3 oder 2 Aminosäuren oder Aminosäurereste.

Zu den geeigneten Varianten zählen auch solche, die für Proteine kodieren, in denen mindestens eine (1) Aminosäure eingefügt (Insertion) oder entfernt (Deletion) ist. Die maximale Anzahl derartiger als Indels bezeichneter Veränderungen kann 2, 3, 5 in keinem Falle aber mehr als 10 Aminosäuren betreffen.

Zu den geeigneten Varianten gehören ferner solche die durch Hybridisierung, insbesondere unter stringenten Bedingungen unter Verwendung von SEQ ID NO: 1, SEQ ID NO: 3 oder SEQ ID NO: 5 oder Teilen davon, insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID NO: 1, SEQ ID NO: 3 oder SEQ ID NO: 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog NO: 1603558).

Das abzuschwächende *proP*-Gen kodiert für das Transporterprotein ProP, das vorzugsweise eine Aminosäuresequenz aufweist, die zu mindestens 85%, insbesondere zu mindestens 90%, bevorzugt zu mindestens 95%, besonders bevorzugt zu mindestens 98%, zu mindestens 99% oder zu 100 % identisch mit der Aminosäuresequenz gemäß SEQ ID NO:2, SEQ ID NO: 4, SEQ ID NO: 6 oder SEQ ID NO: 8, vorzugsweise mit der Aminosäuresequenz gemäß SEQ ID NO: 2 ist, wobei die Identität vorzugsweise über die gesamte Länge der angegebenen Sequenz(en) vorliegt. Das Transporterprotein ProP umfasst oder besitzt vorzugsweise im Wesentlichen eine Länge von 500 Aminosäuren, wobei eine Länge von 500 Aminosäuren bevorzugt wird. Ganz besonders bevorzugt umfasst oder besitzt das ProP-Protein die Aminosäuresequenz von SEQ ID NO: 2, wobei gegebenenfalls in der Aminosäuresequenz von SEQ ID NO: 2 maximal 40, maximal 30, bevorzugt maximal 20, maximal 10, maximal 5, maximal 3, maximal 2, besonders bevorzugt maximal ein konservative(r) Aminosäureaustausch(e) enthalten sein können. Durch die konservativen Aminosäureaustausche wird die Aktivität des Transporters ProP im Wesentlichen nicht verändert, das heißt erfindungsgemäß vorzugsweise, dass die Aktivität durch die konservativen Aminosäureaustauche um nicht mehr als 10% in Bezug auf die Ausgangssequenz verändert wird.

Der Begriff "im Wesentlichen eine Länge von 500 Aminosäuren" trägt in diesem Zusammenhang der Tatsache Rechnung, dass durch Insertion oder Deletion einer (1) oder mehrerer, maximal 10, 9, 8, 7, 6, 5, 4, 3 oder 2, Aminosäuren innerhalb des Polypeptids oder am N- oder C-terminalen Ende des Polypeptids die Länge des kodierten Polypeptids bei verschiedenen Arten oder Stämmen aus der Familie Enterobacteriaceae geringfügig variiert. Ein Beispiel hierfür ist das ProP-Protein von Erwinia pyrifoliae. Die Länge des Polypeptids (siehe SEQ ID No:8) beträgt in diesem Fall 501 Aminosäuren.

In erfindungsgemäß bevorzugten Ausführungsformen wird die Abschwächung des *proP*-Gens gemäß SEQ ID NO: 1 dadurch erreicht, dass das Gen eine der folgenden Mutationen aufweist:
a) Austausch der Nukleobase Guanin an Position 971 oder einer vergleichbaren Position der Polynukleotidsequenz gemäß SEQ ID NO:1 gegen die Nukleobase Thymin;
b) Austausch der Nukleobase Thymin an Position 1399 oder einer vergleichbaren Position der Polynukleotidsequenz gemäß SEQ ID NO:1 gegen die Nukleobase Cytosin;
c) Austausch der Nukleobase Guanin an Position 1234 oder einer vergleichbaren Position der Polynukleotidsequenz gemäß SEQ ID NO:1 gegen die Nukleobase Thymin;
d) Deletion der Nukleobase Adenin an Position 854 der Polynukleotidsequenz gemäß SEQ ID NO: 1;
e) Deletion einer oder mehrerer der Nukleobasen von Position 1173 bis 1223, bevorzugt Deletion sämtlicher Nukleobasen von Position 1173 bis 1223 der Polynukleotidsequenz gemäß SEQ ID NO:1;
f) Insertion der Nukleobase Cytosin an Position 842 der Polynukleotidsequenz gemäß SEQ ID NO:1;
g) Insertion von einer oder mehreren Nukleobase(n) an Position 973, bevorzugt Insertion von 19 Nukleobasen an Position 973 der Polynukleotidsequenz gemäß SEQ ID NO:1;
h) Insertion von einer oder mehreren Nukleobase(n) an Position 183, bevorzugt Insertion von 1359 Nukleobasen an Position 183 der Polynukleotidsequenz gemäß SEQ ID NO:1.

In erfindungsgemäß bevorzugten Ausführungsformen handelt es sich daher bei dem abgeschwächten *proP-*Gen um ein Polynukleotid, das eine Sequenzidentität von mindestens 80 %, vorzugsweise von mindestens 90 %, insbesondere von mindestens 95 %, besonders bevorzugt von mindestens 98, 99 oder 100 %, in Bezug auf das Polynukleotid gemäß SEQ ID NO: 1, vorzugsweise in Bezug auf die komplette Sequenz des Polynukleotids gemäß SEQ ID NO: 1, aufweist und weiterhin gegenüber dem Polynukleotid gemäß SEQ ID NO: 1 zwingend eine oder mehrere, vorzugsweise genau eine, der folgenden Mutationen aufweist:
a) Austausch des für L-Arginin an Position 324 gemäß SEQ ID NO: 2 oder einer vergleichbaren Position der Aminosäuresequenz kodierenden Tripletts gegen ein Triplett, das für eine Aminosäure ausgewählt aus der Gruppe L-Leucin, L-Isoleucin und L-Valin, bevorzugt L-Leucin, kodiert;
b) Austausch des für L-Tyrosin an Position 467 gemäß SEQ ID NO: 2 oder einer vergleichbaren Position der Aminosäuresequenz kodierenden Tripletts gegen ein Triplett, das für eine Aminosäure ausgewählt aus der Gruppe L-Lysin, L-Arginin und L-Histidin, bevorzugt L-Histidin, kodiert;
c) Austausch des für L-Glutaminsäure an Position 412 gemäß SEQ ID NO: 2 oder eine vergleichbaren Position der Aminosäuresequenz kodierenden Tripletts gegen ein Triplett, das für ein Stopp-Codon kodiert;
d) Deletion der Nukleobase Adenin an Position 854 des *proP*-Gens gemäß SEQ ID NO:1;
e) Deletion einer oder mehrerer der Nukleobasen von Position 1173 bis 1223, bevorzugt Deletion sämtlicher Nukleobasen von Position 1173 bis 1223 des *proP*-Gens gemäß SEQ ID NO:1;
f) Insertion der Nukleobase Cytosin an Position 842 des *proP*-Gens gemäß SEQ ID NO:1;
g) Insertion von einer oder mehreren Nukleobase(n) an Position 973, bevorzugt Insertion von 19 Nukleobasen an Position 973 des *proP*-Gens gemäß SEQ ID NO:1;
h) Insertion von einer oder mehreren Nukleobase(n) an Position 183, bevorzugt Insertion von 1359 Nukleobasen an Position 183 des *proP*-Gens gemäß SEQ ID NO:1.

Die Identitätsangaben in Bezug auf das Polynukleotid gemäß SEQ ID NO: 1 beziehen sich hierbei jeweils auf die Polynukleotidsequenz ohne die eine oder mehrere zuvor genannten zwingenden Mutationen.

"Vergleichbare Positionen" lassen sich durch Vergleich der Aminosäuresequenzen in Form eines "alignments" beispielsweise mit Hilfe des Clustal W-Programmes (Thompson et al., Nucleic Acids Research 22, 4637-4680 (1994)) oder mit Hilfe des MAFFT-Programmes (Katoh etz al., Genome Information 2005; 16(1),22-33) leicht identifizieren.

Bei dem erfindungsgemäßen und in erfindungsgemäßen Verfahren einzusetzenden Mikroorganismus aus der Familie der Enterobacteriaceae handelt es sich vorzugsweise um ein Bakterium der Gattungen Escherichia, Erwinia, Providencia oder Serratia, insbesondere der Gattung Escherichia. Besonders bevorzugt handelt es sich um Escherichia coli.

In erfindungsgemäßen Verfahren zur Herstellung der L-Aminosäure, insbesondere der schwefelhaltigen L-Aminosäure, erfolgt die Fermentation vorzugsweise in einem Medium, das eine anorganische Schwefelquelle enthält. Als Schwefelquelle kann ein Salz der Dithioschwefelsäure (Thiosulfat) eingesetzt werden, ggf. gemeinsam mit anderen Schwefelquellen wie zum Beispiel Sulfat, Sulfit oder Dithionit (siehe auch EP-Anmeldung 11151526.8).

Die L-Aminosäure wird vorzugsweise in der erhaltenen Fermentationsbrühe angereichert und anschließend gegebenenfalls isoliert, gesammelt und/oder gereinigt.

Ebenso ist es möglich, dass man die L-Aminosäure gemeinsam mit Bestandteilen aus der Fermentationsbrühe und/oder Biomasse isoliert oder sammelt.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der ProduktAusbeute (gebildetes Produkt pro verbrauchter KohlenstoffQuelle) und der Produkt-Bildung (gebildetes Produkt pro Volumen und Zeit) oder auch anderer Prozess-Parameter und Kombinationen davon, wird vorzugsweise um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Im erfindungsgemäßen Verfahren können die Bakterien kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium enthält weiterhin vorzugsweise Salze, beispielsweise in Form von Chloriden, von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Cobalamin, Thiamin, Biotin oder Pantothensäure zusätzlich zu den oben genannten Stoffen eingesetzt werden.

Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Ionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Die auf diese Weise hergestellte Fermentationsbrühe wird anschließend vorzugsweise zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise das während der Fermentation eingesetzte Medium enthält vorzugsweise sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukten gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete und erfindungsgemäß bevorzugte Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Weiterer Gegenstand der vorliegenden Erfindung sind entsprechend auch Polynukleotide mit einer Identität von mindestens 80 %, vorzugsweise mindestens 90 %, insbesondere mindestens 95 %, vor allem mindestens 98, 99 oder 100 %, in Bezug auf die Sequenz gemäß SEQ ID NO: 1, wobei sich die angegebene Identität vorzugsweise auf die Gesamtsequenz gemäß SEQ ID NO: 1 bezieht, dadurch gekennzeichnet, dass das Polynukleotid zwingend eine oder mehrere Mutationen gegenüber dem Polynukleotid gemäß SEQ ID NO: 1 aufweist, ausgewählt aus:
a) Austausch des für L-Arginin an Position 324 gemäß SEQ ID NO: 2 oder einer vergleichbaren Position der Aminosäuresequenz kodierenden Tripletts gegen ein Triplett, das für eine Aminosäure ausgewählt aus der Gruppe L-Leucin, L-Isoleucin und L-Valin, bevorzugt L-Leucin, kodiert;
b) Austausch des für L-Tyrosin an Position 467 gemäß SEQ ID NO: 2 oder einer vergleichbaren Position der Aminosäuresequenz kodierenden Tripletts gegen ein Triplett, das für eine Aminosäure ausgewählt aus der Gruppe L-Lysin, L-Arginin und L-Histidin, bevorzugt L-Histidin, kodiert;
c) Austausch des für L-Glutaminsäure an Position 412 gemäß SEQ ID NO: 2 oder eine vergleichbaren Position der Aminosäuresequenz kodierenden Tripletts gegen ein Triplett, das für ein Stopp-Codon kodiert;
d) Deletion der Nukleobase Adenin an Position 854 des *proP-*Gens gemäß SEQ ID NO:1;
e) Deletion einer oder mehrerer der Nukleobasen von Position 1173 bis 1223, bevorzugt Deletion sämtlicher Nukleobasen von Position 1173 bis 1223 des *proP*-Gens gemäß SEQ ID NO:1;
f) Insertion der Nukleobase Cytosin an Position 842 des *proP*-Gens gemäß SEQ ID NO:1;
g) Insertion von einer oder mehreren Nukleobase(n) an Position 973, bevorzugt Insertion von 19 Nukleobasen an Position 973 des *proP*-Gens gemäß SEQ ID NO:1;
h) Insertion von einer oder mehreren Nukleobase(n) an Position 183, bevorzugt Insertion von 1359 Nukleobasen an Position 183 des *proP*-Gens gemäß SEQ ID NO:1.

Die Identitätsangaben in Bezug auf das Polynukleotid gemäß SEQ ID NO: 1 beziehen sich hierbei jeweils auf die Polynukleotidsequenz ohne die eine oder mehrere zuvor genannten zwingenden Mutationen.

Besonders bevorzugt sind die eine oder mehrere zwingenden Mutation(en) hierbei ausgewählt aus:
a) Austausch der Nukleobase Guanin an Position 971 oder einer vergleichbaren Position der Polynukleotidsequenz gemäß SEQ ID NO:1 gegen die Nukleobase Thymin;
b) Austausch der Nukleobase Thymin an Position 1399 oder einer vergleichbaren Position der Polynukleotidsequenz gemäß SEQ ID NO:1 gegen die Nukleobase Cytosin;
c) Austausch der Nukleobase Guanin an Position 1234 oder einer vergleichbaren Position der Polynukleotidsequenz gemäß SEQ ID NO:1 gegen die Nukleobase Thymin;
d) Deletion der Nukleobase Adenin an Position 854 der Polynukleotidsequenz gemäß SEQ ID NO: 1;
e) Deletion einer oder mehrerer der Nukleobasen von Position 1173 bis 1223, bevorzugt Deletion sämtlicher Nukleobasen von Position 1173 bis 1223 der Polynukleotidsequenz gemäß SEQ ID NO:1;
f) Insertion der Nukleobase Cytosin an Position 842 der Polynukleotidsequenz gemäß SEQ ID NO:1;
g) Insertion von einer oder mehreren Nukleobase(n) an Position 973, bevorzugt Insertion von 19 Nukleobasen an Position 973 der Polynukleotidsequenz gemäß SEQ ID NO:1;
h) Insertion von einer oder mehreren Nukleobase(n) an Position 183, bevorzugt Insertion von 1359 Nukleobasen an Position 183 der Polynukleotidsequenz gemäß SEQ ID NO:1.

Die zuvor genannten erfindungsgemäßen Polynukleotide zeichnen sich erfindungsgemäß vorzugsweise dadurch aus, dass sie mit einem oder mehreren der Polynukleotide, die komplementär zu SEQ ID NO: 1, 3 oder 5, vorzugsweise komplementär zu SEQ ID NO: 1, sind, hybridisieren, vorzugsweise unter stringenten Hybridisierungsbedingungen, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken.

Besonders bevorzugte erfindungsgemäße Polynukleotide weisen eine Sequenzidentität von mindestens 90 %, vorzugsweise mindestens 95 %, insbesondere mindestens 98, 99 oder 100 %, zu den Polynukleotiden gemäß SEQ ID NO: 9, SEQ ID NO: 11, SEQ ID NO: 13, SEQ ID NO: 15, SEQ ID NO: 17, SEQ ID NO: 19, SEQ ID NO: 21 oder SEQ ID NO: 23 auf.

Ganz besonders bevorzugte erfindungsgemäße Polynukleotide sind und/oder umfassen die Polynukleotide gemäß SEQ ID NO: 9, SEQ ID NO: 11, SEQ ID NO: 13, SEQ ID NO: 15, SEQ ID NO: 17, SEQ ID NO: 19, SEQ ID NO: 21 und SEQ ID NO: 23.

Weiterer Gegenstand der vorliegenden Erfindung sind Vektoren, die erfindungsgemäße Polynukleotide enthalten.

Weiterer Gegenstand der vorliegenden Erfindung sind entsprechend auch Polypeptide mit einer Identität von mindestens 80 %, vorzugsweise mindestens 90 %, insbesondere mindestens 95 %, vor allem mindestens 98, 99 oder 100 %, in Bezug auf die Sequenz gemäß SEQ ID NO: 2, dadurch gekennzeichnet, dass das Polypeptid zwingend eine oder mehrere Mutationen gegenüber dem Polypeptid gemäß SEQ ID NO: 2 aufweist, ausgewählt aus:
a. Austausch des L-Arginin an Position 324 gemäß SEQ ID NO: 2 oder einer vergleichbaren Position der Aminosäuresequenz gegen eine Aminosäure ausgewählt aus der Gruppe L-Leucin, L-Isoleucin und L-Valin, bevorzugt L-Leucin;
b. Austausch des L-Tyrosin an Position 467 gemäß SEQ ID NO: 2 oder einer vergleichbaren Position der Aminosäuresequenz gegen eine Aminosäure ausgewählt aus der Gruppe L-Lysin, L-Arginin und L-Histidin, bevorzugt L-Histidin;
c. Deletion der 17 Aminosäuren von Position 392 bis 408 gemäß SEQ ID NO: 2 oder einer vergleichbaren Position der Aminosäuresequenz;
d. Deletion von bis zu 420 Aminosäuren des C-Terminus in Bezug auf die Sequenz gemäß SEQ ID NO: 2, vorzugsweise Deletion von 88, 163, 202, 212 oder 420 Aminosäuren des C-Terminus der Aminosäursequenz gemäß SEQ ID NO: 2.

Die Identitätsangaben beziehen sich hierbei bei den Mutanten gemäß a und b auf die Gesamtsequenz gemäß SEQ ID NO: 2, bei den Mutanten gemäß c und d jeweils auf die Teilbereiche der Sequenz gemäß SEQ ID NO: 2 ohne die angegebenen deletierten Bereiche.

Bevorzugte erfindungsgemäße Polypeptide sind Polypeptide mit einer Sequenzidentität von mindestens 90 %, vorzugsweise mindestens 95 %, insbesondere mindestens 98, 99 oder 100 %, zu den Polypeptiden gemäß SEQ ID NO: 10, 12, 14, 16, 18, 20, 22 oder 24.
Besonders bevorzugte erfindungsgemäße Polypeptide sind und/oder umfassen Polypeptide gemäß SEQ ID NO: 10, 12, 14, 16, 18, 20, 22 oder 24.

Weiterer Gegenstand der vorliegenden Erfindung sind rekombinante Mikroorganismen, die erfindungsgemäße Polynukleotide und/oder Vektoren und/oder Polypeptide enthalten.

Erfindungsgemäße Mikroorganismen und in erfindungsgemäßen Verfahren eingesetzte Mikroorganismen weisen vorzugsweise eine verstärkte Enzymaktivität der Aspartatkinase (EC 2.7.2.4) auf, wobei feedbackresistente Allele bevorzugt sind. In *E. coli* gibt es drei verschiedene Aspartatkinasen, die von den Genen *thrA, metL* oder *lysC* kodiert werden. Besonders bevorzugt liegt erfindungsgemäß eine verstärkte Aktivität der Aspartatkinase ThrA vor.

Erfindungsgemäße Mikroorganismen und in erfindungsgemäßen Verfahren eingesetzte Mikroorganismen zeichnen sich weiterhin vorzugsweise dadurch aus, dass sie eine erhöhte Aktivität der Homoserin O-Succinyltransferase MetA (EC 2.3.1.46) und/oder der Serin Acetyltransferase CysE (EC 2.3.1.30) aufweisen.

Weiterhin kann durch die Abschwächung oder Deletion des Regulatorproteins MetJ, das von dem Gen *metJ* kodiert wird, eine Steigerung der L-Methionin-Biosynthese erreicht werden. MetJ stellt den Hauptrepressor der L-Methionin-Biosynthese in *E. coli* dar. Entsprechend ist es erfindungsgemäß weiterhin bevorzugt, dass das Gen *metJ* abgeschwächt ist.

Erfindungsgemäße Mikroorganismen und in erfindungsgemäßen Verfahren eingesetzte Mikroorganismen zeichnen sich weiterhin vorzugsweise dadurch aus, dass sie eine abgeschwächte Aktivität der S-Adenosylmethionin Synthase MetK (EC 2.5.1.6) aufweisen.

Weiterhin kann es für die Produktion von schwefelhaltigen Aminosäuren mit Bakterien der Familie Enterobacteriaceae vorteilhaft sein, zusätzlich ein oder mehrere Enzym(e) der bekannten Aminosäure-Biosynthesewege oder Enzym(e) des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken bzw. deren Aktivität zu erhöhen.

In weiteren bevorzugten Ausführungsformen besitzen die L-Methionin produzierenden Bakterien, eines oder mehrere der Merkmale ausgewählt aus der Gruppe:
a) überexprimiertes Polynukleotid, das für eine oder mehrere Komponenten des Thiosulfat-/Sulfat-Transportsystems CysPUWA (EC 3.6.3.25) kodiert,
b) überexprimiertes Polynukleotid, das für eine 3'-Phosphoadenosin 5'-Phosphosulfat Reduktase CysH (EC 1.8.4.8) kodiert,
c) überexprimiertes Polynukleotid, das für eine oder mehrere Komponenten der Sulfit Reduktase CysJI (EC 1.8.1.2) kodiert,
d) überexprimiertes Polynukleotid, das für eine Cystein Synthase A CysK (EC 2.5.1.47) kodiert,
e) überexprimiertes Polynukleotid, das für eine Cystein Synthase B CysM (EC 2.5.1.47) kodiert,
f) überexprimiertes Polynukleotid, das für eine Serin Acetyltransferase CysE (EC 2.3.1.30) kodiert,
g) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
h) überexprimiertes Polynukleotid, das für eine Lipoyl Synthase LipA (EC 2.8.1.8) kodiert,
i) überexprimiertes Polynukleotid, das für eine Lipoyl-Protein Ligase LipB (EC 2.3.1.181) kodiert,
j) überexprimiertes Polynukleotid, das für eine Phosphoglycerat Dehydrogenase SerA (EC 1.1.1.95) kodiert,
k) überexprimiertes Polynukleotid, das für eine 3-Phosphoserin Phosphatase SerB (EC 3.1.3.3) kodiert,
l) überexprimiertes Polynukleotid, das für eine 3-Phosphoserin/Phosphohydroxythreonin Aminotransferase SerC (EC 2.6.1.52) kodiert,
m) überexprimiertes Polynukleotid, das für eine Serin Hydroxymethyltransferase GlyA (EC 2.1.2.1) kodiert,
n) überexprimiertes Polynukleotid, das für eine Aspartokinase I und Homoserin Dehydrogenase I ThrA (EC 2.7.2.4, EC 1.1.1.3) kodiert,
o) überexprimiertes Polynukleotid, das für eine Aspartatkinase LysC (EC 2.7.2.4) kodiert,
p) überexprimiertes Polynukleotid, das für eine HomoserinDehydrogenase Hom (EC 1.1.1.3) kodiert,
q) überexprimiertes Polynukleotid, das für eine Homoserin O-Acetyltransferase MetX (EC 2.3.1.31) kodiert,
r) überexprimiertes Polynukleotid, das für eine Homoserin O-Succinlytransferase MetA (EC 2.3.1.46) kodiert,
s) überexprimiertes Polynukleotid, das für eine Cystathionin gamma-Synthase MetB (EC 2.5.1.48) kodiert,
t) überexprimiertes Polynukleotid, das für eine β-C-S-Lyase AecD (EC 4.4.1.8, auch als beta-Lyase bezeichnet) kodiert,
u) überexprimiertes Polynukleotid, das für eine Cystathionin beta-Lyase MetC (EC 4.4.1.8) kodiert,
v) überexprimiertes Polynukleotid, das für eine B12 unabhängige Homocystein S-Methyltransferase MetE (EC 2.1.1.14) kodiert,
w) überexprimiertes Polynukleotid, das für eine B12 abhängige Homocystein S-Methyltransferase MetH (EC 2.1.1.13) kodiert,
x) überexprimiertes Polynukleotid, das für eine Methylentetrahydrofolat Reduktase MetF (EC 1.5.1.20) kodiert,
y) überexprimiertes Polynukleotid, das für eine oder mehrere Komponenten des L-Methionin Exporters BrnFE aus *Corynebacterium glutamicum* kodiert,
z) überexprimiertes Polynukleotid, das für eine oder mehrere Komponenten des Valin Exporter YgaZH aus *Escherichia coli* (b2682, b2683) kodiert,
aa) überexprimiertes Polynukleotid, das für den putativen Transporter YjeH aus *Escherichia coli* (b4141) kodiert,
bb) überexprimiertes Polynukleotid, das für eine oder mehrere Komponenten der Pyridin Nucleotid Transhydrogenase PntAB (EC 1.6.1.2) kodiert,
cc) überexprimiertes Polynukleotid, das für eine O-Succinylhomoserine Sulfhydrylase MetZ (EC 2.5.1.48) kodiert,
dd) überexprimiertes Polynukleotid, das für eine Phosphoenolpyruvate Carboxylase Pyc (EC 4.1.1.31) kodiert,
ee) überexprimiertes Polynukleotid, das für eine Thiosulfat-Sulfurtransferase RDL2 (EC 2.8.1.1) kodiert,
ff) überexprimiertes Polynukleotid, das für eine Thiosulfat-Thiol Sulfurtransferase (EC 2.8.1.3) kodiert,
gg) überexprimiertes Polynukleotid, das für eine Thiosulfat-Dithiol Sulfurtransferase (EC 2.8.1.5) kodiert.

Bevorzugte Merkmale sind hierbei eine oder mehrere ausgewählt aus der Gruppe:
a) überexprimiertes Polynukleotid, das für eine oder mehrere Komponenten des Thiosulfat-/Sulfat-Transportsystems CysPUWA (EC 3.6.3.25) kodiert,
b) überexprimiertes Polynukleotid, das für eine 3'-Phosphoadenosin 5'-Phosphosulfat Reduktase CysH (EC 1.8.4.8) kodiert,
c) überexprimiertes Polynukleotid, das für eine oder mehrere Komponenten der Sulfit Reduktase CysJI (EC 1.8.1.2) kodiert,
d) überexprimiertes Polynukleotid, das für eine Cystein Synthase A CysK (EC 2.5.1.47) kodiert,
e) überexprimiertes Polynukleotid, das für eine Cystein Synthase B CysM (EC 2.5.1.47) kodiert,
f) überexprimiertes Polynukleotid, das für eine Serin Acetyltransferase CysE (EC 2.3.1.30) kodiert,
g) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
h) überexprimiertes Polynukleotid, das für eine Lipoyl Synthase LipA (EC 2.8.1.8) kodiert,
i) überexprimiertes Polynukleotid, das für eine Lipoyl-Protein Ligase LipB (EC 2.3.1.181) kodiert,
j) überexprimiertes Polynukleotid, das für eine Phosphoglycerat Dehydrogenase SerA (EC 1.1.1.95) kodiert,
k) überexprimiertes Polynukleotid, das für eine 3-Phosphoserin Phosphatase SerB (EC 3.1.3.3) kodiert,
l) überexprimiertes Polynukleotid, das für eine 3-Phosphoserin/Phosphohydroxythreonin Aminotransferase SerC (EC 2.6.1.52) kodiert,
m) überexprimiertes Polynukleotid, das für eine Serin Hydroxymethyltransferase GlyA (EC 2.1.2.1) kodiert,
n) überexprimiertes Polynukleotid, das für eine Aspartokinase I und Homoserin Dehydrogenase I ThrA (EC 2.7.2.4, EC 1.1.1.3) kodiert,
o) überexprimiertes Polynukleotid, das für eine Aspartatkinase LysC (EC 2.7.2.4) kodiert,
p) überexprimiertes Polynukleotid, das für eine HomoserinDehydrogenase Hom (EC 1.1.1.3) kodiert,
q) überexprimiertes Polynukleotid, das für eine Homoserin-Acetyltransferase MetX (EC 2.3.1.31) kodiert,
r) überexprimiertes Polynukleotid, das für eine Homoserin O-Transsuccinylase MetA (EC 2.3.1.46) kodiert,
s) überexprimiertes Polynukleotid, das für eine Cystathionin gamma-Synthase MetB (EC 2.5.1.48) kodiert,
t) überexprimiertes Polynukleotid, das für eine β-C-S-Lyase AecD (EC 4.4.1.8, auch als beta-Lyase bezeichnet) kodiert,
u) überexprimiertes Polynukleotid, das für eine Cystathionin beta-Lyase MetC (EC 4.4.1.8) kodiert,
v) überexprimiertes Polynukleotid, das für eine B12 unabhängige Homocystein S-Methyltransferase MetE (EC 2.1.1.14) kodiert,
w) überexprimiertes Polynukleotid, das für eine B12 abhängige Homocystein S-Methyltransferase MetH (EC 2.1.1.13) kodiert,
x) überexprimiertes Polynukleotid, das für eine Methylentetrahydrofolat Reduktase MetF (EC 1.5.1.20) kodiert,
y) überexprimiertes Polynukleotid, das für eine Thiosulfat-Sulfurtransferase RDL2 (EC 2.8.1.1) kodiert.

Ganz besonders bevorzugte Merkmale sind hierbei ausgewählt aus der Gruppe:
a) überexprimiertes Polynukleotid, das für eine Aspartokinase I und Homoserin Dehydrogenase I ThrA (EC 2.7.2.4, EC 1.1.1.3) kodiert,
b) überexprimiertes Polynukleotid, das für eine Serin Acetyltransferase CysE (EC 2.3.1.30) kodiert,
c) überexprimiertes Polynukleotid, das für eine Aspartatkinase LysC (EC 2.7.2.4) kodiert,
d) überexprimiertes Polynukleotid, das für eine HomoserinDehydrogenase Hom (EC 1.1.1.3) kodiert,
e) überexprimiertes Polynukleotid, das für eine Homoserin-Acetyltransferase MetX (EC 2.3.1.31) kodiert,
f) überexprimiertes Polynukleotid, das für eine Homoserin O-Transsuccinylase MetA (EC 2.3.1.46) kodiert,
g) überexprimiertes Polynukleotid, das für eine Cystathionin gamma-Synthase MetB (EC 2.5.1.48) kodiert,
h) überexprimiertes Polynukleotid, das für eine β-C-S-Lyase AecD (EC 4.4.1.8, auch als beta-Lyase bezeichnet) kodiert,
i) überexprimiertes Polynukleotid, das für eine Cystathionin beta-Lyase MetC (EC 4.4.1.8) kodiert,
j) überexprimiertes Polynukleotid, das für eine B12 unabhängige Homocystein S-Methyltransferase MetE (EC 2.1.1.14) kodiert,
k) überexprimiertes Polynukleotid, das für eine B12 abhängige Homocystein S-Methyltransferase MetH (EC 2.1.1.13) kodiert,
l) überexprimiertes Polynukleotid, das für eine Methylentetrahydrofolat Reduktase MetF (EC 1.5.1.20) kodiert,
m) überexprimiertes Polynukleotid, das für eine Thiosulfat-Sulfurtransferase RDL2 (EC 2.8.1.1) kodiert.

Der Begriff "Überexpression", "Verstärkung" oder "erhöhte Aktivität" beschreibt erfindungsgemäß die Erhöhung der intrazellulären enzymatischen Aktivität eines oder mehrerer Enzyme in einem Mikroorganismus, die durch die entsprechende DNA kodiert werden.

Grundsätzlich lässt sich eine Steigerung der enzymatischen Aktivität beispielsweise dadurch erzielen, dass man die Kopienzahl der Gensequenz bzw. der Gensequenzen erhöht, welche für das Enzym kodieren, einen starken Promotor verwendet oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder einer Kombination dieser Methoden mit einem Vektor erzeugt, der das gewünschte Gen, ein Allel dieses Gens oder Teile davon und einen die Expression des Gens ermöglichenden Vektor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der enzymatischen Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der enzymatischen Aktivität in Zellen einen Teil der Offenbarung der vorliegenden Erfindung bildet.

Die Erhöhung der enzymatischen Aktivität lässt sich beispielsweise dadurch erzielen, dass man die Kopienzahl der entsprechenden Polynukleotide chromosomal oder extrachromosomal um mindestens eine Kopie erhöht.

Eine weit verbreitete Methode zur Erhöhung der Kopienzahl besteht darin, dass man das entsprechende Polynukleotid in einen Vektor, bevorzugt ein Plasmid, einbaut, der von einem Bakterium repliziert wird.

Geeignete Plasmidvektoren für Enterobacteriaceae sind z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. Besonders geeignet sind zudem von pCL1920 (Lerner, C.G. and Inouye, M., Nucl. Acids Res. (1990) 18:4631 [PMID: 2201955]) abgeleitete Plasmide. Von "Bacterial Artificial Chromosomes" (BAC) abgeleitete Plasmidvektoren, wie z.B. pCC1BAC (EPICENTRE Biotechnologies, Madison, U.S.A.) sind ebenfalls geeignet, die Kopienzahl der entsprechenden Polynukleotide in *E. coli* zu erhöhen.

Weiterhin kann man als Vektoren Transposons, Insertionselemente (IS-Elemente) oder Phagen einsetzen. Derartige genetische Systeme sind beispielsweise in den Patentschriften US 4,822,738, US 5,804,414 und US 5,804414 dargestellt. In gleicher Weise kann das in der WO 92/02627 beschriebene IS-Element ISaBl oder das Transposon Tn 45 des Plasmids pXZ10142 (zitiert im "Handbook of Corynebacterium glutamicum" (Herausgeber: L. Eggeling und M. Bott)) verwendet werden.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines Bakteriums eingefügt. Derartige Amplifikationsverfahren sind beispielsweise in der WO 03/014330 oder WO 03/040373 beschrieben.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für *E. coli* sind z.B. die von Amann et al. (Gene 69(2), 301-315 (1988)) und Amann und Brosius (Gene 40(2-3), 183-190 (1985)) beschriebenen Promotoren T3, T7, SP6, M13, lac, tac und trc bekannt. Ein derartiger Promotor kann beispielsweise stromaufwärts des betreffenden Gens, typischerweise im Abstand von ungefähr 1 - 500 Nukleobasen vom Startkodon eingefügt werden. In der US 5,939,307 wird beschrieben, dass durch Einbau von Expressionskassetten oder Promotoren wie beispielsweise tac-Promotor, trp-Promotor, lpp-Promotor oder PL-Promotor und PR-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacIQ-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden gentechnisch veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-inhibierbar sind.

Wird die Erhöhung der Enzymaktivität durch Erhöhung der Expression eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893 , bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Weiterhin eignen sich auch solche Plasmidvektoren, mit Hilfe derer man das Verfahren der Genamplifikation durch Integration in das Chromosom anwenden kann. Nach homologer Rekombination mittels eines "cross-over"-Ereignisses enthält der resultierende Stamm mindestens zwei Kopien des betreffenden Gens. Ein Verfahren für *E. coli* ist beispielsweise bei Link, A.J., Phillips, D. and Church, G.M. (1997), J. Bacteriology 179: 6228-6237 beschrieben.

Zur Insertion oder Deletion von DNA im Chromosom können auch Rekombinase-vermittelte Verfahren verwendet werden, wie sie beispielsweise von Datsenko KA, Wanner BL., 2000, Proc Natl Acad Sci U S A., 97 (12):6640-5 beschrieben wurden.

Unter der vorstehend und in den nachfolgenden Ausführungen verwendeten Formulierung "eine gegenüber ihrem Wildtypstamm bzw. Ausgangsstamm gesteigerte Aktivität eines Enzyms" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms zu verstehen. Weiterhin umfasst die erfindungsgemäße Zelle, welche "eine gegenüber ihrem Wildtypstamm bzw. Ausgangsstamm gesteigerte Aktivität eines Enzyms" aufweist, insbesondere auch eine Zelle, deren Wildtyp bzw. Ausgangsstamm keine oder zumindest keine nachweisbare Aktivität dieses Enzyms aufweist und die erst nach Erhöhung der Enzymaktivität, beispielsweise durch Überexpression, eine nachweisbare Aktivität dieses Enzyms zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms induziert wird.

Methoden zur Bestimmung der enzymatischen Aktivität verschiedener Enzyme sind der Literatur zu entnehmen.

Die Expression der vorstehend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Weiterhin kann es für die Produktion von L-Aminosäuren, insbesondere L-Methionin, vorteilhaft sein, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So ist es für die Verbesserung der Produktion von L-Methionin in *E.coli* gegebenenfalls zweckmäßig, eines oder mehrere der Gene abzuschwächen, gegebenenfalls auszuschalten oder die Expression zu verringern, ausgewählt aus der Gruppe:
a) ein für den Transkriptionsregulator der L-Methioninbiosynthese (MetJ) kodierendes Gen metJ (b3938, ECK3930),
b) ein für die Glucose-6-Phosphat-Isomerase (Pgi, EC Nr. 5.3.1.9) kodierendes Gen pgi (b4025, ECK4017),
c) ein für die Homoserinkinase (ThrB, EC 2.7.1.39) kodierendes Gen thrB (b0003, ECK0003),
d) ein für die S-Adenosylmethionin Synthase (MetK, EC Nr. 2.5.1.6) kodierendes Gen metK (b2942, ECK2937),
e) ein für die Dihydrodipicolinat Synthase (DapA, EC Nr. 4.2.1.52) kodierendes Gen dapA (b2478, ECK2474),
f) ein für die Phosphoenolpyruvat Carboxykinase (Pck, EC Nr. 4.1.1.49) kodierendes Gen pck (b3403, ECK3390),
g) ein für die Formyltetrahydrofolat Hydrolase (PurU, EC Nr. 3.5.1.10) kodierendes Gen purU (b1232, ECK1227),
h) ein für die Pyruvatkinase II (PykA, EC Nr. 2.7.1.40) kodierendes Gen pykA (b1854, ECK1855)
i) ein für die Pyruvatkinase I (PykF, EC 2.7.1.40) kodierendes Gen pykF (b1676, ECK1672),
j) ein für eine Untereinheit des L-Methionintransporters (MetQNI) kodierendes Gen metQ (b0197, ECK0197),
k) ein für eine Untereinheit des L-Methionintransporters (MetQNI) kodierendes Gen metI (b0198, ECK0198),
l) ein für eine Untereinheit des L-Methionintransporters (MetQNI) kodierendes Gen metN (b0199, ECK0199),
m) ein für die Deoxycytidin 5'-Triphosphat Deaminase (Dcd, EC Nr. 3.5.4.13) kodierendes Gen dcd (b2065, ECK2059),
n) ein für die putative N-Acyltransferase (YncA, Metabolic Explorer WO2010/020681) kodierendes Gen yncA (b1448, ECK1442),
o) ein für die regulatorische sRNA FnrS kodierendes Gen fnrS (b4699, ECK4511),
p) ein für den Sigma Faktor RpoS kodierendes Gen rpoS (b2741, ECK2736).

Besonders bevorzugte Merkmale sind hierbei ausgewählt aus der Gruppe:
a) ein für den Transkriptionsregulator der L-Methioninbiosynthese (MetJ) kodierendes Gen metJ (b3938, ECK3930),
b) ein für die S-Adenosylmethionin Synthase (MetK, EC Nr. 2.5.1.6) kodierendes Gen metK (b2942, ECK2937),
c) ein für die Phosphoenolpyruvat Carboxykinase (Pck, EC Nr. 4.1.1.49) kodierendes Gen pck (b3403, ECK3390),
d) ein für die Formyltetrahydrofolat Hydrolase (PurU, EC Nr. 3.5.1.10) kodierendes Gen purU (b1232, ECK1227),
e) ein für die Pyruvatkinase II (PykA, EC Nr. 2.7.1.40) kodierendes Gen pykA (b1854, ECK1855)
f) ein für die Pyruvatkinase I (PykF, EC 2.7.1.40) kodierendes Gen pykF (b1676, ECK1672),
g) ein für eine Untereinheit des L-Methionintransporters (MetQNI) kodierendes Gen metQ (b0197, ECK0197),
h) ein für eine Untereinheit des L-Methionintransporters (MetQNI) kodierendes Gen metI (b0198, ECK0198),
i) ein für eine Untereinheit des L-Methionintransporters (MetQNI) kodierendes Gen metN (b0199, ECK0199),
j) ein für die putative N-Acyltransferase (YncA, Me-tabolic Explorer WO2010/020681) kodierendes Gen yncA (b1448, ECK1442),
k) ein für den Sigma Faktor RpoS kodierendes Gen rpoS (b2741, ECK2736).

Ganz besonders bevorzugte Merkmale sind hierbei ausgewählt aus der Gruppe:
a) ein für den Transkriptionsregulator der L-Methioninbiosynthese (MetJ) kodierendes Gen metJ (b3938, ECK3930),
b) ein für die S-Adenosylmethionin Synthase (MetK, EC Nr. 2.5.1.6) kodierendes Gen metK (b2942, ECK2937),
c) ein für eine Untereinheit des L-Methionintransporters (MetQNI) kodierendes Gen metQ (b0197, ECK0197),
d) ein für eine Untereinheit des L-Methionintransporters (MetQNI) kodierendes Gen metI (b0198, ECK0198),
e) ein für eine Untereinheit des L-Methionintransporters (MetQNI) kodierendes Gen metN (b0199, ECK0199),
f) ein für die putative N-Acyltransferase (YncA, Me-tabolic Explorer WO2010/020681) kodierendes Gen yncA (b1448, ECK1442),
g) ein für den Sigma Faktor RpoS kodierendes Gen rpoS (b2741, ECK2736).

Die Massnahmen zur Abschwächung werden gegebenenfalls zusätzlich zu bzw. in geeigneter Kombination mit den angegebenen Massnahmen der Verstärkung von Genen zur Erhöhung der Methionin-Produktion durchgeführt.

Zu den vor der erfindungsgemäßen Maßnahme L-Aminosäure produzierenden Mikroorganismen zählen erfindungsgemäß nicht die Wildtypstämme und häufig benutzten Laborstämme wie unter anderen DH5α, DH5αmcr, W3110, MG1655, MC4100, Y1089, H560, BL21 und MM152.

Die L-Aminosäure produzierenden Mikroorganismen können jedoch von diesen Wildtypstämmen und häufig benutzten Laborstämmen abgeleitet sein.

So ist der Ausgangsstamm des Mikroorganismus bevorzugt abgeleitet aus der Gruppe bestehend aus *Escherichia coli* MG1655, *Escherichia coli* W3110, *Escherichia coli* DH5α, *Escherichia coli* DH10B, *Escherichia coli* BW2952, *Escherichia coli* REL606.

Ein erfindungsgemäß bevorzugter L-Methionin ausscheidender bzw. produzierender Stamm ist beispielsweise der *E. coli* Produktionsstamm MG1655 ΔmetJ metA*11 Ptrc-metH Ptrc-metF PtrcF-cysPUWAM PtrcF-cysJIH ΔpykF ΔpykA Ptrc09-gcvTHP ΔpurU Ptrc36-ARNmst17-metF enthaltend die Produktionsplasmide pME101-thrA*1-cysE-Pgap-metA*11 und pCC1BAC-serB-glyA-serA-serC (WO2009/043803).

Ein weiterer erfindungsgemäß bevorzugter L-Methionin ausscheidender bzw. produzierender Stamm ist beispielsweise der *E. coli* Produktionsstamm MG1655 ΔmetJ Ptrc-metH Ptrc-metF PtrcF-cysPUWAM PtrcF-cysJIH Ptrc09-gcvTHP enthaltend das Produktionsplasmid pME101-thrA*1-cysE-Pgap-metA*11.

Die Klonierung des *E. coli* Produktionsstammes MG1655ΔmetJ Ptrc-metH Ptrc-metF PtrcF-cysPUWAM PtrcF-cysJIH Ptrc09-gcvTHP kann, wie in der Patentanmeldung WO2009/043803 beschrieben, durch eine Reihe von P1-Transduktionen und Kurierungen erfolgen. Der Stamm basiert auf dem Wildtypstamm *E. coli* K12 MG1655. Im Genom dieses Stammes wurden die folgenden Veränderungen eingeführt:
- Das Gen für den Repressor der L-Methioninbiosynthese *metJ* wurde deletiert.
- Upstream des Gens *metH* (kodiert für die Cobalaminabhängige Methioninsynthase) wurde der starke trc-Promoter inseriert.
- Upstream des Gens *metF* (kodiert für die 5,10-Methylentetrahydrofolat-Reduktase) wurde der starke trc-Promoter inseriert.
- Upstream des Operons *cysPUWAM* wurde der starke trcF-Promoter inseriert. *cysPUWA* kodiert für einen Sulfat/Thiosulfat-Aufnahmetransporter. *cysM* kodiert für die Cystein-Synthase B.
- Upstream des Operons *cysJIH* wurde der starke trcF-Promoter inseriert. *cysJI* kodiert für die Sulfit-Reductase und *cysH* kodiert für die 3'-Phospho-Adenylylsulfat-Reduktase.
- Upstream des Operons *gcvTHP* wurde der starke trc09-Promoter inseriert. *gcvT, gcvH* und *gcvP* kodieren für drei Komponenten des Glycin-Cleavage-Systems.

Die Klonierung des *E. coli* Produktionsplasmides pME101-thrA*1-cysE-Pgap-metA*11 wird in den Patentanmeldungen WO2007/077041 und WO2009/043803 beschrieben. Es handelt sich um ein Plasmid niedriger Kopienzahl *(low copy* plasmid) auf Basis des Vektors pCL1920 (Lerner, C.G. and Inouye, M., Nucl. Acids Res. (1990) 18:4631 [PMID: 2201955]). Das Leerplasmid pME101 besitzt das *lacI^{q}*-Gen, welches für ein stark exprimiertes Allel des lac-Repressors kodiert. Downstream eines starken, durch den Lac-Repressor reprimierbaren trc-Promoters wurde das Gen *thrA*1* kloniert. Es kodiert für eine *feedback*-resistente Variante der Aspartatkinase / Homoserindehydrogenase ThrA aus *E. coli.* In der gleichen Orientierung dahinter liegt das Gen *cysE* mitsamt seinem natürlichen Promoter. Es kodiert für die Serin-Acetyltransferase aus *E. coli.* Downstream von *cysE* folgt der starke *gapA*-Promoter aus *E. coli,* welcher die Expression des Gens *metA*11* kontrolliert. *metA*11* kodiert für eine *feedback*-resistente Variante der Homoserin O-Succinyltransferase aus *E. coli.*

Als Beispiele für weitere erfindungsgemäß bevorzugte L-Methionin ausscheidende bzw. produzierende Mikroorganismen können folgende Stämme genannt werden:
- *E. coli* TF4076BJF metA#10 + metYX(Lm) (WO2008/127240; Seite 46);
- *E. coli* W3110ΔJ/pKP451 (EP 1 445 310 B1, Seite 7 Bsp. 4);
- *E. coli* WΔthrBCΔmetJmetK32 pMWPthrmetA4Δ5Δ9 (Yoshihiro Usuda and Osamu Kurahashi, 2005, Applied and Environmental Microbiology, Vol. 71, NO: 6, p. 3228-3234);
- W3110/pHC34 (WO01/27307 Seite 13, Bsp. 3);
- *E. coli* ECM2 (EP2205754A2, EP2326724A1 und EP12156052.8).

Weitere Beispiele verschiedener geeigneter Mikroorganismen sind bei Gomes et al. beschrieben (Enzyme and Microbial Technology 37 (2005), 3-18).

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Der Begriff "Gen" bedeutet hier ein Abschnitt auf der Desoxyribonukleinsäure (DNA), der die Informationen zur Herstellung (Transkription) zunächst einer Ribonukleinsäure (RNA) und diese die Information zur Herstellung (Translation) eines Proteins (Polypeptids) enthält, hier ein Polypeptid mit der Aktivität einer (p)ppGpp Synthetase II. Die Tatsache, dass ein Gen oder ein Polynukleotid die Informationen zur Herstellung eines Proteins enthält, bezeichnet man auch als Kodierung eines Protein bzw. Polypeptides durch das Gen bzw. durch die RNA. Unter endogenen Genen beziehungsweise Polynukleotiden versteht man die in der Population einer Art vorhandenen offenen Leserahmen (ORF), Gene oder Allele beziehungsweise deren Polynukleotide. Die Begriffe "Gen" und "ORF" (offenen Leserahmen) werden in dieser Erfindung synonym verwendet.

Der Begriff "Polynukleotid" bezieht sich im Allgemeinen auf Polyribonukleotide und Polydeoxyribonukleotide, wobei es sich um nicht modifizierte RNA oder DNA oder modifizierte RNA oder DNA handeln kann.

Der Begriff "Polypeptid" bezeichnet Peptide oder Proteine, die zwei oder mehr über Peptidbindungen verbundene Aminosäuren enthalten. Die Begriffe Polypeptid und Protein werden als Synonyme verwendet. Proteine gehören zu den Grundbausteinen aller Zellen. Sie verleihen der Zelle nicht nur Struktur, sondern sind die molekularen "Maschinen", die Stoffe transportieren, chemische Reaktionen katalysieren und Signalstoffe erkennen.

Unter "proteinogenen Aminosäuren" versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin, sowie Selenocystein. Dabei handelt es sich bei den proteinogenen Aminosäuren stets um α-Aminosäuren. Bis auf die Aminosäure Glycin ist für alle proteinogenen Aminosäuren das α-Kohlenstoffatom asymmentrisch (die Moleküle sind chiral): Es existieren von jeder dieser Aminosäuren zwei Enantiomere. Dabei ist nur eines der beiden Enatiomere proteinogen und zwar die L-Aminosäure: der zum Aufbau der Proteine notwendige Apparat - das Ribosom, die tRNA, die Aminoacyl-tRNA Synthetase (diese belädt die tRNA mit Aminosäuren) und andere- sind selbst auch chiral und können nur die L-Variante erkennen.

Der Begriff "Genexpression" (kurz "Expression") bezeichnet im Allgemeinen die Ausprägung der genetischen Information zu einem Phänotyp. Im engeren Sinne bezeichnet Genexpression die Transkription eines Gens zu einer RNA, die anschließende Translation der RNA zu einem Polypeptid, welches eine enzymatische Aktivität besitzen kann.

Unter einem "Ausgangsstamm" (Elternstamm) versteht man den Mikroorganismenstamm, an dem Maßnahmen zur Erhöhung der Produktivität einer oder mehrerer Aminosäuren, Peptide oder Proteine, bzw. Maßnahmen zur Erhöhung der Aktivität eines oder mehrerer Enzyme (z.B. eine zur Überexpression führende Maßnahme) durchgeführt werden. Bei einem Ausgangsstamm kann es sich um einen wildtypischen Stamm, aber auch um einen bereits vorher veränderten Stamm handeln (beispielsweise um einen L-Aminosäure-produzierenden Mikroorganismus (Produktionsstamm)).

Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden, wenn nicht anders beschrieben, nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Wenn nicht anders beschrieben beträgt die Bebrütungstemperatur bei der Herstellung von Stämmen und Transformanten 37°C.

### Beispiel 1

### Screening L-Methionin toleranter Mutanten

Der L-Methionin produzierende *E. coli* Stamm ECM2 basiert auf dem wildtypischen K12-Stamm MG1655. Der Stamm ECM2 trägt wie in EP2205754A2, EP2326724A1 und EP12156052.8 beschrieben ein feedbackresistentes metA-Allel, eine Deletion der Gene metJ, yncA, pykA und pykF, eine Variante des spoT-Gens, sowie jeweils eine Promotorverstärkung vor den Genen metH, metF, gcvT, cysP und cysJ.

### 1.1 Klonierung des serC-Gens in das Plasmid pUC18

Das Gen serC aus Escherichia coli MG1655 wurde mit Hilfe der Polymerase-Kettenreaktion (PCR) amplifiziert und anschließend in das Plasmid pUC18 (Fermentas GmbH, St. Leon-Rot, Deutschlan) kloniert.

Die PCR-Primer serCF(XbaI) und serCR(HindIII) besitzen an den 5'-Enden jeweils 6 random Nucleotide gefolgt von Erkennungssequenzen für die Restriktionsendonucleasen XbaI (TCTAGA) bzw. HindIII (AAGCTT). Die Nukleotide 13 bis 38 von serCF(XbaI) binden im E. coli MG1655-Genom von Pos. 956619 bis 956644. Die Nukleotide 13 bis 37 von serCR(HindIII) binden im E. coli MG1655-Genom von Pos. 958028 bis 958004.
serCF(XbaI) (SEQ ID NO: 25)
   5' AGGTGCTCTAGAGTCCGCGCTGTGCAAATCCAGAATGG 3'
serCR(HindIII) (SEQ ID NO: 26)
   5' TACACCAAGCTTAACTCTCTACAACAGAAATAAAAAC 3'

Das Gen serC wurde mit den Primern serCF(XbaI) und ser-CR(HindIII) unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1434 bp. Es wurde mit den Restriktionsendonucleasen XbaI und HindIII gespalten und mit Hilfe eines QIAquick PCR Purification Kit (Qiagen, Hilden, Deutschland) aufgereinigt. Nicht methylierte DNA des Plasmides pUC18 wurde mit den Restriktionsendonucleasen XbaI und HindIII gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in E. coli DH5α transformiert. Plasmidklone, die das serC-Gen enthielten wurden durch Restriktionsspaltung und DNA-Sequenzierung identifiziert. Das resultierende Plasmid wurde mit pUC18-serC bezeichnet.

### 1.2 Klonierung des serA-Gens in das Plasmid pUC18-serC

Das Gen serA aus Escherichia coli MG1655 wurde mit Hilfe der Polymerase-Kettenreaktion (PCR) amplifiziert und anschließend in das Plasmid pUC18-serC kloniert.

Der PCR-Primer serAF(XbaI) besitzt am 5'-Ende 6 random Nucleotide gefolgt von einer Erkennungssequenz für die Restriktionsendonuclease XbaI (TCTAGA). Die Nukleotide 12 bis 33 binden im E. coli MG1655-Genom von Pos. 3055199 bis 3055220. Der PCR-Primer serAR(SHSSNB) besitzt am 5'-Ende 6 random Nucleotide gefolgt von Erkennungssequenzen für die Restriktionsendonucleasen SacI, HindIII, SphI, SmaI, NotI und BglII. Die Nukleotide 49 bis 58 binden im E. coli MG1655-Genom von Pos. 3056880 bis 3056861.
serAF(XbaI) (SEQ ID NO: 27)
   5' CTGTAGTCTAGATTAGTACAGCAGACGGGCGCG 3'
serAR(SHSSNB) (SEQ ID NO: 28)

Das Gen serA wurde mit den Primern serAF(XbaI) und se-rAR(SHSSNB) unter Anwendung der Po-lymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1731 bp.

Es wurde mit den Restriktionsendonucleasen XbaI und SacI gespalten und mit Hilfe eines QIAquick PCR Purification Kit (Qiagen, Hilden, Deutschland) aufgereinigt. Das Plasmid pUC18-serC wurde ebenfalls mit den Restriktionsendonucleasen XbaI und SacI gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in E. coli DH5α transformiert. Plasmidklone, die das serA-Gen enthielten wurden durch Restriktionsspaltung und DNA-Sequenzierung identifiziert. Das resultierende Plasmid wurde mit pUC18-serAC bezeichnet.

### 1.3 Klonierung des serB-Gens in das Plasmid pUC18-serAC

Das Gen serB aus Escherichia coli MG1655 wurde mit Hilfe der Polymerase-Kettenreaktion (PCR) amplifiziert und anschließend in das Plasmid pUC18-serAC kloniert.

Der PCR-Primer serB(SphI) besitzt am 5'-Ende 6 random Nucleotide gefolgt von einer Erkennungssequenz für die Restriktionsendonuclease SphI (GCATGC). Die Nukleotide 13 bis 34 binden im E. coli MG1655-Genom von Pos. 4622816 bis 4622837.

Der PCR-Primer serB(SmaI) besitzt am 5'-Ende 6 random Nucleotide gefolgt von Erkennungssequenzen für die Restriktionsendonucleasen SalI (GTCGAC) und SmaI (CCCGGG). Die Nukleotide 54 bis 75 binden im E. coli MG1655-Genom von Pos. 4623887 bis 4623866.
serB(SphI) (SEQ ID NO: 29)
   5' CCATGCGCATGCCCACCCTTTGAAAATTTGAGAC 3'
serB(SmaI) (SEQ ID NO: 30)

Das Gen serB wurde mit den Primern serB(SphI) und serB(SmaI) unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1137 bp.

Es wurde mit den Restriktionsendonucleasen SphI und SmaI gespalten und mit Hilfe eines QIAquick PCR Purification Kit (Qiagen, Hilden, Deutschland) aufgereinigt. Das Plasmid pUC18-serAC wurde ebenfalls mit den Restriktionsendonucleasen SphI und SmaI gespalten, mit alkalischer Phosphatase dephosphoryliert und mit Hilfe eines QIAquick PCR Purification Kit (Qiagen, Hilden, Deutschland) aufgereinigt. Anschließend wurde das gespaltene Plasmid mit dem PCR-Produkt ligiert und in E. coli DH5α transformiert. Plasmidklone, die das serB-Gen enthielten wurden durch Restriktionsspaltung und DNA-Sequenzierung identifiziert. Das resultierende Plasmid wurde mit pUC18-serBAC bezeichnet.

### 1.4 Klonierung des glyA-Gens in das Plasmid pUC18-serBAC

Das Gen glyA aus Escherichia coli MG1655 wurde mit Hilfe der Polymerase-Kettenreaktion (PCR) amplifiziert und anschließend in das Plasmid pUC18-serBAC kloniert.

Der PCR-Primer glyA-downstream besitzt am 5'-Ende 6 random Nucleotide gefolgt von einer Erkennungssequenz für die Restriktionsendonuclease BglII (AGATCT). Die Nukleotide 13 bis 35 binden im E. coli MG1655-Genom von Pos. 2682063 bis 2682085.

Der PCR-Primer glyA-upstream besitzt am 5'-Ende 6 random Nucleotide gefolgt von Erkennungssequenzen für die Restriktionsendonuclease NotI (GCGGCCGC). Die Nukleotide 15 bis 33 binden im E. coli MG1655-Genom von Pos. 2683762 bis 2683744.
glyA-downstream(SEQ ID NO: 31)
   5' ATCTAAAGATCTGTTACGACAGATTTGATGGCGCG 3'
glyA-upstream (SEQ ID NO: 32)
   5' TTCATCGCGGCCGCGAAAGAATGTGATGAAGTG 3'

Das Gen glyA wurde mit den Primern glyA-downstream und glyA-upstream unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1726 bp.

Es wurde mit den Restriktionsendonucleasen BglII und NotI gespalten und mit Hilfe eines QIAquick PCR Purification Kit (Qiagen, Hilden, Deutschland) aufgereinigt. Das Plasmid pUC18-serBAC wurde ebenfalls mit den Restriktionsendonucleasen BglII und NotI gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in E. coli DH5α transformiert. Plasmidklone, die das glyA-Gen enthielten wurden durch Restriktionsspaltung und DNA-Sequenzierung identifiziert. Das resultierende Plasmid wurde mit pUC18-serB-glyA-serAC bezeichnet.

### 1.5 Klonierung der Gene serB-glyA-serAC aus pUC18-serB-glyA-serAC nach pCC1-BAC

Das Plasmid pUC18-serB-glyA-serAC wurde mit der Restriktionsendonuclease HindIII gespalten und die DNA-Fragmente durch eine Agarosegelelektrophorese aufgetrennt. Ein 5,9 kb großes DNA-Fragment wurde aus dem Gel isoliert. Es enthielt die Gene serB, glyA, serA und serC. Das Fragment wurde mit dem bereits mit HindIII gespaltenen Plasmid pCC1BAC Cloning-Ready Vector (Hind III) der Firma Epicentre /Madison, USA) ligiert und nach E. coli EPI300 transformiert. Plasmidklone, die das DNA-Fragment aus serB, glyA, serA und serC enthielten wurden durch Restriktionsspaltung und DNA-Sequenzierung identifiziert. Das resultierende Produktionsplasmid wurde mit pCC3 bezeichnet.

### 1.6 Klonierung des Produktionsplasmides pME-RDL2a

Die Klonierung des Produktionsplasmides pME-RDL2a erfolgte, wie in der EP-Anmeldung 11151526.8 beschrieben. Es beinhaltet das Gen cysE aus Escherichia coli, feedbackresistente Allele der Gene thrA und metA aus Escherichia coli sowie das Gen RDL2a, welches für die Thiosulfat-Sulfurtransferase RDL2p aus Saccharomyces cerevisiae codiert. Zusätzlich beinhaltet es ein Streptomycin-Resistenzgen.

### 1.7 Transformation des Stammes ECM2 mit den Produktionsplasmiden

Der Stamm ECM2 wurden mit dem Produktionsplasmid pCC3 aus Beispiel 1.5 transformiert und die Transformanden mit 20 mg/l Chloramphenicol selektioniert. Anschließend wurden die Zellen mit dem Plasmid pME-RDL2a aus Beispiel 1.6 transformiert und die resultierenden Transformanden mit 20 mg/l Chloramphenicol + 100 mg/l Streptomycin selektioniert. Der resultierende Stamm wurde mit ECM2/pCC3/pME-RDL2a bezeichnet.

### 1.8 Screening und Sequenzierung L-Methionin toleranter Mutanten

Zur Selektion L-Methionin toleranter Mutanten wurde von dem Stamm ECM2/pCC3/pME-RDL2a eine Vorkultur auf PC1-Minimalmediumplatten (Tabelle 1, mit 14 g/l Agar) mit 75g/l L-Methionin (Merck, Frankfurt, Deutschland) ausplattiert. Als Vorkultur wurde 10 ml Vorkulturmedium (10% LB-Medium mit 2,5 g/l Glucose und 90% Minimalmedium PC1) mit 100µl Zellkultur beimpft und 10 Stunden bei 37°C kultiviert.

**Tabelle 1: Minimal Medium PC1**

| Substanz | Konzentration |
|---|---|
| ZnS04 * 7 H2O | 4 mg/l |
| CuCl2 * 2 H2O | 2 mg/l |
| MnSO4 * H2O | 20 mg/l |
| H3BO3 | 1 mg/l |
| Na2MoO4 * 2 H2O | 0,4 mg/l |
| MgSO4 * 7 H2O | 1 g/l |
| Citronensäure * 1 H2O | 6,56 g/l |
| CaCl2 * 2 H2O | 40 mg/l |
| K2HPO4 | 8,02 g/l |
| Na2HPO4 | 2 g/l |
| (NH4)2HPO4 | 8 g/l |
| NH4Cl | 0,13 g/l |
| (NH4)2SO3 | 5,6 g/l |
| MOPS | 5 g/l |
| NaOH 10M | auf pH 6,8 eingestellt |
| | |
| FeSO4 * 7 H2O | 40 mg/l |
| Thiaminhydrochlorid | 10 mg/l |
| Vitamin B12 | 10 mg/l |
| Glucose | 10 g/l |
| Isopropyl-thio-β-galaktosid (IPTG) | 2,4 mg/l |
| Spectinomycin | 50 mg/l |
| Chloramphenicol | 20 mg/l |

Nach 5 Tagen Inkubation konnten Einzelkolonien L-Methionin toleranter Mutanten von den Platten isoliert werden.

Zur Vorbereitung der Sequenzierung wurden nach Vermehrung in Antibiotika-freiem LB-Medium für circa sechs Generationen Derivate der L-Methionin toleranten Mutanten des Stammes ECM2/pCC3/pME-RDL2a isoliert, welche die Plasmide pCC3 und pME-RDL2a nicht mehr tragen. Die erhaltenen Stämme sind Streptomycin- und Chloramphenicol-sensitiv.

Von 8 Mutanten wurde chromosomale DNA isoliert. Diese DNA wurde zur Gesamtgenomsequenzierung (GATC, Konstanz, Deutschland) eingesetzt.

Im Vergleich zu dem Ausgangsstamm ECM2 zeigten sich auschließlich Mutationen im *proP*-Gen. In der nachfolgenden Tabelle 2 sind die Mutationen aufgelistet. Die Sequenzen der *proP*-Allele sind in den SEQ ID No: 9 bis SEQ ID No: 24 dargestellt.

**Tabelle 2:**

| **Stamm** | **Mutation im *proP*-Gen** | **Art der Mutation** | **Bezeichnung der Mutation** |
|---|---|---|---|
| DM2321 | E412* | AA-Austausch | M8 (SEQ ID NO:9-10) |
| DM2322 | Insertion C nach Nukleotidposition 842 | Nukleotidinsertion | M3 (SEQ ID NO:11-12) |
| DM2323 | Deletion A nach Nukleotidposition 854 | Nukleotiddeletion | M4 (SEQ ID NO:13-14) |
| DM2324 | Y467H | AA-Austausch | M11 (SEQ ID NO:15-16) |
| DM2325 | Deletion 51bp von Nukleotidposition 1173 bis 1223 | Nukleotiddeletion | M7 (SEQ ID NO:17-18) |
| DM2326 | 19 bp-Insertion nach Nukleotidposition 973 | Nukleotidinsertion | M6 (SEQ ID NO:19-20) |
| DM2327 | R324L | AA-Austausch | M5 (SEQ ID NO:21-22) |
| DM2328 | 1359 bp-Insertion nach Nukleotidposition 183 | Nukleotidinsertion | M2 (SEQ ID NO:23-24) |

Die Stämme DM2321, DM2322, DM2323, DM2324, DM2325, DM2326, DM2327 und DM2328 wurden gemäß dem Budapester Vertrag am 23. August 2011 bei der DSMZ (Deutsche Sammlung von Mikroorganismen und Zellkulturen GmbH, Inhoffenstraße 7 B, 38124 Braunschweig, Deutschland) unter den DSM-Nummern DSM 25095 (= DM2321), 25096 (= DM2322), 25097 (= DM2323), 25098 (= DM2324), 25099 (= DM2325), 25100 (= DM2326), 25101 (= DM2327), und 25102 (= DM2328) hinterlegt.

### Beispiel 2

### Aktivitätsnachweis der L-Methionin toleranten proP-Mutanten

Zur Bestimmung der ProP-Aktivität der *proP*-Mutanten wurde exemplarisch die Prolinaufnahme für den Stamm DM2328 im Vergleich zum Ausgangsstamm ECM2 analysiert. Hierzu wurde eine 10 ml Vorkultur 1 in LB-Medium über Tag bei 37 °C schüttelnd inkubiert. 1 ml der Vorkultur wurde in 20 ml M9-Medium als Vorkultur 2 überführt und über Nacht kultiviert. Vorkultur 2 wurde auf eine OD600 von 0,2 in 50 ml frischem M9-Medium angeimpft. Die Zellen wurden daraufhin 3-4 h kultiviert, anschließend dreimal mit eiskaltem M9-Puffer gewaschen, auf eine OD600 von 2 eingestellt und auf Eis aufbewahrt.

Die Prolinaufnahme wurde durch radioaktiv markiertes [14C(U)]L-Prolin (spezifische Aktivität: 1,85 MBq; Hartmann Analytic, Deutschland) bestimmt. Jeweils 2,3 ml Zellsuspension wurde dafür in ein Rührgefäß gegeben und für 2 min bei 37°C durch die Zugabe von 10 mM Glucose energetisiert. Unter Rühren wurde die Transportmessung durch Zugabe von 20 µl L-[14C]-Prolin mit einer Konzentrationen von 24,6 mM gestartet. Zu verschiedenen Zeitpunkten (0, 15, 30, 45, 60, 75, 90, 105 und 120 s) wurden jeweils 200 µl entnommen und auf Glasfaserfilter (Millipore) pipettiert. Durch eine Vakuum-Mehrfachfiltrationsanlage wurde das umgebende Medium abgesaugt und die Zellen wurden nachfolgend zweimal mit 2,5 ml 0,1 M LiCl-Lösung gewaschen. Die Filter wurden mit 3,8 ml Scintillationsflüssigkeit (Roth, Karlsruhe, Deutschland) versetzt und die Radioaktivität mit Hilfe eines Scintillationszählers (LS 6500, Beckman Instruments, München, Deutschland) ermittelt. Die Gesamtaktivität im Reaktionsansatz wurde durch die direkte Messung einer Probe ohne Filtration bestimmt. Für jede Probe wurde der Zerfall pro Minute (dpm) ermittelt. Die Aufnahmeaktivität wurde in nmol/min (mg TG) (0,36 = Trockengewicht-Relation von E. coli [mg/ml OD=1]) berechnet. Aus dem linearen Teil der Aufnahmekinetik konnte die Transportrate in nmol/mg*min abgeleitet werden. In der nachfolgenden Tabelle 3 sind die Mittelwerte aus drei parallelen Messungen angegeben.

**Tabelle 3:**

| Stamm | L-Prolin-Transportrate (nmol/mg*min) |
|---|---|
| ECM2 | 2,39 |
| DM2328 | 1,27 |

### Beispiel 3

### Transformation der Stämme DM2321, DM2322, DM2323, DM2324, DM2325, DM2326, DM2327 und DM2328 mit den Produktionsplasmiden

Die Stämme DM2321, DM2322, DM2323, DM2324, DM2325, DM2326, DM2327 und DM2328 wurden mit dem Produktionsplasmid pCC3 aus Beispiel 1.5 transformiert und die Transformanden mit 20 mg/l Chloramphenicol selektioniert. Anschließend wurden die Zellen mit dem Plasmid pME-RDL2a aus Beispiel 1.6 transformiert und die resultierenden Transformanden mit 20 mg/l Chloramphenicol und 100 mg/l Streptomycin selektioniert. Die resultierenden Stämme wurden mit DM2321/pCC3/pME-RDL2a, DM2322/pCC3/pME-RDL2a, DM2323/pCC3/pME-RDL2a, DM2324/pCC3/pME-RDL2a, DM2325/pCC3/pME-RDL2a, DM2326/pCC3/pME-RDL2a, DM2327/pCC3/pME-RDL2a und DM2328/pCC3/pME-RDL2a bezeichnet.

### Beispiel 4

### Leistungstest im Schüttelkolbenversuch

Die Leistungsfähigkeit der *E.coli* L-Methionin-Produktionsstämme wurde durch Produktionsversuche in 100 ml Erlenmeyer-Kolben bewertet. Als Vorkulturen wurden jeweils 10 ml Vorkulturmedium (10% LB-Medium mit 2,5 g/l Glucose und 90% Minimalmedium PC1) mit 100µl Zellkultur beimpft und 10 Stunden bei 37°C kultiviert. Hiermit wurden anschließend je 10 ml PC1-Minimalmedium (siehe Tabelle 1 in Beispiel 1) auf eine OD 600 nm von 0,2 (Eppendorf Bio-Photometer; Eppendorf AG, Hamburg, Deutschland) beimpft und für 24 Stunden bei 37°C kultiviert. Die extrazelluläre L-Methionin-Konzentration wurde mit einem Aminosäureanalysator (Sykam GmbH, Eresing, Deutschland) durch Ionenaustauschchromatographie und Nachsäulenderivatisierung mit Ninhydrindetektion bestimmt. Die extrazelluläre Glucose-Konzentration wurde mit einem YSI 2700 Select Glucose Analyzer (YSI Life Sciences, Yellow Springs, Ohio, USA) bestimmt. Die Ergebnisse sind in Tabelle 4 dargestellt. Nach 24 Stunden war die Glucose in beiden Kulturen vollständig verbraucht.

**Tabelle 4: L-Methioninkonzentrationen in den Fermentationsbrühen der untersuchten E. coli Stämme**

| Stamm | OD(600nm) | L-Methionin (g/l) |
|---|---|---|
| ECM2/pCC3/pME-RDL2a | 3,27 | 1,71 |
| DM2321/pCC3/pME-RDL2a | 3,04 | 1,83 |
| DM2322/pCC3/pME-RDL2a | 3,38 | 1,83 |
| DM2323/pCC3/pME-RDL2a | 3,20 | 1,83 |
| DM2324/pCC3/pME-RDL2a | 3,38 | 1,82 |
| DM2325/pCC3/pME-RDL2a | 3,44 | 1,83 |
| DM2326/pCC3/pME-RDL2a | 3,23 | 1,87 |
| DM2327/pCC3/pME-RDL2a | 3,22 | 1,99 |
| DM2328/pCC3/pME-RDL2a | 3,12 | 1,86 |

### Beispiel 5

### Klonierung der proP-Allele aus M8, M4 und M6 in das Plasmid pMAK705

Die *proP*-Allele M8 aus DM2321, M4 aus DM2323 und M6 aus DM2326 wurden mit Hilfe der Polymerase-Kettenreaktion (PCR) amplifiziert und anschließend in das Plasmid pMAK705 kloniert.

Auf Basis der für die *proP*-Allele erhaltenen Sequenzen (siehe Beispiel 1) wurde ein Primerpaar designt, mit dem jeweils ein die jeweilige Mutation umfassendes Fragment amplifiziert werden kann. Dieses kann anschließend in das Plasmid pMAK705 (Hamilton CM, Aldea M, Washburn BK, Babitzke P, Kushner SR (1989); J Bacteriol.; 171(9): 4617-4622) kloniert werden.

Der PCR-Primer proPmut1(NotI) besitzt am 5'-Ende 4 random Nucleotide, gefolgt von der Erkennungssequenz für die Restriktionsendonuclease HindIII.

Der PCR-Primer proPmut2(BamHI) besitzt am 5'-Ende 4 random Nucleotide, gefolgt von der Erkennungssequenz für die Restriktionsendonuclease BamHI.

Die Nukleotide 13 bis 32 von proPmut1(NotI) binden im E. coli MG1655-Genom von Pos. 4328800 bis 4328819. Die Nukleotide 13 bis 37 von proPmut2(NotI) binden im E. coli MG1655-Genom von Pos. 4330303 bis 4330322.
proPmut1(HindIII) (SEQ ID NO: 33)
   5' GTCAAAGCTT ATATGGTCGCCAGAAGATCC 3'
proPmut2(BamHI) (SEQ ID NO: 34)
   5' GTCAGGATCC TCAGCCGCATTACACAGTTG 3'

Als Template diente genomische DNA der Stämme DM2321, DM2323 und DM2328 (siehe Beispiel 1).

Es wurde jeweils ein die entsprechende Mutation im Gen *proP* überspannendes Fragment unter Anwendung der Polymerase-Kettenreaktion (PCR) mit der Phusion DNA-Polymerase (Finnzymes Oy, Espoo, Finnland) amplifiziert. Das Fragment aus DM2321 (trägt proP-M8), enthaltend die Mutation E412*, hatte eine Größe von 1564bp (SEQ ID NO: 35). Das Fragment aus DM2323 (trägt proP-M4), enthaltend eine Deletion eines "A" nach Nukleotidposition 854, hatte eine Größe von 1542bp (SEQ ID NO: 36). Das Fragment aus DM2326 (trägt proP-M6), enthaltend eine 19bp Insertion nach Nukleotidposition 973, hatte eine Größe von 1563bp (SEQ ID NO: 37).

Alle 3 Fragmente wurden mit den Restriktionsendonucleasen HindIII und BamHI gespalten und mit Hilfe Hilfe eines QIAquick PCR Purification Kit (Qiagen, Hilden, Deutschland) aufgereinigt. Anschließend wurden die Fragmente zur Ligation mit dem Plasmid pMAK705 eingesetzt.

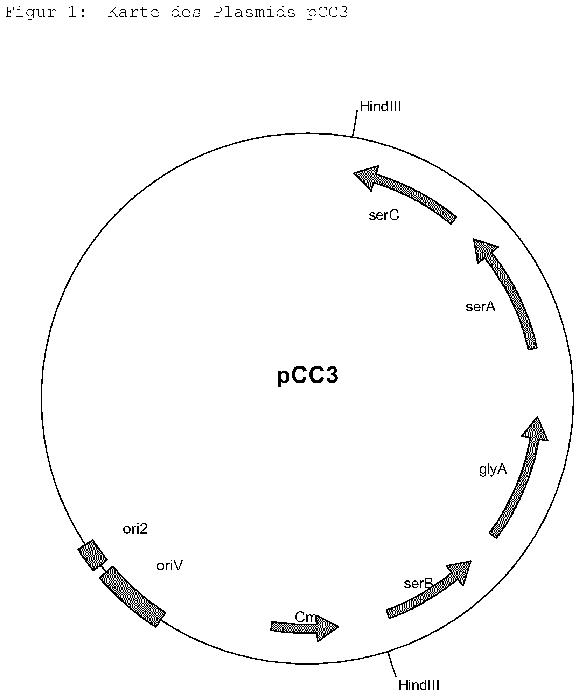

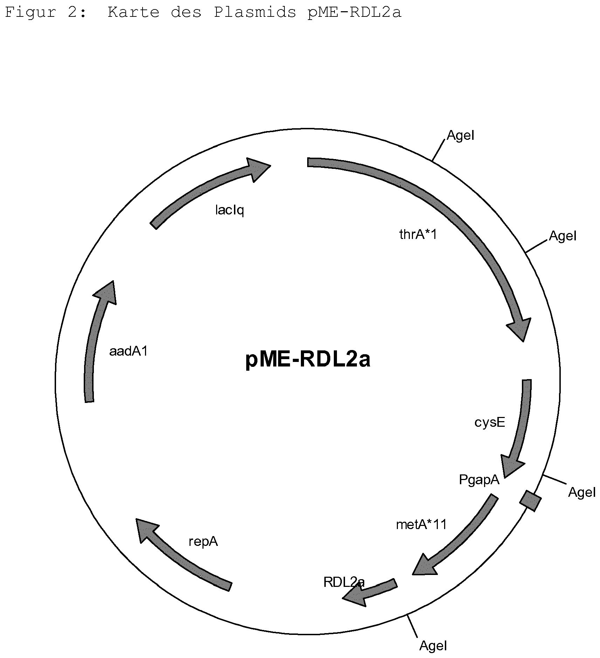

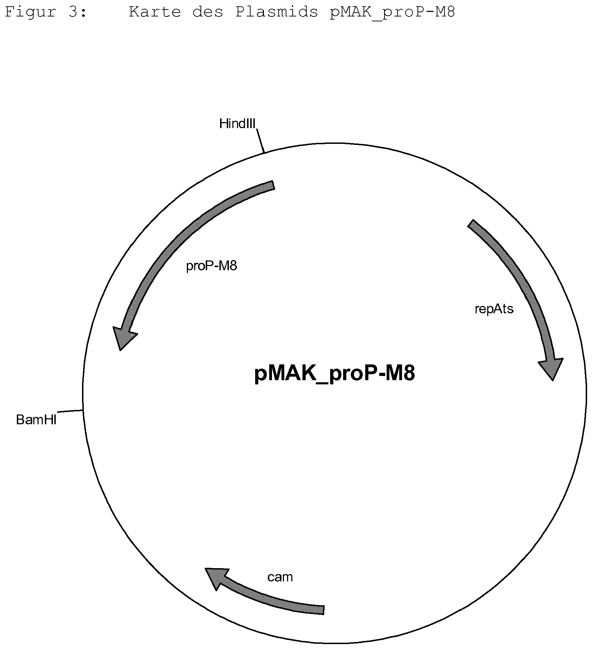

Das Plasmid pMAK705 wurde mit den Restriktionsendonucleasen HindIII und BamHI gespalten, mit Alkalischer Phosphatase dephosphoryliert (Alkaline Phosphatase, Calf Intestinal, Fa. New England Biolabs, Frankfurt a.M.) und über ein QIAquick PCR Purification Kit (Fa. Qiagen, Hilden) aufgereinigt. Anschließend wurde das Plasmid mit den jeweiligen proP-Fragmenten gemischt und mittels der Quick DNA-Ligase (New England BioLabs, Frankfurt, Germany) ligiert. Die Ligationsansätze wurden in E.coli DH5α (Grant et al.; Proceedings of the National Academy of Sciences USA, 87 (1990) 4645-4649) transformiert. Korrekte Plasmidklone wurden durch 20 mg/l Chloramphenicol selektioniert und per Restriktionsspaltung sowie anschließender Sequenzierung der Inserts identifiziert. Die so erhaltenen Plasmide wurden mit pMAK_proP-M4, pMAK_proP-M6 und pMAK_proP-M8 bezeichnet. Beispielhaft ist pMAK_proP-M8 in Figur 3 dargestellt.

### Beispiel 6

### Mutagenese des proP-Gens im E.coli Stamm ECM2

In dem L-Methionin produzierenden E.coli Stamm ECM2 (siehe Beispiel 1) wurde das chromosomale *proP*-Wildtyp-Allel jeweils gegen die eine Methionin-Resistenz vermittelnden *proP*-Allele M4, M6 und M8 ausgetauscht. Dazu wurde der Stamm per Elektroporation jeweils mit den Plasmiden pMAK_proP-M4, pMAK_proP-M6 und pMAK_proP-M8 (siehe Beispiel 5) transformiert. Die pMAK-Plasmide besitzen ein Chloramphenicol-Resistenzgen und einen temperatursensitiven Replikationsursprung. Das Plasmid wird bei 30°C von E.coli repliziert, bei 44°C jedoch nicht. Die Transformationsansätze wurden jeweils auf LB-Agar mit 20mg/l Chloramphenicol ausplattiert lund 40 Stunden bei 30°C inkubiert. Anschließend wurden mit einer Impföse Zellen aufgenommen, in LB-Medium resuspendiert und mit LB-Medium 10000-fach verdünnt. 100µl der Verdünnung wurden auf LB-Agar mit 20mg/l Chloramphenicol ausplattiert und weitere 24 Stunden bei 44°C inkubiert. Dadurch wurden Kolonien selektioniert, bei denen die Plasmide pMAK_proP-M4, pMAK_proP-M6 bzw. pMAK_proP-M8 jeweils chromosomal integriert waren. Jeweils eine dieser Kolonien wurde auf LB-Agar mit 20mg/l Chloramphenicol mit einer Impföse vereinzelt und für 24 Stunden bei 44°C inkubiert. Der Nachweis der eingebrachten Mutationen erfolgte mittels Standard PCR-Methoden (Innis et al. (1990) PCR Protocols. A guide to methods and applications, Academic Press) mit dem folgenden Primerpaar (siehe Beispiel 11):
proPmut1(HindIII) (SEQ ID NO: 33)
   5' GTCAAAGCTT ATATGGTCGCCAGAAGATCC 3'
proPmut2(BamHI) (SEQ ID NO: 34)
   5' GTCAGGATCC TCAGCCGCATTACACAGTTG 3'

Das jeweils resultierende PCR-Produkt wurde mit beiden Primern bei GATC (Konstanz, Deutschland) sequenziert. Alle drei proP-Allele konnten jeweils in den E.coli Stamm ECM2 übertragen werden, die resultierenden Stämme wurden mit ECM2_proP-M4, ECM2_proP-M6 und ECM2_proP-M8 bezeichnet.

### Beispiel 7

### Transformation der Stämme ECM2 proP-M4, ECM2 proP-M6 und ECM2 proP-M8 mit den Produktionsplasmiden

Die Stämme ECM2_proP-M4, ECM2_proP-M6 und ECM2_proP-M8 wurden mit dem Produktionsplasmid pCC3 aus Beispiel 1.5 transformiert und die Transformanden mit 20 mg/l Chloramphenicol selektioniert. Anschließend wurden die Zellen mit dem Plasmid pME-RDL2a aus Beispiel 1.6 transformiert und die resultierenden Transformanden mit 20 mg/l Chloramphenicol und 100 mg/l Streptomycin selektioniert. Die resultierenden Stämme wurden mit ECM2_proP-M4/pCC3/pME-RDL2a, ECM2_proP-M6/pCC3/pME-RDL2a und ECM2_proP-M8/pCC3/pME-RDL2a bezeichnet.

### Beispiel 8

### Leistungstest im Schüttelkolbenversuch

Die Leistungsfähigkeit der *E.coli* L-Methionin-Produktionsstämme ECM2_proP-M4/pCC3/pME-RDL2a, ECM2_proP-M6/pCC3/pME-RDL2a und ECM2_proP-M8/pCC3/pME-RDL2a wurde durch Produktionsversuche in 100 ml Erlenmeyer-Kolben bewertet. Als Vorkulturen wurden jeweils 10 ml Vorkulturmedium (10% LB-Medium mit 2,5 g/l Glucose und 90% Minimalmedium PC1) mit 100µl Zellkultur beimpft und 10 Stunden bei 37°C kultiviert. Hiermit wurden anschließend je 10 ml PC1-Minimalmedium (siehe Tabelle 1 in Beispiel 1) auf eine OD 600 nm von 0,2 (Eppendorf Bio-Photometer; Eppendorf AG, Hamburg, Deutschland) beimpft und für 24 Stunden bei 37°C kultiviert. Die extrazelluläre L-Methionin-Konzentration wurde mit einem Aminosäureanalysator (Sykam GmbH, Eresing, Deutschland) durch Ionenaustauschchromatographie und Nachsäulenderivatisierung mit Ninhydrindetektion bestimmt. Die extrazelluläre Glucose-Konzentration wurde mit einem YSI 2700 Select Glucose Analyzer (YSI Life Sciences, Yellow Springs, Ohio, USA) bestimmt. Die Ergebnisse sind in Tabelle 5 dargestellt. Nach 24 Stunden war die Glucose in beiden Kulturen vollständig verbraucht.

**Tabelle 5: L-Methioninkonzentrationen in den Fermentationsbrühen der untersuchten E. coli Stämme**

| Stamm | OD(600nm) | L-Methionin (g/l) |
|---|---|---|
| ECM2/pCC3/pME-RDL2a | 3,27 | 1,71 |
| ECM2_proP-M4/pCC3/pME-RDL2a | 3,38 | 1,83 |
| ECM2_proP-M6/pCC3/pME-RDL2a | 3,04 | 1,87 |
| ECM2_proP-M8/pCC3/pME-RDL2a | 3,22 | 1,83 |

## Patentansprüche

1. Verfahren zur Herstellung von L-Aminosäuren oder von L-Aminosäure enthaltenden Futtermitteladditiven durch Fermentation eines Mikroorganismus der Familie der Enterobacteriaceae, **dadurch gekennzeichnet, dass** man einen Mikroorganismus einsetzt, in dem das *proP*-Gen abgeschwächt ist, so dass die Aktivität oder Konzentration des Proteins ProP auf 0 bis 75 % der Aktivität oder Konzentration des Proteins im für das entsprechende Protein nicht rekombinanten Mikroorganismus herabgesenkt wurde.

2. Verfahren nach Anspruch 1, **dadurch gekennzeichnet, dass** das *proP*-Gen ausgeschaltet ist.

3. Verfahren nach einem der Ansprüche 1 oder 2, **dadurch gekennzeichnet, dass** der Mikroorganismus die L-Aminosäure überproduziert.

4. Verfahren nach einem der vorhergehenden Ansprüche, **dadurch gekennzeichnet, dass** der Mikroorganismus die L-Aminosäure im Medium anreichert.

5. Verfahren nach einem der Ansprüche 1 bis 4, **dadurch gekennzeichnet, dass** der Mikroorganismus die L-Aminosäure in den Zellen anreichert.

6. Verfahren nach einem der vorhergehenden Ansprüche, **dadurch gekennzeichnet, dass** es sich bei der L-Aminosäure um eine schwefelhaltige Aminosäure, vorzugsweise um L-Methionin, L-Cystein, L-Cystin, L-Homocystein oder L-Homocystin, handelt.

7. Verfahren nach Anspruch 6, **dadurch gekennzeichnet, dass** es sich bei der schwefelhaltigen Aminosäure um L-Methionin handelt.

8. Verfahren nach einem der vorhergehenden Ansprüche, **dadurch gekennzeichnet, dass** der Mikroorganismus eine erhöhte Methionin-Toleranz im Vergleich zu dem Mikroorganismus ohne abgeschwächtes proP-Gen aufweist, wobei unter erhöhter Methionin-Toleranz zu verstehen ist, dass die Mikroorganismen dazu in der Lage sind noch bei einer L-Methionin-Konzentration von 50 Gramm pro Liter zu wachsen.

9. Verfahren nach einem der vorhergehenden Ansprüche, **dadurch gekennzeichnet, dass** es sich bei dem Mikroorganismus aus der Familie der Enterobacteriaceae um ein Bakterium der Gattungen Escherichia, Erwinia, Providencia oder Serratia, insbesondere der Gattung Escherichia, besonders bevorzugt um Escherichia coli, handelt.

10. Verfahren nach einem der vorhergehenden Ansprüche, **dadurch gekennzeichnet, dass** es sich bei dem proP-Gen um ein Gen mit einer Sequenzidentität von mindestens 80 %, vorzugsweise von mindestens 90 %, insbesondere von mindestens 95 %, vor allem von mindestens 98, 99 oder 100 %, in Bezug auf die Polynukleotidesequenzen gemäß SEQ ID NO: 1, 3, 5 oder 7, besonders bevorzugt in Bezug auf die Polynukleotidsequenz gemäß SEQ ID NO: 1, handelt.

11. Verfahren nach einem der vorhergehenden Ansprüche, **dadurch gekennzeichnet, dass** es sich bei dem abgeschwächten *proP-*Gen um ein Polynukleotid handelt, das eine Sequenzidentität von mindestens 80 %, vorzugsweise mindestens 90 %, besonders bevorzugt mindestens 95 %, insbesondere mindestens 98, 99 oder 100 % in Bezug auf die Sequenz des Polynukleotids gemäß SEQ ID NO: 1 aufweist und weiterhin gegenüber dem Polynukleotid gemäß SEQ ID NO: 1 zwingend eine oder mehrere der folgenden Mutationen aufweist:
a. Austausch des für L-Arginin an Position 324 gemäß SEQ ID NO: 2 oder einer vergleichbaren Position der Aminosäuresequenz kodierenden Tripletts gegen ein Triplett, das für eine Aminosäure ausgewählt aus der Gruppe L-Leucin, L-Isoleucin und L-Valin, bevorzugt L-Leucin, kodiert;
b. Austausch des für L-Tyrosin an Position 467 gemäß SEQ ID NO: 2 oder einer vergleichbaren Position der Aminosäuresequenz kodierenden Tripletts gegen ein Triplett, das für eine Aminosäure ausgewählt aus der Gruppe L-Lysin, L-Arginin und L-Histidin, bevorzugt L-Histidin, kodiert;
c. Austausch des für L-Glutaminsäure an Position 412 gemäß SEQ ID NO: 2 oder eine vergleichbaren Position der Aminosäuresequenz kodierenden Tripletts gegen ein Triplett, das für ein Stopp-Codon kodiert;
d. Deletion der Nukleobase Adenin an Position 854 des *proP-*Gens gemäß SEQ ID NO:1;
e. Deletion einer oder mehrerer der Nukleobasen von Position 1173 bis 1223, bevorzugt Deletion sämtlicher Nukleobasen von Position 1173 bis 1223 des *proP*-Gens gemäß SEQ ID NO:1;
f. Insertion der Nukleobase Cytosin an Position 842 des *proP*-Gens gemäß SEQ ID NO:1;
g. Insertion von einer oder mehreren Nukleobase(n) an Position 973, bevorzugt Insertion von 19 Nukleobasen an Position 973 des *proP*-Gens gemäß SEQ ID NO:1;
h. Insertion von einer oder mehreren Nukleobase(n) an Position 183, bevorzugt Insertion von 1359 Nukleobasen an Position 183 des proP-Gens gemäß SEQ ID NO:1.

12. Verfahren nach einem der vorhergehenden Ansprüche, **dadurch gekennzeichnet, dass** die Fermentation in einem Medium erfolgt, das eine anorganische Schwefelquelle enthält.

13. Verfahren nach einem der vorhergehenden Ansprüche, **dadurch gekennzeichnet, dass** die L-Aminosäure in der erhaltenen Fermentationsbrühe angereichert und anschließend gegebenenfalls isoliert, gesammelt und/oder gereinigt wird.

14. Verfahren nach einem der vorhergehenden Ansprüche, **dadurch gekennzeichnet, dass** man die L-Aminosäure gemeinsam mit Bestandteilen aus der Fermentationsbrühe und/oder Biomasse isoliert oder sammelt.

15. Mikroorganismus aus der Familie der Enterobacteriaceae, der ein abgeschwächtes proP-Gen enthält, **dadurch gekennzeichnet, dass** er L-Methionin überproduziert und vorzugsweise ins Medium sezerniert,
**dadurch gekennzeichnet, dass** er eines oder mehrere der Merkmale ausgewählt aus der folgenden Gruppe besitzt:
a) überexprimiertes Polynukleotid, das für eine oder mehrere Komponenten des Thiosulfat-/Sulfat-Transportsystems CysPUWA (EC 3.6.3.25) kodiert,
b) überexprimiertes Polynukleotid, das für eine 3'-Phosphoadenosin 5'-Phosphosulfat Reduktase CysH (EC 1.8.4.8) kodiert,
c) überexprimiertes Polynukleotid, das für eine oder mehrere Komponenten der Sulfit Reduktase CysJI (EC 1.8.1.2) kodiert,
d) überexprimiertes Polynukleotid, das für eine Cystein Synthase A CysK (EC 2.5.1.47) kodiert,
e) überexprimiertes Polynukleotid, das für eine Cystein Synthase B CysM (EC 2.5.1.47) kodiert,
f) überexprimiertes Polynukleotid, das für eine Serin Acetyltransferase CysE (EC 2.3.1.30) kodiert,
g) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
h) überexprimiertes Polynukleotid, das für eine Lipoyl Synthase LipA (EC 2.8.1.8) kodiert,
i) überexprimiertes Polynukleotid, das für eine Lipoyl-Protein Ligase LipB (EC 2.3.1.181) kodiert,
j) überexprimiertes Polynukleotid, das für eine Phosphoglycerat Dehydrogenase SerA (EC 1.1.1.95) kodiert,
k) überexprimiertes Polynukleotid, das für eine 3-Phosphoserin Phosphatase SerB (EC 3.1.3.3) kodiert,
l) überexprimiertes Polynukleotid, das für eine 3-Phosphoserin/Phosphohydroxythreonin Aminotransferase SerC (EC 2.6.1.52) kodiert,
m) überexprimiertes Polynukleotid, das für eine Serin Hydroxymethyltransferase GlyA (EC 2.1.2.1) kodiert,
n) überexprimiertes Polynukleotid, das für eine Aspartokinase I und Homoserin Dehydrogenase I ThrA (EC 2.7.2.4, EC 1.1.1.3) kodiert,
o) verstärkte Enzymaktivität der Aspartatkinase, insbesondere ein überexprimiertes Polynukleotid, das für eine Aspartatkinase LysC (EC 2.7.2.4) kodiert,
p) überexprimiertes Polynukleotid, das für eine Homoserin-Dehydrogenase Hom (EC 1.1.1.3) kodiert,
q) überexprimiertes Polynukleotid, das für eine Homoserin O-Acetyltransferase MetX (EC 2.3.1.31) kodiert,
r) überexprimiertes Polynukleotid, das für eine Homoserin O-Succinlytransferase MetA (EC 2.3.1.46) kodiert,
s) überexprimiertes Polynukleotid, das für eine Cystathionin gamma-Synthase MetB (EC 2.5.1.48) kodiert,
t) überexprimiertes Polynukleotid, das für eine β-C-S-Lyase AecD (EC 4.4.1.8, auch als beta-Lyase bezeichnet) kodiert,
u) überexprimiertes Polynukleotid, das für eine Cystathionin beta-Lyase MetC (EC 4.4.1.8) kodiert,
v) überexprimiertes Polynukleotid, das für eine B12 unabhängige Homocystein S-Methyltransferase MetE (EC 2.1.1.14) kodiert,
w) überexprimiertes Polynukleotid, das für eine B12 abhängige Homocystein S-Methyltransferase MetH (EC 2.1.1.13) kodiert,
x) überexprimiertes Polynukleotid, das für eine Methylentetrahydrofolat Reduktase MetF (EC 1.5.1.20) kodiert,
y) überexprimiertes Polynukleotid, das für eine oder mehrere Komponenten des L-Methionin Exporters BrnFE aus Corynebacterium glutamicum kodiert,
z) überexprimiertes Polynukleotid, das für eine oder mehrere Komponenten des Valin Exporter YgaZH aus E-scherichia coli (b2682, b2683) kodiert,
aa) überexprimiertes Polynukleotid, das für den putativen Transporter YjeH aus Escherichia coli (b4141) kodiert,
bb) überexprimiertes Polynukleotid, das für eine oder mehrere Komponenten der Pyridin Nucleotid Transhydrogenase PntAB (EC 1.6.1.2) kodiert,
cc) überexprimiertes Polynukleotid, das für eine O-Succinylhomoserine Sulfhydrylase MetZ (EC 2.5.1.48) kodiert,
dd) überexprimiertes Polynukleotid, das für eine Phosphoenolpyruvate Carboxylase Pyc (EC 4.1.1.31) kodiert,
ee) überexprimiertes Polynukleotid, das für eine Thiosulfat-Sulfurtransferase RDL2 (EC 2.8.1.1) kodiert,
ff) überexprimiertes Polynukleotid, das für eine Thiosulfat-Thiol Sulfurtransferase (EC 2.8.1.3) kodiert,
gg) überexprimiertes Polynukleotid, das für eine Thiosulfat-Dithiol Sulfurtransferase (EC 2.8.1.5) kodiert,
hh) erhöhte Aktivität der Homoserin O-Succinyltransferase MetA (EC 2.3.1.46),
ii) erhöhte Aktivität der Serin Acetyltransferase CysE (EC 2.3.1.30),
jj) Abschwächung des für den Transkriptionsregulators der L-Methioninbiosynthese (MetJ) kodierenden Gens metJ (b3938, ECK3930),
kk) Abschwächung des für die Glucose-6-Phosphat-Isomerase (Pgi, EC Nr. 5.3.1.9) kodierenden Gens pgi (b4025, ECK4017),
ll) Abschwächung des für die Homoserinkinase (ThrB, EC 2.7.1.39) kodierenden Gens thrB (b0003, ECK0003),
mm) Abschwächung des für die S-Adenosylmethionin Synthase (MetK, EC Nr. 2.5.1.6) kodierenden Gens metK (b2942, ECK2937),
nn) Abschwächung des für die Dihydrodipicolinat Synthase (DapA, EC Nr. 4.2.1.52) kodierenden Gens dapA (b2478, ECK2474),
oo) Abschwächung des für die Phosphoenolpyruvat Carboxykinase (Pck, EC Nr. 4.1.1.49) kodierenden Gens pck (b3403, ECK3390),
pp) Abschwächung des für die Formyltetrahydrofolat Hydrolase (PurU, EC Nr. 3.5.1.10) kodierenden Gens purU (b1232, ECK1227),
qq) Abschwächung des für die Pyruvatkinase II (PykA, EC Nr. 2.7.1.40) kodierenden Gens pykA (b1854, ECK1855) rr) Abschwächung des für die Pyruvatkinase I (PykF, EC 2.7.1.40) kodierenden Gens pykF (b1676, ECK1672),
ss) Abschwächung des für eine Untereinheit des L-Methionintransporters (MetQNI) kodierenden Gens metQ (b0197, ECK0197),
tt) Abschwächung des für eine Untereinheit des L-Methionintransporters (MetQNI) kodierenden Gens metI (b0198, ECK0198),
uu) Abschwächung des für eine Untereinheit des L-Methionintransporters (MetQNI) kodierenden Gens metN (b0199, ECK0199),
vv) Abschwächung des für die Deoxycytidin 5'-Triphosphat Deaminase (Dcd, EC Nr. 3.5.4.13) kodierenden Gens dcd (b2065, ECK2059),
ww) Abschwächung des für die putative N-Acyltransferase (YncA, Metabo-lic Explorer WO2010/020681) kodierenden Gens yncA (b1448, ECK1442),
xx) Abschwächung des für den Sigma Faktor RpoS kodierenden Gens rpoS (b2741, ECK2736),
yy) Abschwächung des Regulatorproteins MetJ,
zz) Abschwächung der S-Adenosylmethionin Synthase MetK;
wobei unter "Abschwächung" zu verstehen ist, dass die Aktivität oder Konzentration des entsprechenden Proteins auf 0 bis 75 % der Aktivität oder Konzentration des Proteins im für das entsprechende Protein nicht rekombinanten Mikroorganismus herabgesenkt wurde und wobei unter "Überexpression", "Verstärkung" oder "erhöhte Aktivität" zu verstehen ist, dass die Aktivität des entsprechenden Proteins gegenüber dem Wildtypstamm um einen Faktor von mindestens 2 gesteigert ist.

16. Mikroorganismus nach Anspruch 15, **dadurch gekennzeichnet, dass** er L-Methionin in den Zellen anreichert.

17. Mikroorganismus nach Anspruch 15, **dadurch gekennzeichnet, dass** er L-Methionin im Medium anreichert.

18. Mikroorganismus nach einem der Ansprüche 15 bis 17, **dadurch gekennzeichnet, dass** das *proP*-Gen ausgeschaltet ist.

19. Mikroorganismus nach einem der Ansprüche 15 bis 18, **dadurch gekennzeichnet, dass** er eine erhöhte Methionin-Toleranz im Vergleich zu dem Mikroorganismus ohne abgeschwächtes *proP-*Gen aufweist, wobei unter erhöhter Methionin-Toleranz zu verstehen ist, dass die Mikroorganismen dazu in der Lage sind noch bei einer L-Methionin-Konzentration von 50 Gramm pro Liter zu wachsen.

20. Mikroorganismus nach einem der Ansprüche 15 bis 19, **dadurch gekennzeichnet, dass** es sich bei dem Mikroorganismus aus der Familie der Enterobacteriaceae um ein Bakterium der Gattungen Escherichia, Erwinia, Providencia oder Serratia, vorzugsweise der Gattung Escherichia, besonders bevorzugt um Escherichia coli, handelt.

21. Mikroorganismus nach einem der Ansprüche 15 bis 20, **dadurch gekennzeichnet, dass** es sich bei dem proP-Gen um ein Gen mit einer Sequenzidentität von mindestens 80 %, vorzugsweise von mindestens 90 %, insbesondere von mindestens 95 %, vor allem von mindestens 98, 99 oder 100 %, in Bezug auf die Polynukleotidesequenz gemäß SEQ ID NO: 1, gemäß SEQ ID NO: 3, gemäß SEQ ID NO: 5 oder gemäß SEQ ID NO: 7, besonders bevorzugt gemäß SEQ ID NO: 1, handelt.

22. Mikroorganismus nach einem der Ansprüche 15 bis 21, **dadurch gekennzeichnet, dass** es sich bei dem abgeschwächten *proP-*Gen um ein Polynukleotid handelt, das eine Sequenzidentität von mindestens 80 %, vorzugsweise mindestens 90 %, insbesondere mindestens 95 %, vor allem von mindestens 98, 99 oder 100 %, in Bezug auf die Gesamtsequenz des Polynukleotids gemäß SEQ ID NO: 1 aufweist und weiterhin gegenüber dem Polynukleotid gemäß SEQ ID NO: 1 zwingend eine oder mehrere der folgenden Mutationen aufweist:
a. Austausch des für L-Arginin an Position 324 gemäß SEQ ID NO: 2 oder einer vergleichbaren Position der Aminosäuresequenz kodierenden Tripletts gegen ein Triplett, das für eine Aminosäure ausgewählt aus der Gruppe L-Leucin, L-Isoleucin und L-Valin, bevorzugt L-Leucin, kodiert;
b. Austausch des für L-Tyrosin an Position 467 gemäß SEQ ID NO: 2 oder einer vergleichbaren Position der Aminosäuresequenz kodierenden Tripletts gegen ein Triplett, das für eine Aminosäure ausgewählt aus der Gruppe L-Lysin, L-Arginin und L-Histidin, bevorzugt L-Histidin, kodiert;
c. Austausch des für L-Glutaminsäure an Position 412 gemäß SEQ ID NO: 2 oder eine vergleichbaren Position der Aminosäuresequenz kodierenden Tripletts gegen ein Triplett, das für ein Stopp-Codon kodiert;
d. Deletion der Nukleobase Adenin an Position 854 des *proP-*Gens gemäß SEQ ID NO:1;
e. Deletion einer oder mehrerer der Nukleobasen von Position 1173 bis 1223, bevorzugt Deletion sämtlicher Nukleobasen von Position 1173 bis 1223 des *proP*-Gens gemäß SEQ ID NO:1;
f. Insertion der Nukleobase Cytosin an Position 842 des *proP*-Gens gemäß SEQ ID NO:1;
g. Insertion von einer oder mehreren Nukleobase(n) an Position 973, bevorzugt Insertion von 19 Nukleobasen an Position 973 des *proP*-Gens gemäß SEQ ID NO:1;
h. Insertion von einer oder mehreren Nukleobase(n) an Position 183, bevorzugt Insertion von 1359 Nukleobasen an Position 183 des *proP*-Gens gemäß SEQ ID NO:1.

23. Polynukleotid mit einer Identität von mindestens 80 %, vorzugsweise mindestens 90 %, insbesondere mindestens 95 %, vor allem mindestens 98, 99 oder 100 %, in Bezug auf die Sequenz gemäß SEQ ID NO: 1, **dadurch gekennzeichnet, dass** das Polynukleotid zwingend eine oder mehrere Mutationen gegenüber dem Polynukleotid gemäß SEQ ID NO: 1 aufweist, ausgewählt aus:
a. Austausch des für L-Arginin an Position 324 gemäß SEQ ID NO: 2 oder einer vergleichbaren Position der Aminosäuresequenz kodierenden Tripletts gegen ein Triplett, das für eine Aminosäure ausgewählt aus der Gruppe L-Leucin, L-Isoleucin und L-Valin, bevorzugt L-Leucin, kodiert;
b. Austausch des für L-Tyrosin an Position 467 gemäß SEQ ID NO: 2 oder einer vergleichbaren Position der Aminosäuresequenz kodierenden Tripletts gegen ein Triplett, das für eine Aminosäure ausgewählt aus der Gruppe L-Lysin, L-Arginin und L-Histidin, bevorzugt L-Histidin, kodiert;
c. Austausch des für L-Glutaminsäure an Position 412 gemäß SEQ ID NO: 2 oder eine vergleichbaren Position der Aminosäuresequenz kodierenden Tripletts gegen ein Triplett, das für ein Stopp-Codon kodiert;
d. Deletion der Nukleobase Adenin an Position 854 des *proP-*Gens gemäß SEQ ID NO:1;
e. Deletion einer oder mehrerer der Nukleobasen von Position 1173 bis 1223, bevorzugt Deletion sämtlicher Nukleobasen von Position 1173 bis 1223 des *proP*-Gens gemäß SEQ ID NO:1;
f. Insertion der Nukleobase Cytosin an Position 842 des *proP*-Gens gemäß SEQ ID NO:1;
g. Insertion von einer oder mehreren Nukleobase(n) an Position 973, bevorzugt Insertion von 19 Nukleobasen an Position 973 des *proP*-Gens gemäß SEQ ID NO:1;
h. Insertion von einer oder mehreren Nukleobase(n) an Position 183, bevorzugt Insertion von 1359 Nukleobasen an Position 183 des *proP*-Gens gemäß SEQ ID NO:1.

24. Polynukleotid gemäß Anspruch 23, **dadurch gekennzeichnet, dass** es mit einem oder mehreren der Polynukleotide ausgewählt aus der Gruppe Polynukleotid komplementär zu SEQ ID NO:1, Polynukleotid komplementär zu SEQ ID NO:3 und Polynnukleotid komplementär zu SEQ ID NO:5, bevorzugt Polynukleotid komplementär zu SEQ ID NO:1, hybridisiert.

25. Vektor, der ein Polynukleotid gemäß Anspruch 23 oder 24 enthält.

26. Polypeptid mit einer Identität von mindestens 80 %, vorzugsweise mindestens 90 %, insbesondere mindestens 95 %, vor allem mindestens 98, 99 oder 100 %, in Bezug auf die Sequenz gemäß SEQ ID NO: 2, **dadurch gekennzeichnet, dass** das Polypeptid zwingend eine oder mehrere Mutationen gegenüber dem Polypeptid gemäß SEQ ID NO: 2 aufweist, ausgewählt aus:
a. Austausch des L-Arginin an Position 324 gemäß SEQ ID NO: 2 oder einer vergleichbaren Position der Aminosäuresequenz gegen eine Aminosäure ausgewählt aus der Gruppe L-Leucin, L-Isoleucin und L-Valin, bevorzugt L-Leucin;
b. Austausch des L-Tyrosin an Position 467 gemäß SEQ ID NO: 2 oder einer vergleichbaren Position der Aminosäuresequenz gegen eine Aminosäure ausgewählt aus der Gruppe L-Lysin, L-Arginin und L-Histidin, bevorzugt L-Histidin;
c. Deletion der 17 Aminosäuren von Position 392 bis 408 gemäß SEQ ID NO: 2 oder einer vergleichbaren Position der Aminosäuresequenz;
d. Deletion von bis zu 420 Aminosäuren des C-Terminus in Bezug auf die Sequenz gemäß SEQ ID NO: 2, vorzugsweise Deletion von 88, 163, 202, 212 oder 420 Aminosäuren des C-Terminus der Aminosäursequenz gemäß SEQ ID NO: 2.

27. Rekombinanter Mikroorganismus, der ein Polynukleotid gemäß Anspruch 23 oder 24 und/oder einen Vektor gemäß Anspruch 25 und/oder ein Polypeptid gemäß Anspruch 26 enthält.

## Claims

1. Method for producing L-amino acids or L-amino acid-containing feed additives by fermentation of a microorganism of the Enterobacteriaceae family, **characterized in that** use is made of a microorganism in which the *proP* gene is attenuated, with the result that the activity or concentration of the protein ProP has been lowered to 0 to 75% of the activity or concentration of the protein in the microorganism which is not recombinant for the corresponding protein.

2. Method according to Claim 1, **characterized in that** the *proP* gene is switched off.

3. Method according to either of Claims 1 and 2, **characterized in that** the microorganism overproduces the L-amino acid.

4. Method according to any of the preceding claims, **characterized in that** the microorganism enriches the L-amino acid in the medium.

5. Method according to any of Claims 1 to 4, **characterized in that** the microorganism enriches the L-amino acid in the cells.

6. Method according to any of the preceding claims, **characterized in that** the L-amino acid is a sulfur-containing amino acid, preferably L-methionine, L-cysteine, L-cystine, L-homocysteine or L-homocystine.

7. Method according to Claim 6, **characterized in that** the sulfur-containing amino acid is L-methionine.

8. Method according to any of the preceding claims, **characterized in that** the microorganism has an increased methionine tolerance in comparison with the microorganism without attenuated *proP* gene, wherein increased methionine tolerance is to be understood to mean that the microorganisms are capable of still growing at an L-methionine concentration of 50 grams per litre.

9. Method according to any of the preceding claims, **characterized in that** the microorganism from the Enterobacteriaceae family is a bacterium of the genera Escherichia, Erwinia, Providencia or Serratia, especially of the genera Escherichia, and is particularly preferably Escherichia coli.

10. Method according to any of the preceding claims, **characterized in that** the *proP* gene is a gene having a sequence identity of at least 80%, preferably of at least 90%, especially of at least 95%, particularly of at least 98%, 99% or 100%, with respect to the polynucleotide sequences as per SEQ ID NO: 1, 3, 5 or 7, particularly preferably with respect to the polynucleotide sequence as per SEQ ID NO: 1.

11. Method according to any of the preceding claims, **characterized in that** the attenuated *proP* gene is a polynucleotide which has a sequence identity of at least 80%, preferably at least 90%, particularly preferably at least 95%, especially at least 98%, 99% or 100%, with respect to the sequence of the polynucleotide as per SEQ ID NO: 1 and which, in addition, must have one or more of the following mutations compared to the polynucleotide as per SEQ ID NO: 1:
a. substitution of the triplet encoding L-arginine at position 324 as per SEQ ID NO: 2 or a comparable position of the amino acid sequence with a triplet encoding an amino acid selected from the group consisting of L-leucine, L-isoleucine and L-valine, preferably L-leucine;
b. substitution of the triplet encoding L-tyrosine at position 467 as per SEQ ID NO: 2 or a comparable position of the amino acid sequence with a triplet encoding an amino acid selected from the group consisting of L-lysine, L-arginine and L-histidine, preferably L-histidine;
c. substitution of the triplet encoding L-glutamic acid at position 412 as per SEQ ID NO: 2 or a comparable position of the amino acid sequence with a triplet encoding a stop codon;
d. deletion of the nucleobase adenine at position 854 of the *proP* gene as per SEQ ID NO: 1;
e. deletion of one or more of the nucleobases from position 1173 to 1223, preferably deletion of all nucleobases from position 1173 to 1223, of the *proP* gene as per SEQ ID NO: 1;
f. insertion of the nucleobase cytosine at position 842 of the *proP* gene as per SEQ ID NO: 1;
g. insertion of one or more nucleobases at position 973, preferably insertion of 19 nucleobases at position 973, of the *proP* gene as per SEQ ID NO: 1;
h. insertion of one or more nucleobases at position 183, preferably insertion of 1359 nucleobases at position 183, of the *proP* gene as per SEQ ID NO: 1.

12. Method according to any of the preceding claims, **characterized in that** the fermentation takes place in a medium containing an inorganic sulfur source.

13. Method according to any of the preceding claims, **characterized in that** the L-amino acid is enriched in the fermentation broth obtained and is then, if necessary, isolated, accumulated and/or purified.

14. Method according to any of the preceding claims, **characterized in that** the L-amino acid is isolated or accumulated together with constituents from the fermentation broth and/or biomass.

15. Microorganism from the Enterobacteriaceae family, which microorganism contains an attenuated *proP* gene, **characterized in that** said microorganism overproduces L-methionine and preferably secretes it into the medium,
**characterized in that** said microorganism has one or more of the features selected from the following group:
a) overexpressed polynucleotide encoding one or more components of the thiosulfate/sulfate transport system CysPUWA (EC 3.6.3.25),
b) overexpressed polynucleotide encoding a 3'-phosphoadenosine 5'-phosphosulfate reductase CysH (EC 1.8.4.8),
c) overexpressed polynucleotide encoding one or more components of sulfite reductase CysJI (EC 1.8.1.2),
d) overexpressed polynucleotide encoding a cysteine synthase A CysK (EC 2.5.1.47),
e) overexpressed polynucleotide encoding a cysteine synthase B CysM (EC 2.5.1.47),
f) overexpressed polynucleotide encoding a serine acetyltransferase CysE (EC 2.3.1.30),
g) overexpressed polynucleotide encoding one or more components of the glycine cleavage system GcvTHP-Lpd (EC 2.1.2.10, EC 1.4.4.2, EC 1.8.1.4),
h) overexpressed polynucleotide encoding a lipoyl synthase LipA (EC 2.8.1.8),
i) overexpressed polynucleotide encoding a lipoyl protein ligase LipB (EC 2.3.1.181),
j) overexpressed polynucleotide encoding a phosphoglycerate dehydrogenase SerA (EC 1.1.1.95),
k) overexpressed polynucleotide encoding a 3-phosphoserine phosphatase SerB (EC 3.1.3.3),
l) overexpressed polynucleotide encoding a 3-phosphoserine/phosphohydroxythreonine aminotransferase SerC (EC 2.6.1.52),
m) overexpressed polynucleotide encoding a serine hydroxymethyltransferase GlyA (EC 2.1.2.1),
n) overexpressed polynucleotide encoding an aspartokinase I and homoserine dehydrogenase I ThrA (EC 2.7.2.4, EC 1.1.1.3),
o) enhanced enzyme activity of aspartate kinase, especially an overexpressed polynucleotide encoding an aspartate kinase LysC (EC 2.7.2.4),
p) overexpressed polynucleotide encoding a homoserine dehydrogenase Horn (EC 1.1.1.3),
q) overexpressed polynucleotide encoding a homoserine O-acetyltransferase MetX (EC 2.3.1.31),
r) overexpressed polynucleotide encoding a homoserine O-succinyltransferase MetA (EC 2.3.1.46),
s) overexpressed polynucleotide encoding a cystathionine gamma-synthase MetB (EC 2.5.1.48),
t) overexpressed polynucleotide encoding a β-C-S-lyase AecD (EC 4.4.1.8, also referred to as beta-lyase),
u) overexpressed polynucleotide encoding a cystathionine beta-lyase MetC (EC 4.4.1.8),
v) overexpressed polynucleotide encoding a B12-independent homocysteine S-methyltransferase MetE (EC 2.1.1.14),
w) overexpressed polynucleotide encoding a B12-dependent homocysteine S-methyltransferase MetH (EC 2.1.1.13),
x) overexpressed polynucleotide encoding a methylene tetrahydrofolate reductase MetF (EC 1.5.1.20),
y) overexpressed polynucleotide encoding one or more components of the L-methionine exporter BrnFE from Corynebacterium glutamicum,
z) overexpressed polynucleotide encoding one or more components of the valine exporter YgaZH from Escherichia coli (b2682, b2683),
aa) overexpressed polynucleotide encoding the putative transporter YjeH from Escherichia coli (b4141),
bb) overexpressed polynucleotide encoding one or more components of pyridine nucleotide transhydrogenase PntAB (EC 1.6.1.2),
cc) overexpressed polynucleotide encoding an O-succinylhomoserine sulfhydrylase MetZ (EC 2.5.1.48),
dd) overexpressed polynucleotide encoding a phosphoenolpyruvate carboxylase Pyc (EC 4.1.1.31),
ee) overexpressed polynucleotide encoding a thiosulfate sulfurtransferase RDL2 (EC 2.8.1.1),
ff) overexpressed polynucleotide encoding a thiosulfate-thiol sulfurtransferase (EC 2.8.1.3),
gg) overexpressed polynucleotide encoding a thiosulfate-dithiol sulfurtransferase (EC 2.8.1.5),
hh) increased activity of homoserine O-succinyltransferase MetA (EC 2.3.1.46),
ii) increased activity of serine acetyltransferase CysE (EC 2.3.1.30),
jj) attenuation of the gene metJ (b3938, ECK3930) which encodes the transcription regulator of L-methionine biosynthesis (MetJ),
kk) attenuation of the gene pgi (b4025, ECK4017) which encodes glucose-6-phosphate isomerase (Pgi, EC No. 5.3.1.9),
ll) attenuation of the gene thrB (b0003, ECK0003) which encodes homoserine kinase (ThrB, EC 2.7.1.39),
mm) attenuation of the gene metK (b2942, ECK2937) which encodes S-adenosylmethionine synthase (MetK, EC No. 2.5.1.6),
nn) attenuation of the gene dapA (b2478, ECK2474) which encodes dihydrodipicolinate synthase (DapA, EC No. 4.2.1.52),
oo) attenuation of the gene pck (b3403, ECK3390) which encodes phosphoenolpyruvate carboxykinase (Pck, EC No. 4.1.1.49),
pp) attenuation of the gene purU (b1232, ECK1227) which encodes formyltetrahydrofolate hydrolase (PurU, EC No. 3.5.1.10),
qq) attenuation of the gene pykA (b1854, ECK1855) which encodes pyruvate kinase II (PykA, EC No. 2.7.1.40),
rr) attenuation of the gene pykF (b1676, ECK1672) which encodes pyruvate kinase I (PykF, EC No. 2.7.1.40),
ss) attenuation of the gene metQ (b0197, ECK0197) which encodes a subunit of the L-methionine transporter (MetQNI),
tt) attenuation of the gene metI (b0198, ECK0198) which encodes a subunit of the L-methionine transporter (MetQNI),
uu) attenuation of the gene metN (b0199, ECK0199) which encodes a subunit of the L-methionine transporter (MetQNI),
vv) attenuation of the gene dcd (b2065, ECK2059) which encodes deoxycytidine 5'-triphosphate deaminase (Dcd, EC No. 3.5.4.13),
ww) attenuation of the gene yncA (b1448, ECK1442) which encodes the putative N-acyltransferase (YncA, Metabolic Explorer WO2010/020681),
xx) attenuation of the gene rpoS (b2741, ECK2736) which encodes the sigma factor RpoS,
yy) attenuation of the regulator protein MetJ,
zz) attenuation of S-adenosylmethionine synthase MetK;
wherein "attenuation" is to be understood to mean that the activity or concentration of the corresponding protein has been lowered to 0 to 75% of the activity or concentration of the protein in the microorganism which is not recombinant for the corresponding protein and wherein "overexpression", "enhancement" or "increased activity" is to be understood to mean that the activity of the corresponding protein is raised by a factor of at least 2 compared to the wild-type strain.

16. Microorganism according to Claim 15, **characterized in that** it enriches L-methionine in the cells.

17. Microorganism according to Claim 15, **characterized in that** it enriches L-methionine in the medium.

18. Microorganism according to any of Claims 15 to 17, **characterized in that** the *proP* gene is switched off.

19. Microorganism according to any of Claims 15 to 18, **characterized in that** it has an increased methionine tolerance in comparison with the microorganism without attenuated *proP* gene, wherein increased methionine tolerance is to be understood to mean that the microorganisms are capable of still growing at an L-methionine concentration of 50 grams per litre.

20. Microorganism according to any of Claims 15 to 19, **characterized in that** the microorganism from the Enterobacteriaceae family is a bacterium of the genera Escherichia, Erwinia, Providencia or Serratia, preferably of the genera Escherichia, and is particularly preferably Escherichia coli.

21. Microorganism according to any of Claims 15 to 20, **characterized in that** the *proP* gene is a gene having a sequence identity of at least 80%, preferably of at least 90%, especially of at least 95%, particularly of at least 98%, 99% or 100%, with respect to the polynucleotide sequence as per SEQ ID NO: 1, as per SEQ ID NO: 3, as per SEQ ID NO: 5 or as per SEQ ID NO: 7, particularly preferably as per SEQ ID NO: 1.

22. Microorganism according to any of Claims 15 to 21, **characterized in that** the attenuated *proP* gene is a polynucleotide which has a sequence identity of at least 80%, preferably at least 90%, especially at least 95%, particularly of at least 98%, 99% or 100%, with respect to the entire sequence of the polynucleotide as per SEQ ID NO: 1 and which, in addition, must have one or more of the following mutations compared to the polynucleotide as per SEQ ID NO: 1:
a. substitution of the triplet encoding L-arginine at position 324 as per SEQ ID NO: 2 or a comparable position of the amino acid sequence with a triplet encoding an amino acid selected from the group consisting of L-leucine, L-isoleucine and L-valine, preferably L-leucine;
b. substitution of the triplet encoding L-tyrosine at position 467 as per SEQ ID NO: 2 or a comparable position of the amino acid sequence with a triplet encoding an amino acid selected from the group consisting of L-lysine, L-arginine and L-histidine, preferably L-histidine;
c. substitution of the triplet encoding L-glutamic acid at position 412 as per SEQ ID NO: 2 or a comparable position of the amino acid sequence with a triplet encoding a stop codon;
d. deletion of the nucleobase adenine at position 854 of the *proP* gene as per SEQ ID NO: 1;
e. deletion of one or more of the nucleobases from position 1173 to 1223, preferably deletion of all nucleobases from position 1173 to 1223, of the *proP* gene as per SEQ ID NO: 1;
f. insertion of the nucleobase cytosine at position 842 of the *proP* gene as per SEQ ID NO: 1;
g. insertion of one or more nucleobases at position 973, preferably insertion of 19 nucleobases at position 973, of the *proP* gene as per SEQ ID NO: 1;
h. insertion of one or more nucleobases at position 183, preferably insertion of 1359 nucleobases at position 183, of the *proP* gene as per SEQ ID NO: 1.

23. Polynucleotide having an identity of at least 80%, preferably at least 90%, especially at least 95%, particularly at least 98%, 99% or 100%, with respect to the sequence as per SEQ ID NO: 1, **characterized in that** the polynucleotide must have one or more mutations compared to the polynucleotide as per SEQ ID NO: 1, selected from:
a. substitution of the triplet encoding L-arginine at position 324 as per SEQ ID NO: 2 or a comparable position of the amino acid sequence with a triplet encoding an amino acid selected from the group consisting of L-leucine, L-isoleucine and L-valine, preferably L-leucine;
b. substitution of the triplet encoding L-tyrosine at position 467 as per SEQ ID NO: 2 or a comparable position of the amino acid sequence with a triplet encoding an amino acid selected from the group consisting of L-lysine, L-arginine and L-histidine, preferably L-histidine;
c. substitution of the triplet encoding L-glutamic acid at position 412 as per SEQ ID NO: 2 or a comparable position of the amino acid sequence with a triplet encoding a stop codon;
d. deletion of the nucleobase adenine at position 854 of the *proP* gene as per SEQ ID NO: 1;
e. deletion of one or more of the nucleobases from position 1173 to 1223, preferably deletion of all nucleobases from position 1173 to 1223, of the *proP* gene as per SEQ ID NO: 1;
f. insertion of the nucleobase cytosine at position 842 of the *proP* gene as per SEQ ID NO: 1;
g. insertion of one or more nucleobases at position 973, preferably insertion of 19 nucleobases at position 973, of the *proP* gene as per SEQ ID NO: 1;
h. insertion of one or more nucleobases at position 183, preferably insertion of 1359 nucleobases at position 183, of the *proP* gene as per SEQ ID NO: 1.

24. Polynucleotide according to Claim 23, **characterized in that** it hybridizes to one or more of the polynucleotides selected from the group consisting of polynucleotide complementary to SEQ ID NO: 1, polynucleotide complementary to SEQ ID NO: 3 and polynucleotide complementary to SEQ ID NO: 5, preferably polynucleotide complementary to SEQ ID NO: 1.

25. Vector containing a polynucleotide according to Claim 23 or 24.

26. Polypeptide having an identity of at least 80%, preferably at least 90%, especially at least 95%, particularly at least 98%, 99% or 100%, with respect to the sequence as per SEQ ID NO: 2, **characterized in that** the polypeptide must have one or more mutations compared to the polypeptide as per SEQ ID NO: 2, selected from:
a. substitution of the L-arginine at position 324 as per SEQ ID NO: 2 or a comparable position of the amino acid sequence with an amino acid selected from the group consisting of L-leucine, L-isoleucine and L-valine, preferably L-leucine;
b. substitution of the L-tyrosine at position 467 as per SEQ ID NO: 2 or a comparable position of the amino acid sequence with an amino acid selected from the group consisting of L-lysine, L-arginine and L-histidine, preferably L-histidine;
c. deletion of the 17 amino acids from position 392 to 408 as per SEQ ID NO: 2 or a comparable position of the amino acid sequence;
d. deletion of up to 420 amino acids of the C-terminus with respect to the sequence as per SEQ ID NO: 2, preferably deletion of 88, 163, 202, 212 or 420 amino acids of the C-terminus of the amino acid sequence as per SEQ ID NO: 2.

27. Recombinant microorganism containing a polynucleotide according to Claim 23 or 24 and/or a vector according to Claim 25 and/or a polypeptide according to Claim 26.

## Revendications

1. Procédé pour la production d'acides aminés L ou d'additifs pour l'alimentation pour animaux contenant des acides aminés L par fermentation d'un micro-organisme de la famille des Enterobacteriaceae, **caractérisé en ce qu'**on utilise un micro-organisme dans lequel le gène *proP* a été affaibli, de telle sorte que l'activité de la protéine ProP ou sa concentration a été abaissée jusqu'à 0 à 75% de l'activité de la protéine ProP ou sa concentration dans le micro-organisme non recombinant pour la protéine correspondante.

2. Procédé selon la revendication 1, **caractérisé en ce que** le gène *proP* est désactivé.

3. Procédé selon l'une quelconque des revendications 1 ou 2, **caractérisé en ce que** le micro-organisme surproduit l'acide aminé L.

4. Procédé selon l'une quelconque des revendications précédentes, **caractérisé en ce que** le micro-organisme enrichit l'acide aminé L dans le milieu.

5. Procédé selon l'une quelconque des revendications 1 à 4, **caractérisé en ce que** le micro-organisme enrichit l'acide aminé L dans les cellules.

6. Procédé selon l'une quelconque des revendications précédentes, **caractérisé en ce qu'**il s'agit, pour l'acide aminé L, d'un acide aminé contenant du soufre, de préférence de la L-méthionine, de la L-cystéine, de la L-cystine, de la L-homocystéine ou de la L-homocystine.

7. Procédé selon la revendication 6, **caractérisé en ce qu'**il s'agit, pour l'acide aminé contenant du soufre, de la L-méthionine.

8. Procédé selon l'une quelconque des revendications précédentes, **caractérisé en ce que** le micro-organisme présente une tolérance augmentée à la méthionine par rapport au micro-organisme sans gène *proP* affaibli, une tolérance augmentée à la méthionine signifiant que les micro-organismes sont encore en mesure de croître à une concentration en L-méthionine de 50 grammes par litre.

9. Procédé selon l'une quelconque des revendications précédentes, **caractérisé en ce qu'**il s'agit, pour le micro-organisme de la famille des Enterobacteriaceae, d'une bactérie des genres Escherichia, Erwinia, Providencia ou Serratia, en particulier du genre Escherichia, de manière particulièrement préférée d'Escherichia coli.

10. Procédé selon l'une quelconque des revendications précédentes, **caractérisé en ce qu'**il s'agit, pour le gène *proP,* d'un gène présentant une identité de séquence d'au moins 80%, de préférence d'au moins 90%, en particulier d'au moins 95%, surtout d'au moins 98, 99 ou 100%, par rapport aux séquences polynucléotidiques selon les séquences SEQ ID NO : 1, 3, 5 ou 7, de manière particulièrement préférée par rapport à la séquence polynucléotidique selon la séquence SEQ ID NO : 1.

11. Procédé selon l'une quelconque des revendications précédentes, **caractérisé en ce qu'**il s'agit, pour le gène *proP* affaibli, d'un polynucléotide qui présente une identité de séquence d'au moins 80%, de préférence d'au moins 90%, de manière particulièrement préférée d'au moins 95%, en particulier d'au moins 98, 99 ou 100% par rapport à la séquence polynucléotidique selon la séquence SEQ ID NO : 1 et qui présente en outre obligatoirement une ou plusieurs des mutations suivantes par rapport au polynucléotide selon la séquence SEQ ID NO : 1 :
a. remplacement du triplet codant pour la L-arginine en position 324 selon la séquence SEQ ID NO : 2 ou en une position comparable de la séquence d'acides aminés par un triplet qui code pour un acide aminé choisi dans le groupe L-leucine, L-isoleucine et L-valine, de préférence la L-leucine ;
b. remplacement du triplet codant pour la L-tyrosine en position 467 selon la séquence SEQ ID NO : 2 ou en une position comparable de la séquence d'acides aminés par un triplet qui code pour un acide aminé choisi dans le groupe L-lysine, L-arginine et L-histidine, de préférence la L-histidine ;
c. remplacement du triplet codant pour l'acide glutamique L en position 412 selon la séquence SEQ ID NO : 2 ou en une position comparable de la séquence d'acides aminés par un triplet qui code pour un codon stop ;
d. délétion de la nucléobase adénine en position 854 du gène *proP* selon la séquence SEQ ID NO : 1 ;
e. délétion d'une ou de plusieurs nucléobases des positions 1173 à 1223, de préférence délétion de l'ensemble des nucléobases des positions 1173 à 1223 du gène *proP* selon la séquence SEQ ID NO : 1 ;
f. insertion de la nucléobase cytosine en position 842 du gène *proP* selon la séquence SEQ ID NO : 1 ;
g. insertion d'une ou de plusieurs nucléobases en position 973, de préférence insertion de 19 nucléobases en position 973 du gène *proP* selon la séquence SEQ ID NO : 1 ;
h. insertion d'une ou de plusieurs nucléobases en position 183, de préférence insertion de 1359 nucléobases en position 183 du gène *proP* selon la séquence SEQ ID NO : 1.

12. Procédé selon l'une quelconque des revendications précédentes, **caractérisé en ce que** la fermentation a lieu dans un milieu qui contient une source inorganique de soufre.

13. Procédé selon l'une quelconque des revendications précédentes, **caractérisé en ce que** l'acide aminé L est enrichi dans le bouillon de fermentation obtenu et ensuite le cas échéant isolé, rassemblé et/ou purifié.

14. Procédé selon l'une quelconque des revendications précédentes, **caractérisé en ce qu'**on isole ou récupère l'acide aminé L ensemble avec les constituants du bouillon de fermentation et/ou de la biomasse.

15. Micro-organisme de la famille des Enterobacteriaceae, qui contient un gène *proP* affaibli, **caractérisé en ce qu'**il surproduit de la L-méthionine et la sécrète de préférence dans le milieu,
**caractérisé en ce qu'**il présente une ou plusieurs des caractéristiques choisies dans le groupe suivant :
a) polynucléotide surexprimé, qui code pour un ou plusieurs composants du système transporteur thiosulfate/sulfate CysPUWA (EC 3.6.3.25),
b) polynucléotide surexprimé, qui code pour une 3'-phosphoadénosine 5'-phosphosulfate réductase CysH (EC 1.8.4.8),
c) polynucléotide surexprimé, qui code pour un ou plusieurs composants de la sulfite réductase CysJI (EC 1.8.1.2),
d) polynucléotide surexprimé, qui code pour une cystéine synthase A CysK (EC 2.5.1.47),
e) polynucléotide surexprimé, qui code pour une cystéine synthase B CysM (EC 2.5.1.47),
f) polynucléotide surexprimé, qui code pour une sérine acétyltransférase CysE (EC 2.3.1.30),
g) polynucléotide surexprimé, qui code pour un ou plusieurs composants du système de clivage de la glycine GcvTHP-Lpd (EC 2.1.2.10, EC 1.4.4.2, EC 1.8.1.4),
h) polynucléotide surexprimé, qui code pour une lipoyl-synthase LipA (EC 2.8.1.8),
i) polynucléotide surexprimé, qui code pour une lipoyl-protéine ligase LipB (EC 2.3.1.181),
j) polynucléotide surexprimé, qui code pour une phosphoglycérate déshydrogénase SerA (EC 1.1.1.95),
k) polynucléotide surexprimé, qui code pour une 3-phosphosérine phosphatase SerB (EC 3.1.3.3),
l) polynucléotide surexprimé, qui code pour une 3-phosphosérine/phosphohydroxythréonine aminotransférase SerC (EC 2.6.1.52),
m) polynucléotide surexprimé, qui code pour une sérine hydroxyméthyltransférase GlyA (EC 2.1.2.1),
n) polynucléotide surexprimé, qui code pour une aspartokinase I et une homosérine déshydrogénase I ThrA (EC 2.7.2.4, EC 1.1.1.3),
o) activité enzymatique renforcée de l'aspartate kinase, en particulier un polynucléotide surexprimé qui code pour une aspartate kinase LysC (EC 2.7.2.4),
p) polynucléotide surexprimé, qui code pour une homosérine déshydrogénase Hom (EC 1.1.1.3),
q) polynucléotide surexprimé, qui code pour une homosérine O-acétyltransférase MetX (EC 2.3.1.31),
r) polynucléotide surexprimé, qui code pour une homosérine-O-succinyltransférase MetA (EC 2.3.1.46),
s) polynucléotide surexprimé, qui code pour une cystathionine gamma-synthase MetB (EC 2.5.1.48),
t) polynucléotide surexprimé, qui code pour une β-C-S-lyase AecD (EC 4.4.1.8, également appelée bêta-lyase),
u) polynucléotide surexprimé, qui code pour une cystathionine bêta-lyase MetC (EC 4.4.1.8),
v) polynucléotide surexprimé, qui code pour une homocystéine S-méthyltransférase MetE indépendante de B12 (EC 2.1.1.14),
w) polynucléotide surexprimé, qui code pour une homocystéine S-méthyltransférase MetH dépendante de B12 (EC 2.1.1.13),
x) polynucléotide surexprimé, qui code pour une méthylène-tétrahydrofolate réductase MetF (EC 1.5.1.20),
y) polynucléotide surexprimé, qui code pour un ou plusieurs composants de l'exportateur de la L-méthionine BrnFE de Corynebacterium glutamicum,
z) polynucléotide surexprimé, qui code pour un ou plusieurs des composants de l'exportateur de la valine YgaZH d'Escherichia coli (b2682, b2683),
aa) polynucléotide surexprimé qui code pour le transporteur putatif YjeH d'Escherichia coli (b4141),
bb) polynucléotide surexprimé, qui code pour un ou plusieurs composants de la pyridine nucléotide transhydrogénase PntAB (EC 1.6.1.2),
cc) polynucléotide surexprimé, qui code pour une O-succinylhomosérine sulfhydrylase MetZ (EC 2.5.1.48),
dd) polynucléotide surexprimé, qui code pour une phosphoénolpyruvate carboxylase Pyc (EC 4.1.1.31),
ee) polynucléotide surexprimé, qui code pour une thiosulfate-soufre transférase RDL2 (EC 2.8.1.1),
ff) polynucléotide surexprimé, qui code pour une thiosulfate-thiol soufre transférase (EC 2.8.1.3),
gg) polynucléotide surexprimé, qui code pour une thiosulfate-dithiol soufre transférase (EC 2.8.1.5),
hh) activité augmentée de l'homosérine O-succinyltransférase MetA (EC 2.3.1.46),
ii) activité augmentée de la sérine acétyltransférase CysE (EC 2.3.1.30),
jj) affaiblissement du gène metJ (b3938, ECK3930) codant pour le régulateur de transcription de la L-méthionine-biosynthèse (MetJ),
kk) affaiblissement du gène pgi (b4025, ECK4017) codant pour la glucose-6-phosphate-isomérase (Pgi, EC N° 5.3.1.9),
ll) affaiblissement du gène thrB (b0003, ECK0003) codant pour l'homosérine kinase (ThrB, EC 2.7.1.39),
mm) affaiblissement du gène metK (b2942, ECK2937) codant pour la S-adénosylméthionine synthase (MetK, EC N° 2.5.1.6),
nn) affaiblissement du gène dapA (b2478, ECK2474) codant pour la dihydrodipicolinate synthase (DapA, EC N° 4.2.1.52),
oo) affaiblissement du gène pck (b3403, ECK3390) codant pour la phosphoénolpyruvate carboxykinase (Pck, EC N° 4.1.1.49),
pp) affaiblissement du gène purU (b1232, ECK1227) codant pour la formyltétrahydrofolate hydrolase (PurU, EC N° 3.5.1.10),
qq) affaiblissement du gène pykA (b1854, ECK1855) codant pour la pyruvate kinase II (PykA, EC 2.7.1.40)
rr) affaiblissement du gène pykF (b1676, ECK1672) codant pour la pyruvate kinase I (PykF, EC 2.7.1.40),
ss) affaiblissement du gène metQ (b0197, ECK0197) codant pour une sous-unité du transporteur de la L-méthionine (MetQNI),
tt) affaiblissement du gène metI (b0198, ECK0198) codant pour une sous-unité du transporteur de la L-méthionine (MetQNI),
uu) affaiblissement du gène metN (b0199, ECK0199) codant pour une sous-unité du transporteur de la L-méthionine (MetQNI),
vv) affaiblissement du gène dcd (b2065, ECK2059) codant pour la désoxycytidine 5'-triphosphate désaminase (Dcd, EC N° 3.5.4.13),
ww) affaiblissement du gène yncA (b1448, ECK1442) codant pour la N-acyltransférase putative (YncA, Metabolic Explorer WO2010/020681),
xx) affaiblissement du gène rpoS codant pour le facteur sigma RpoS (b2741, ECK2736),
yy) affaiblissement de la protéine régulatrice MetJ,
zz) affaiblissement de la S-adénosylméthionine synthase MetK ;
"affaiblissement" signifiant que l'activité de la protéine ProP ou sa concentration correspondante a été abaissée jusqu'à 0 à 75% de l'activité de la protéine ou sa concentration dans le micro-organisme non recombinant pour la protéine correspondante et "surexpression", "renforcement" ou "activité augmentée" signifiant que l'activité de la protéine correspondante est augmentée d'un facteur d'au moins 2 par rapport à la souche de type sauvage.

16. Micro-organisme selon la revendication 15, **caractérisé en ce qu'**il enrichit la L-méthionine dans les cellules.

17. Micro-organisme selon la revendication 15, **caractérisé en ce qu'**il enrichit la L-méthionine dans le milieu.

18. Micro-organisme selon l'une quelconque des revendications 15 à 17, **caractérisé en ce que** le gène *proP* est désactivé.

19. Micro-organisme selon l'une quelconque des revendications 15 à 18, **caractérisé en ce qu'**il présente une tolérance augmentée à la méthionine par rapport au micro-organisme sans gène *proP* affaibli, une tolérance augmentée à la méthionine signifiant que les micro-organismes sont encore en mesure de croître à une concentration en L-méthionine de 50 grammes par litre.

20. Micro-organisme selon l'une quelconque des revendications 15 à 19, **caractérisé en ce qu'**il s'agit, pour le micro-organisme de la famille des Enterobacteriaceae, d'une bactérie des genres Escherichia, Erwinia, Providencia ou Serratia, de préférence du genre Escherichia, de manière particulièrement préférée d'Escherichia coli.

21. Micro-organisme selon l'une quelconque des revendications 15 à 20, **caractérisé en ce qu'**il s'agit, pour le gène *proP,* d'un gène présentant une identité de séquence d'au moins 80%, de préférence d'au moins 90%, en particulier d'au moins 95%, surtout d'au moins 98, 99 ou 100%, par rapport à la séquence polynucléotidique selon les séquences SEQ ID NO : 1, SEQ ID NO : 3, selon la séquence SEQ ID NO : 5 ou selon la séquence SEQ ID NO : 7, de manière particulièrement préférée selon la séquence SEQ ID NO : 1.

22. Micro-organisme selon l'une quelconque des revendications 15 à 21, **caractérisé en ce qu'**il s'agit, pour le gène *proP* affaibli, d'un polynucléotide qui présente une identité de séquence d'au moins 80%, de préférence d'au moins 90%, en particulier d'au moins 95%, surtout d'au moins 98, 99 ou 100% par rapport à la séquence totale du polynucléotide selon la séquence SEQ ID NO : 1 et qui présente en outre obligatoirement une ou plusieurs des mutations suivantes par rapport au polynucléotide selon la séquence SEQ ID NO : 1 :
a. remplacement du triplet codant pour la L-arginine en position 324 selon la séquence SEQ ID NO : 2 ou en une position comparable de la séquence d'acides aminés par un triplet qui code pour un acide aminé choisi dans le groupe L-leucine, L-isoleucine et L-valine, de préférence la L-leucine ;
b. remplacement du triplet codant pour la L-tyrosine en position 467 selon la séquence SEQ ID NO : 2 ou en une position comparable de la séquence d'acides aminés par un triplet qui code pour un acide aminé choisi dans le groupe L-lysine, L-arginine et L-histidine, de préférence la L-histidine ;
c. remplacement du triplet codant pour l'acide glutamique L en position 412 selon la séquence SEQ ID NO : 2 ou en une position comparable de la séquence d'acides aminés par un triplet qui code pour un codon stop ;
d. délétion de la nucléobase adénine en position 854 du gène *proP* selon la séquence SEQ ID NO : 1 ;
e. délétion d'une ou de plusieurs nucléobases des positions 1173 à 1223, de préférence délétion de l'ensemble des nucléobases des positions 1173 à 1223 du gène *proP* selon la séquence SEQ ID NO : 1 ;
f. insertion de la nucléobase cytosine en position 842 du gène *proP* selon la séquence SEQ ID NO : 1 ;
g. insertion d'une ou de plusieurs nucléobases en position 973, de préférence insertion de 19 nucléobases en position 973 du gène *proP* selon la séquence SEQ ID NO : 1 ;
h. insertion d'une ou de plusieurs nucléobases en position 183, de préférence insertion de 1359 nucléobases en position 183 du gène *proP* selon la séquence SEQ ID NO : 1.

23. Polynucléotide présentant une identité d'au moins 80%, de préférence d'au moins 90%, en particulier d'au moins 95%, surtout d'au moins 98, 99 ou 100%, par rapport à la séquence SEQ ID NO : 1, **caractérisé en ce que** le polynucléotide présente obligatoirement une ou plusieurs mutations par rapport au polynucléotide selon la séquence SEQ ID NO : 1, choisies parmi :
a. remplacement du triplet codant pour la L-arginine en position 324 selon la séquence SEQ ID NO : 2 ou en une position comparable de la séquence d'acides aminés par un triplet qui code pour un acide aminé choisi dans le groupe L-leucine, L-isoleucine et L-valine, de préférence la L-leucine ;
b. remplacement du triplet codant pour la L-tyrosine en position 467 selon la séquence SEQ ID NO : 2 ou en une position comparable de la séquence d'acides aminés par un triplet qui code pour un acide aminé choisi dans le groupe L-lysine, L-arginine et L-histidine, de préférence la L-histidine ;
c. remplacement du triplet codant pour l'acide glutamique L en position 412 selon la séquence SEQ ID NO : 2 ou en une position comparable de la séquence d'acides aminés par un triplet qui code pour un codon stop ;
d. délétion de la nucléobase adénine en position 854 du gène *proP* selon la séquence SEQ ID NO : 1 ;
e. délétion d'une ou de plusieurs des nucléobases des positions 1173 à 1223, de préférence délétion de l'ensemble des nucléobases des positions 1173 à 1223 du gène *proP* selon la séquence SEQ ID NO : 1 ;
f. insertion de la nucléobase cytosine en position 842 du gène *proP* selon la séquence SEQ ID NO : 1 ;
g. insertion d'une ou de plusieurs nucléobases en position 973, de préférence insertion de 19 nucléobases en position 973 du gène *proP* selon la séquence SEQ ID NO : 1 ;
h. insertion d'une ou de plusieurs nucléobases en position 183, de préférence insertion de 1359 nucléobases en position 183 du gène *proP* selon la séquence SEQ ID NO : 1.

24. Polynucléotide selon la revendication 23, **caractérisé en ce qu'**il s'hybride avec un ou plusieurs des polynucléotides choisi(s) parmi le groupe formé par un polynucléotide complémentaire à la séquence SEQ ID NO : 1, un polynucléotide complémentaire à la séquence SEQ ID NO : 3 et un polynucléotide complémentaire à la séquence SEQ ID NO : 5, de préférence un polynucléotide complémentaire à la séquence SEQ ID NO : 1.

25. Vecteur qui contient un polynucléotide selon la revendication 23 ou 24.

26. Polypeptide présentant une identité d'au moins 80%, de préférence d'au moins 90%, en particulier d'au moins 95%, surtout d'au moins 98, 99 ou 100%, par rapport à la séquence selon SEQ ID NO : 2, **caractérisé en ce que** le polypeptide présente obligatoirement une ou plusieurs mutations par rapport au polypeptide selon la séquence SEQ ID NO : 2, choisies parmi :
a. remplacement de la L-arginine en position 324 selon la séquence SEQ ID NO : 2 ou en une position comparable de la séquence d'acides aminés par un acide aminé choisi parmi le groupe L-leucine, L-isoleucine et L-valine, de préférence L-leucine ;
b. remplacement de la L-tyrosine en position 467 selon la séquence SEQ ID NO : 2 ou en une position comparable de la séquence d'acides aminés par un acide aminé choisi parmi le groupe L-lysine, L-arginine et L-histidine, de préférence L-histidine ;
c. délétion des 17 acides aminés des positions 392 à 408 selon la séquence SEQ ID NO : 2 ou d'une position comparable de la séquence d'acides aminés ;
d. délétion de jusqu'à 420 acides aminés de l'extrémité C par rapport à la séquence selon la séquence SEQ ID NO : 2, de préférence une délétion de 88, 163, 202, 212 ou 420 acides aminés de l'extrémité C de la séquence d'acides aminés selon la séquence SEQ ID NO : 2.

27. Micro-organisme recombinant qui contient un polynucléotide selon la revendication 23 ou 24 et/ou un vecteur selon la revendication 25 et/ou un polypeptide selon la revendication 26.


### SEQUENCE LISTING

<110> Evonik Industries AG
<120> Verfahren zur fermentativen Herstellung von organisch-chemischen Verbindungen unter Verwendung von verbesserten Stämmen der Familie Enterobacteriaceae
<130> 2011E00314
<160> 38
<170> PatentIn version 3.5
<210> 1
   <211> 1503
   <212> DNA
   <213> Escherichia coli
<220>
   <221> CDS
   <222> (1)..(1503)
   <223> Kodierregion proP
<400> 1
   atg ctg aaa agg aaa aaa gta aaa ccg att acc ctt cgt gat gtc acc        48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg        96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct       144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag       192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg       240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag       288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt       336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att       384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa       432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa       480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt        528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa        576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg        624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act        672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat        864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att        912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac        960
   Met Ile Gly Met Leu Phe Val Gln Pro Val Met Gly Leu Leu Ser Asp
   305                 310                 315                 320
   cgt ttt ggc cgt cgt ccg ttt gtg cta ctt ggt agt gtt gcc ctg ttt       1008
   Arg Phe Gly Arg Arg Pro Phe Val Leu Leu Gly Ser Val Ala Leu Phe
                   325                 330                 335
   gtg ttg gcg atc ccg gcg ttt att ctg att aac agt aac gtc atc ggc       1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt       1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc       1152
   Thr Gly Val Met Ala Ser Thr Leu Pro Ala Met Phe Pro Thr His Ile
       370                 375                 380
   cgt tac agc gcg ctg gcg gcg gca ttt aat att tcg gtg ctg gtt gcc       1200
   Arg Tyr Ser Ala Leu Ala Ala Ala Phe Asn Ile Ser Val Leu Val Ala
   385                 390                 395                 400
   ggt ctg acg cca acg ctg gcg gcc tgg ctg gtc gaa agc tcg cag aat       1248
   Gly Leu Thr Pro Thr Leu Ala Ala Trp Leu Val Glu Ser Ser Gln Asn
                   405                 410                 415
   ctg atg atg cct gcc tat tac ctg atg gta gtg gcg gtg gtt ggt tta       1296
   Leu Met Met Pro Ala Tyr Tyr Leu Met Val Val Ala Val Val Gly Leu
               420                 425                 430
   atc acc ggc gta acc atg aaa gag acg gca aat cgt ccg ttg aaa ggt       1344
   Ile Thr Gly Val Thr Met Lys Glu Thr Ala Asn Arg Pro Leu Lys Gly
           435                 440                 445
   gcg aca ccg gcg gcg tca gat ata cag gaa gcg aag gaa att ctc gtc       1392
   Ala Thr Pro Ala Ala Ser Asp Ile Gln Glu Ala Lys Glu Ile Leu Val
       450                 455                 460
   gag cat tac gat aat atc gag cag aaa atc gat gat att gac cac gag       1440
   Glu His Tyr Asp Asn Ile Glu Gln Lys Ile Asp Asp Ile Asp His Glu
   465                 470                 475                 480
   att gcc gat ttg cag gcg aaa cgt acc cgc ctg gtg cag caa cat ccg       1488
   Ile Ala Asp Leu Gln Ala Lys Arg Thr Arg Leu Val Gln Gln His Pro
                   485                 490                 495
   cga att gat gaa taa                                                   1503
   Arg Ile Asp Glu
               500
<210> 2
   <211> 500
   <212> PRT
   <213> Escherichia coli
<400> 2
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
   130 135 140
   Tyr Thr Gly Ala Ser Ile Phe Val Ala Glu Tyr Ser Pro Asp Arg Lys
   145 150 155 160
   Arg Gly Phe Met Gly Ser Trp Leu Asp Phe Gly Ser Ile Ala Gly Phe
   165 170 175
   Val Leu Gly Ala Gly Val Val Val Leu Ile Ser Thr Ile Val Gly Glu
   180 185 190
   Ala Asn Phe Leu Asp Trp Gly Trp Arg Ile Pro Phe Phe Ile Ala Leu
   195 200 205
   Pro Leu Gly Ile Ile Gly Leu Tyr Leu Arg His Ala Leu Glu Glu Thr
   210 215 220
   Pro Ala Phe Gln Gln His Val Asp Lys Leu Glu Gln Gly Asp Arg Glu
   225 230 235 240
   Gly Leu Gln Asp Gly Pro Lys Val Ser Phe Lys Glu Ile Ala Thr Lys
   245 250 255
   Tyr Trp Arg Ser Leu Leu Thr Cys Ile Gly Leu Val Ile Ala Thr Asn
   260 265 270
   Val Thr Tyr Tyr Met Leu Leu Thr Tyr Met Pro Ser Tyr Leu Ser His
   275 280 285
   Asn Leu His Tyr Ser Glu Asp His Gly Val Leu Ile Ile Ile Ala Ile
   290 295 300
   Met Ile Gly Met Leu Phe Val Gln Pro Val Met Gly Leu Leu Ser Asp
   305 310 315 320
   Arg Phe Gly Arg Arg Pro Phe Val Leu Leu Gly Ser Val Ala Leu Phe
   325 330 335
   Val Leu Ala Ile Pro Ala Phe Ile Leu Ile Asn Ser Asn Val Ile Gly
   340 345 350
   Leu Ile Phe Ala Gly Leu Leu Met Leu Ala Val Ile Leu Asn Cys Phe
   355 360 365
   Thr Gly Val Met Ala Ser Thr Leu Pro Ala Met Phe Pro Thr His Ile
   370 375 380
   Arg Tyr Ser Ala Leu Ala Ala Ala Phe Asn Ile Ser Val Leu Val Ala
   385 390 395 400
   Gly Leu Thr Pro Thr Leu Ala Ala Trp Leu Val Glu Ser Ser Gln Asn
                   405                 410                 415
   Leu Met Met Pro Ala Tyr Tyr Leu Met Val Val Ala Val Val Gly Leu
               420                 425                 430
   Ile Thr Gly Val Thr Met Lys Glu Thr Ala Asn Arg Pro Leu Lys Gly
           435                 440                 445
   Ala Thr Pro Ala Ala Ser Asp Ile Gln Glu Ala Lys Glu Ile Leu Val
       450                 455                 460
   Glu His Tyr Asp Asn Ile Glu Gln Lys Ile Asp Asp Ile Asp His Glu
   465                 470                 475                 480
   Ile Ala Asp Leu Gln Ala Lys Arg Thr Arg Leu Val Gln Gln His Pro
                   485                 490                 495
   Arg Ile Asp Glu
               500
<210> 3
   <211> 1503
   <212> DNA
   <213> Salmonella enterica
<220>
   <221> CDS
   <222> (1)..(1503)
   <223> Kodierregion proP
<400> 3
   atg ctg aaa agg aaa aaa ata aaa ccg att aca ctg ggc gat gtg acc       48
   Met Leu Lys Arg Lys Lys Ile Lys Pro Ile Thr Leu Gly Asp Val Thr
   1               5                   10                  15
   atc att gat gat ggt aaa ctt cgc aaa gcg att acc gcc gcc tcg ctg       96
   Ile Ile Asp Asp Gly Lys Leu Arg Lys Ala Ile Thr Ala Ala Ser Leu
               20                  25                  30
   ggc aac gcg atg gag tgg ttt gat ttt ggt gtt tat gga ttt gtt gcc      144
   Gly Asn Ala Met Glu Trp Phe Asp Phe Gly Val Tyr Gly Phe Val Ala
           35                  40                  45
   tac gcg ttg ggt aaa gtc ttt ttc ccc ggc gcc gat ccc agc gtc cag      192
   Tyr Ala Leu Gly Lys Val Phe Phe Pro Gly Ala Asp Pro Ser Val Gln
       50                  55                  60
   atg att gcc gcg ctg gcc acg ttt tcc gtt ccc ttc ctg att cgt ccg      240
   Met Ile Ala Ala Leu Ala Thr Phe Ser Val Pro Phe Leu Ile Arg Pro
   65                  70                  75                  80
   ctc ggc ggg tta ttc ttt ggt atg ctc ggc gat aaa tac ggg cgc cag      288
   Leu Gly Gly Leu Phe Phe Gly Met Leu Gly Asp Lys Tyr Gly Arg Gln
                   85                  90                  95
   aag atc ctg gcg atc acg att gtg att atg tcg atc agt acc ttc tgt         336
   Lys Ile Leu Ala Ile Thr Ile Val Ile Met Ser Ile Ser Thr Phe Cys
               100                 105                 110
   atc ggg tta atc ccc tct tac gcg acg atc ggt atc tgg gcg cca ata         384
   Ile Gly Leu Ile Pro Ser Tyr Ala Thr Ile Gly Ile Trp Ala Pro Ile
           115                 120                 125
   ctg ttg ttg ctg tgt aaa atg gcg cag ggc ttc tcg gtt ggc ggg gaa         432
   Leu Leu Leu Leu Cys Lys Met Ala Gln Gly Phe Ser Val Gly Gly Glu
       130                 135                 140
   tat acc ggc gcg tcg atc ttt gtc gcg gaa tat tcg ccg gat cgt aaa         480
   Tyr Thr Gly Ala Ser Ile Phe Val Ala Glu Tyr Ser Pro Asp Arg Lys
   145                 150                 155                 160
   cgc gga ttt atg gga agc tgg ctg gat ttt ggt tct atc gcc gga ttc         528
   Arg Gly Phe Met Gly Ser Trp Leu Asp Phe Gly Ser Ile Ala Gly Phe
                   165                 170                 175
   gtg ctg ggc gcg ggc gtg gtg gtc ttg atc tcg acg att gtc ggc gag         576
   Val Leu Gly Ala Gly Val Val Val Leu Ile Ser Thr Ile Val Gly Glu
               180                 185                 190
   gag aat ttc ctt gag tgg ggc tgg cgt att ccg ttc ttt atc gcc ctg         624
   Glu Asn Phe Leu Glu Trp Gly Trp Arg Ile Pro Phe Phe Ile Ala Leu
           195                 200                 205
   cca ttg ggg att att ggt ctc tac tta cgc cat gcg ctg gag gag acg         672
   Pro Leu Gly Ile Ile Gly Leu Tyr Leu Arg His Ala Leu Glu Glu Thr
       210                 215                 220
   cca gcg ttt cag cag cac gtg gat aaa ctg gag cag ggc gac cgc gaa         720
   Pro Ala Phe Gln Gln His Val Asp Lys Leu Glu Gln Gly Asp Arg Glu
   225                 230                 235                 240
   ggg ttg cag gat ggg ccg aaa gtc tcc ttt aaa gag att gcc acc aaa         768
   Gly Leu Gln Asp Gly Pro Lys Val Ser Phe Lys Glu Ile Ala Thr Lys
                   245                 250                 255
   cac tgg cgt agc ctg ttg tca tgt atc ggt ctg gtg att gcc acc aac         816
   His Trp Arg Ser Leu Leu Ser Cys Ile Gly Leu Val Ile Ala Thr Asn
               260                 265                 270
   gtg acc tac tac atg ctg ctc acc tac atg ccg agc tac ctg tcg cat         864
   Val Thr Tyr Tyr Met Leu Leu Thr Tyr Met Pro Ser Tyr Leu Ser His
           275                 280                 285
   aac ctg cac tat tct gaa gat cac ggc gtg ttg att atc atc gcc att         912
   Asn Leu His Tyr Ser Glu Asp His Gly Val Leu Ile Ile Ile Ala Ile
       290                 295                 300
   atg atc ggg atg ctg ttt gtg cag ccg gtg atg ggg ctg ctg agc gac         960
   Met Ile Gly Met Leu Phe Val Gln Pro Val Met Gly Leu Leu Ser Asp
   305                 310                 315                 320
   cgt ttc ggt cga cgt cca ttt gtg att atg ggc agc att gcg ctg ttc        1008
   Arg Phe Gly Arg Arg Pro Phe Val Ile Met Gly Ser Ile Ala Leu Phe
                   325                 330                 335
   gcg ctg gcg atc ccg gcc ttc atc ctg att aac agt aac gtt att ggc        1056
   Ala Leu Ala Ile Pro Ala Phe Ile Leu Ile Asn Ser Asn Val Ile Gly
               340                 345                 350
   ctg att ttt gca ggt ttg ttg atg ctg gcg gtg att ctg aac tgc ttt      1104
   Leu Ile Phe Ala Gly Leu Leu Met Leu Ala Val Ile Leu Asn Cys Phe
           355                 360                 365
   acc ggg gtg atg gcc tcg aca ttg ccg gcg atg ttt ccg acg cat att      1152
   Thr Gly Val Met Ala Ser Thr Leu Pro Ala Met Phe Pro Thr His Ile
       370                 375                 380
   cgt tat agc gcg ctg gcg gcg gct ttt aat atc tct gta ttg att gcc      1200
   Arg Tyr Ser Ala Leu Ala Ala Ala Phe Asn Ile Ser Val Leu Ile Ala
   385                 390                 395                 400
   ggt ctg acg cca acg ctg gcg gcc tgg ctg gtg gaa agc tcg cag gat      1248
   Gly Leu Thr Pro Thr Leu Ala Ala Trp Leu Val Glu Ser Ser Gln Asp
                   405                 410                 415
   ctg atg atg ccg gcg tat tat ttg atg gtc atc gcg gtg ata ggc ttg      1296
   Leu Met Met Pro Ala Tyr Tyr Leu Met Val Ile Ala Val Ile Gly Leu
               420                 425                 430
   att acc ggt att tcc atg aaa gag acg gcc aat cgt ccg tta aaa ggc      1344
   Ile Thr Gly Ile Ser Met Lys Glu Thr Ala Asn Arg Pro Leu Lys Gly
           435                 440                 445
   gca acg cca gcg gcg tcg gac atc cag gaa gcg aag gaa att ctg ggc      1392
   Ala Thr Pro Ala Ala Ser Asp Ile Gln Glu Ala Lys Glu Ile Leu Gly
       450                 455                 460
   gag cat tac gat aat att gag cag aaa atc gac gac atc gat cag gaa      1440
   Glu His Tyr Asp Asn Ile Glu Gln Lys Ile Asp Asp Ile Asp Gln Glu
   465                 470                 475                 480
   att gcg gag ctg cag gtc aaa cgt tcg cgt ctg gta cag caa cat ccg      1488
   Ile Ala Glu Leu Gln Val Lys Arg Ser Arg Leu Val Gln Gln His Pro
                   485                 490                 495
   cgt atc gat gaa taa                                                  1503
   Arg Ile Asp Glu
               500
<210> 4
   <211> 500
   <212> PRT
   <213> Salmonella enterica
<400> 4
   Met Leu Lys Arg Lys Lys Ile Lys Pro Ile Thr Leu Gly Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Ile Ala Ala Leu Ala Thr Phe Ser Val Pro Phe Leu Ile Arg Pro
   65 70 75 80
   Leu Gly Gly Leu Phe Phe Gly Met Leu Gly Asp Lys Tyr Gly Arg Gln
   85 90 95
   Lys Ile Leu Ala Ile Thr Ile Val Ile Met Ser Ile Ser Thr Phe Cys
   100 105 110
   Ile Gly Leu Ile Pro Ser Tyr Ala Thr Ile Gly Ile Trp Ala Pro Ile
   115 120 125
   Leu Leu Leu Leu Cys Lys Met Ala Gln Gly Phe Ser Val Gly Gly Glu
   130 135 140
   Tyr Thr Gly Ala Ser Ile Phe Val Ala Glu Tyr Ser Pro Asp Arg Lys
   145 150 155 160
   Arg Gly Phe Met Gly Ser Trp Leu Asp Phe Gly Ser Ile Ala Gly Phe
   165 170 175
   Val Leu Gly Ala Gly Val Val Val Leu Ile Ser Thr Ile Val Gly Glu
   180 185 190
   Glu Asn Phe Leu Glu Trp Gly Trp Arg Ile Pro Phe Phe Ile Ala Leu
   195 200 205
   Pro Leu Gly Ile Ile Gly Leu Tyr Leu Arg His Ala Leu Glu Glu Thr
   210 215 220
   Pro Ala Phe Gln Gln His Val Asp Lys Leu Glu Gln Gly Asp Arg Glu
   225 230 235 240
   Gly Leu Gln Asp Gly Pro Lys Val Ser Phe Lys Glu Ile Ala Thr Lys
   245 250 255
   His Trp Arg Ser Leu Leu Ser Cys Ile Gly Leu Val Ile Ala Thr Asn
   260 265 270
   Val Thr Tyr Tyr Met Leu Leu Thr Tyr Met Pro Ser Tyr Leu Ser His
   275 280 285
   Asn Leu His Tyr Ser Glu Asp His Gly Val Leu Ile Ile Ile Ala Ile
   290 295 300
   Met Ile Gly Met Leu Phe Val Gln Pro Val Met Gly Leu Leu Ser Asp
   305 310 315 320
   Arg Phe Gly Arg Arg Pro Phe Val Ile Met Gly Ser Ile Ala Leu Phe
   325 330 335
   Ala Leu Ala Ile Pro Ala Phe Ile Leu Ile Asn Ser Asn Val Ile Gly
               340                 345                 350
   Leu Ile Phe Ala Gly Leu Leu Met Leu Ala Val Ile Leu Asn Cys Phe
           355                 360                 365
   Thr Gly Val Met Ala Ser Thr Leu Pro Ala Met Phe Pro Thr His Ile
       370                 375                 380
   Arg Tyr Ser Ala Leu Ala Ala Ala Phe Asn Ile Ser Val Leu Ile Ala
   385                 390                 395                 400
   Gly Leu Thr Pro Thr Leu Ala Ala Trp Leu Val Glu Ser Ser Gln Asp
                   405                 410                 415
   Leu Met Met Pro Ala Tyr Tyr Leu Met Val Ile Ala Val Ile Gly Leu
               420                 425                 430
   Ile Thr Gly Ile Ser Met Lys Glu Thr Ala Asn Arg Pro Leu Lys Gly
           435                 440                 445
   Ala Thr Pro Ala Ala Ser Asp Ile Gln Glu Ala Lys Glu Ile Leu Gly
       450                 455                 460
   Glu His Tyr Asp Asn Ile Glu Gln Lys Ile Asp Asp Ile Asp Gln Glu
   465                 470                 475                 480
   Ile Ala Glu Leu Gln Val Lys Arg Ser Arg Leu Val Gln Gln His Pro
                   485                 490                 495
   Arg Ile Asp Glu
               500
<210> 5
   <211> 1503
   <212> DNA
   <213> Shigella sonnei
<220>
   <221> CDS
   <222> (1)..(1503)
   <223> Kodierregion proP
<400> 5
   atg ctg aaa agg aaa aaa gta aaa ccg att acc ctt cgt gat gtc acc          48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg          96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct       144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag       192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg       240
   Met Val Ala Ala Leu Ala Thr Phe Ser Val Pro Phe Leu Ile Arg Pro
   65                  70                  75                  80
   ctt ggc ggg ctc ttc ttt ggt atg ttg ggc gat aaa tat ggt cgc cag       288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt       336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att       384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa       432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa       480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt       528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa       576
   Val Leu Gly Ala Gly Val Val Val Leu Ile Ser Thr Ile Val Gly Glu
               180                 185                 190
   gcg aac ttc ctc gac tgg ggc tgg cgt att ccg ttc ttt att gct ctg       624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ttg gaa gaa act       672
   Pro Leu Gly Ile Ile Gly Leu Tyr Leu Arg His Ala Leu Glu Glu Thr
       210                 215                 220
   ccg gcg ttc cag cag cat gtt gat aaa ctg gaa cag ggc gac cgc gaa       720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa       768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac       816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat       864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att      912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac      960
   Met Ile Gly Met Leu Phe Val Gln Pro Val Met Gly Leu Leu Ser Asp
   305                 310                 315                 320
   cgt ttt ggc cgt cgt ccg ttt gtg cta ctt ggt agt gtt gcc ctg ttt     1008
   Arg Phe Gly Arg Arg Pro Phe Val Leu Leu Gly Ser Val Ala Leu Phe
                   325                 330                 335
   gtg ttg gcg atc ccg gcg ttt att ctg att aac agt aac gtc atc ggc     1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt     1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc     1152
   Thr Gly Val Met Ala Ser Thr Leu Pro Ala Met Phe Pro Thr His Ile
       370                 375                 380
   cgt tac agc gcg ctg gcg gcg gca ttt aat att tcg gtg ctg gtt gcc     1200
   Arg Tyr Ser Ala Leu Ala Ala Ala Phe Asn Ile Ser Val Leu Val Ala
   385                 390                 395                 400
   ggt ctg acg cca aca ctg gcg gcc tgg ctg gtc gaa agc tcg cag aat     1248
   Gly Leu Thr Pro Thr Leu Ala Ala Trp Leu Val Glu Ser Ser Gln Asn
                   405                 410                 415
   ctg atg atg cct gcc tat tac ctg atg gta gtg gcg gtg att ggt tta     1296
   Leu Met Met Pro Ala Tyr Tyr Leu Met Val Val Ala Val Ile Gly Leu
               420                 425                 430
   atc acc ggc gta acc atg aaa gag acg gca aat cgt ccg ttg aaa ggt     1344
   Ile Thr Gly Val Thr Met Lys Glu Thr Ala Asn Arg Pro Leu Lys Gly
           435                 440                 445
   gcg aca ccg gcg gcg tca gat ata cag gaa gcg aag gaa att ctc gtc     1392
   Ala Thr Pro Ala Ala Ser Asp Ile Gln Glu Ala Lys Glu Ile Leu Val
       450                 455                 460
   gag cat tac gat aat atc gag cag aaa atc gat gat att gac cac gag     1440
   Glu His Tyr Asp Asn Ile Glu Gln Lys Ile Asp Asp Ile Asp His Glu
   465                 470                 475                 480
   att gcc gat ttg cag gcg aaa cgt acc cgc ctg gtg cag caa cat ccg     1488
   Ile Ala Asp Leu Gln Ala Lys Arg Thr Arg Leu Val Gln Gln His Pro
                   485                 490                 495
   cga att gat gaa taa                                                 1503
   Arg Ile Asp Glu
               500
<210> 6
   <211> 500
   <212> PRT
   <213> Shigella sonnei
<400> 6
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Tyr Leu Ser His
           275                 280                 285
   Asn Leu His Tyr Ser Glu Asp His Gly Val Leu Ile Ile Ile Ala Ile
       290                 295                 300
   Met Ile Gly Met Leu Phe Val Gln Pro Val Met Gly Leu Leu Ser Asp
   305                 310                 315                 320
   Arg Phe Gly Arg Arg Pro Phe Val Leu Leu Gly Ser Val Ala Leu Phe
                   325                 330                 335
   Val Leu Ala Ile Pro Ala Phe Ile Leu Ile Asn Ser Asn Val Ile Gly
               340                 345                 350
   Leu Ile Phe Ala Gly Leu Leu Met Leu Ala Val Ile Leu Asn Cys Phe
           355                 360                 365
   Thr Gly Val Met Ala Ser Thr Leu Pro Ala Met Phe Pro Thr His Ile
       370                 375                 380
   Arg Tyr Ser Ala Leu Ala Ala Ala Phe Asn Ile Ser Val Leu Val Ala
   385                 390                 395                 400
   Gly Leu Thr Pro Thr Leu Ala Ala Trp Leu Val Glu Ser Ser Gln Asn
                   405                 410                 415
   Leu Met Met Pro Ala Tyr Tyr Leu Met Val Val Ala Val Ile Gly Leu
               420                 425                 430
   Ile Thr Gly Val Thr Met Lys Glu Thr Ala Asn Arg Pro Leu Lys Gly
           435                 440                 445
   Ala Thr Pro Ala Ala Ser Asp Ile Gln Glu Ala Lys Glu Ile Leu Val
       450                 455                 460
   Glu His Tyr Asp Asn Ile Glu Gln Lys Ile Asp Asp Ile Asp His Glu
   465                 470                 475                 480
   Ile Ala Asp Leu Gln Ala Lys Arg Thr Arg Leu Val Gln Gln His Pro
                   485                 490                 495
   Arg Ile Asp Glu
               500
<210> 7
   <211> 1506
   <212> DNA
   <213> Erwinia pyrifoliae
<220>
   <221> CDS
   <222> (1)..(1506)
   <223> Kodierregion proP
<400> 7
   atg aaa tta cgt agg aag cgt gtt aag cct atc gga tta aag gac gtc      48
   Met Lys Leu Arg Arg Lys Arg Val Lys Pro Ile Gly Leu Lys Asp Val
   1               5                   10                  15
   acc att att gac gat gcc aga tta cgt aag gcg att aca gct gcc tca      96
   Thr Ile Ile Asp Asp Ala Arg Leu Arg Lys Ala Ile Thr Ala Ala Ser
               20                  25                  30
   ttg ggc aat gcc atg gag tgg ttc gac ttt ggc gtt tat ggt ttt gtt     144
   Leu Gly Asn Ala Met Glu Trp Phe Asp Phe Gly Val Tyr Gly Phe Val
           35                  40                  45
   gcc tac gca ctg ggg caa gtt ttc ttc ccc ggc gcc gat cca ggg acg     192
   Ala Tyr Ala Leu Gly Gln Val Phe Phe Pro Gly Ala Asp Pro Gly Thr
       50                  55                  60
   cag atg att gcc gcc ctg gca acc ttc tcc gtg ccc ttc ctg atc cgc     240
   Gln Met Ile Ala Ala Leu Ala Thr Phe Ser Val Pro Phe Leu Ile Arg
   65                  70                  75                  80
   ccg tta ggc ggc ctg ttc ttt ggg tcg ctg ggg gat aaa tac ggc cgc     288
   Pro Leu Gly Gly Leu Phe Phe Gly Ser Leu Gly Asp Lys Tyr Gly Arg
                   85                  90                  95
   cag aaa ata ctg tcg ata acc att att atc atg tcg gtc agt acg ttc     336
   Gln Lys Ile Leu Ser Ile Thr Ile Ile Ile Met Ser Val Ser Thr Phe
               100                 105                 110
   tgt att ggt tta atc ccg tct tat gcc tcg att ggt atc tgg gca ccg     384
   Cys Ile Gly Leu Ile Pro Ser Tyr Ala Ser Ile Gly Ile Trp Ala Pro
           115                 120                 125
   atc ctg cta ttg ctg tgt aaa atg gcg cag ggc ttc tcg gtg ggt ggt     432
   Ile Leu Leu Leu Leu Cys Lys Met Ala Gln Gly Phe Ser Val Gly Gly
       130                 135                 140
   gaa tat acc ggg gct tcc atc ttc gtt gct gaa tac tca ccg gat cgc     480
   Glu Tyr Thr Gly Ala Ser Ile Phe Val Ala Glu Tyr Ser Pro Asp Arg
   145                 150                 155                 160
   aaa cgt ggc ttt atg ggc agc tgg ctg gac ttc ggt tcc atc gcc gga     528
   Lys Arg Gly Phe Met Gly Ser Trp Leu Asp Phe Gly Ser Ile Ala Gly
                   165                 170                 175
   ttt gtg ctg ggt gcc gga ctg gtg gtg ctg att tca gcg gtt atc ggt     576
   Phe Val Leu Gly Ala Gly Leu Val Val Leu Ile Ser Ala Val Ile Gly
               180                 185                 190
   gaa gcg agt ttc ctt gaa tgg ggc tgg cgt att ccg ttc ttc gtg gcg     624
   Glu Ala Ser Phe Leu Glu Trp Gly Trp Arg Ile Pro Phe Phe Val Ala
           195                 200                 205
   cta ccg ctg ggt atc atc ggg ctt tat ctg cgc cac gcg ctt gaa gag     672
   Leu Pro Leu Gly Ile Ile Gly Leu Tyr Leu Arg His Ala Leu Glu Glu
       210                 215                 220
   act ccg gcg ttc cag cag cat gtc gac aag ctg gaa aag ggc gat cgg        720
   Thr Pro Ala Phe Gln Gln His Val Asp Lys Leu Glu Lys Gly Asp Arg
   225                 230                 235                 240
   gaa gga ttg gcc gat ggg ccg caa gtc tca ttt aaa gag att gcc act        768
   Glu Gly Leu Ala Asp Gly Pro Gln Val Ser Phe Lys Glu Ile Ala Thr
                   245                 250                 255
   aag cac tgg aaa agc ctg ctg gcc tgc atc ggt ctg gtg att gcc acc        816
   Lys His Trp Lys Ser Leu Leu Ala Cys Ile Gly Leu Val Ile Ala Thr
               260                 265                 270
   aac gtg acc tat tac atg ctg ctg acc tac atg ccg agc tac ctg tcg        864
   Asn Val Thr Tyr Tyr Met Leu Leu Thr Tyr Met Pro Ser Tyr Leu Ser
           275                 280                 285
   cat aac ctc cat tat tcg gaa gat cat ggc gtg atg atc att atc gcc        912
   His Asn Leu His Tyr Ser Glu Asp His Gly Val Met Ile Ile Ile Ala
       290                 295                 300
   att atg ttg ggg atg ctg ttt gtg cag ccg gtg atg ggc ctg atg agc        960
   Ile Met Leu Gly Met Leu Phe Val Gln Pro Val Met Gly Leu Met Ser
   305                 310                 315                 320
   gac aaa ttc ggt cgt cgc ccg ttt gtt att atc ggc agt atc gcg ctg       1008
   Asp Lys Phe Gly Arg Arg Pro Phe Val Ile Ile Gly Ser Ile Ala Leu
                   325                 330                 335
   ctg acg ctg tca gta ccg tgc ttt atg ctg atc aac agc ggc gtg atg       1056
   Leu Thr Leu Ser Val Pro Cys Phe Met Leu Ile Asn Ser Gly Val Met
               340                 345                 350
   ggt ctg att ttt gcc ggg ctg ctg acg ctg gcg gtg atc ctt aac agc       1104
   Gly Leu Ile Phe Ala Gly Leu Leu Thr Leu Ala Val Ile Leu Asn Ser
           355                 360                 365
   ttc acc ggg gtc atg gca tca agc ctg ccg gcg atg ttc ccc act cat       1152
   Phe Thr Gly Val Met Ala Ser Ser Leu Pro Ala Met Phe Pro Thr His
       370                 375                 380
   atc cgc tac agt gcg ctg gcc agt gcc ttt aac atc tcg gtg ctg gtt       1200
   Ile Arg Tyr Ser Ala Leu Ala Ser Ala Phe Asn Ile Ser Val Leu Val
   385                 390                 395                 400
   gcc ggc ctg acg ccg acc gct gcc gcc tgg ctg gta gaa acg acc agc       1248
   Ala Gly Leu Thr Pro Thr Ala Ala Ala Trp Leu Val Glu Thr Thr Ser
                   405                 410                 415
   aat ttg tat atg cca gct tat tat ctg atg gtc gtc gcg gtg att ggt       1296
   Asn Leu Tyr Met Pro Ala Tyr Tyr Leu Met Val Val Ala Val Ile Gly
               420                 425                 430
   ctg gta acc ggg att atg atg aag gaa acg gcc aat ctg ccg ctg cgt       1344
   Leu Val Thr Gly Ile Met Met Lys Glu Thr Ala Asn Leu Pro Leu Arg
           435                 440                 445
   ggc gct gcg cct gcg gct tcg gat atg gct gaa gcg aaa gag atc ctg       1392
   Gly Ala Ala Pro Ala Ala Ser Asp Met Ala Glu Ala Lys Glu Ile Leu
       450                 455                 460
   cag gaa cat cac gat aat atc gaa cat aag atc gca gat att aac gag       1440
   Gln Glu His His Asp Asn Ile Glu His Lys Ile Ala Asp Ile Asn Glu
   465                 470                 475                 480
   cag atc gct gag ctt gaa gca aaa cgc tcg cat ctt att tat caa cat       1488
   Gln Ile Ala Glu Leu Glu Ala Lys Arg Ser His Leu Ile Tyr Gln His
                   485                 490                 495
   ccg cgt atc aac gag taa                                         1506
   Pro Arg Ile Asn Glu
               500
<210> 8
   <211> 501
   <212> PRT
   <213> Erwinia pyrifoliae
<400> 8
   Met Lys Leu Arg Arg Lys Arg Val Lys Pro Ile Gly Leu Lys Asp Val
   1               5                   10                  15
   Thr Ile Ile Asp Asp Ala Arg Leu Arg Lys Ala Ile Thr Ala Ala Ser
               20                  25                  30
   Leu Gly Asn Ala Met Glu Trp Phe Asp Phe Gly Val Tyr Gly Phe Val
           35                  40                  45
   Ala Tyr Ala Leu Gly Gln Val Phe Phe Pro Gly Ala Asp Pro Gly Thr
       50                  55                  60
   Gln Met Ile Ala Ala Leu Ala Thr Phe Ser Val Pro Phe Leu Ile Arg
   65                  70                  75                  80
   Pro Leu Gly Gly Leu Phe Phe Gly Ser Leu Gly Asp Lys Tyr Gly Arg
                   85                  90                  95
   Gln Lys Ile Leu Ser Ile Thr Ile Ile Ile Met Ser Val Ser Thr Phe
               100                 105                 110
   Cys Ile Gly Leu Ile Pro Ser Tyr Ala Ser Ile Gly Ile Trp Ala Pro
           115                 120                 125
   Ile Leu Leu Leu Leu Cys Lys Met Ala Gln Gly Phe Ser Val Gly Gly
       130                 135                 140
   Glu Tyr Thr Gly Ala Ser Ile Phe Val Ala Glu Tyr Ser Pro Asp Arg
   145                 150                 155                 160
   Lys Arg Gly Phe Met Gly Ser Trp Leu Asp Phe Gly Ser Ile Ala Gly
                   165                 170                 175
   Phe Val Leu Gly Ala Gly Leu Val Val Leu Ile Ser Ala Val Ile Gly
               180                 185                 190
   Glu Ala Ser Phe Leu Glu Trp Gly Trp Arg Ile Pro Phe Phe Val Ala
           195                 200                 205
   Leu Pro Leu Gly Ile Ile Gly Leu Tyr Leu Arg His Ala Leu Glu Glu
       210                 215                 220
   Thr Pro Ala Phe Gln Gln His Val Asp Lys Leu Glu Lys Gly Asp Arg
   225                 230                 235                 240
   Glu Gly Leu Ala Asp Gly Pro Gln Val Ser Phe Lys Glu Ile Ala Thr
                   245                 250                 255
   Lys His Trp Lys Ser Leu Leu Ala Cys Ile Gly Leu Val Ile Ala Thr
               260                 265                 270
   Asn Val Thr Tyr Tyr Met Leu Leu Thr Tyr Met Pro Ser Tyr Leu Ser
           275                 280                 285
   His Asn Leu His Tyr Ser Glu Asp His Gly Val Met Ile Ile Ile Ala
       290                 295                 300
   Ile Met Leu Gly Met Leu Phe Val Gln Pro Val Met Gly Leu Met Ser
   305                 310                 315                 320
   Asp Lys Phe Gly Arg Arg Pro Phe Val Ile Ile Gly Ser Ile Ala Leu
                   325                 330                 335
   Leu Thr Leu Ser Val Pro Cys Phe Met Leu Ile Asn Ser Gly Val Met
               340                 345                 350
   Gly Leu Ile Phe Ala Gly Leu Leu Thr Leu Ala Val Ile Leu Asn Ser
           355                 360                 365
   Phe Thr Gly Val Met Ala Ser Ser Leu Pro Ala Met Phe Pro Thr His
       370                 375                 380
   Ile Arg Tyr Ser Ala Leu Ala Ser Ala Phe Asn Ile Ser Val Leu Val
   385                 390                 395                 400
   Ala Gly Leu Thr Pro Thr Ala Ala Ala Trp Leu Val Glu Thr Thr Ser
                   405                 410                 415
   Asn Leu Tyr Met Pro Ala Tyr Tyr Leu Met Val Val Ala Val Ile Gly
               420                 425                 430
   Leu Val Thr Gly Ile Met Met Lys Glu Thr Ala Asn Leu Pro Leu Arg
           435                 440                 445
   Gly Ala Ala Pro Ala Ala Ser Asp Met Ala Glu Ala Lys Glu Ile Leu
       450                 455                 460
   Gln Glu His His Asp Asn Ile Glu His Lys Ile Ala Asp Ile Asn Glu
   465                 470                 475                 480
   Gln Ile Ala Glu Leu Glu Ala Lys Arg Ser His Leu Ile Tyr Gln His
                   485                 490                 495
   Pro Arg Ile Asn Glu
               500
<210> 9
   <211> 1503
   <212> DNA
   <213> Escherichia coli
<220>
   <221> CDS
   <222> (1)..(1236)
   <223> Kodierregion proP M8-Allel (Austausch g zu t an Position 1234 bedingt
   AA-Austausch E412*)
<400> 9
   atg ctg aaa agg aaa aaa gta aaa ccg att acc ctt cgt gat gtc acc        48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg        96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct       144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag       192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg       240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag       288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt       336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att       384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa       432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa       480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt       528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa       576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg       624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act       672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa       720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa       768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac       816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat       864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att       912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac       960
   Met Ile Gly Met Leu Phe Val Gln Pro Val Met Gly Leu Leu Ser Asp
   305                 310                 315                 320
   cgt ttt ggc cgt cgt ccg ttt gtg cta ctt ggt agt gtt gcc ctg ttt      1008
   Arg Phe Gly Arg Arg Pro Phe Val Leu Leu Gly Ser Val Ala Leu Phe
                   325                 330                 335
   gtg ttg gcg atc ccg gcg ttt att ctg att aac agt aac gtc atc ggc      1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt      1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc      1152
   Thr Gly Val Met Ala Ser Thr Leu Pro Ala Met Phe Pro Thr His Ile
       370                 375                 380
   cgt tac agc gcg ctg gcg gcg gca ttt aat att tcg gtg ctg gtt gcc      1200
   Arg Tyr Ser Ala Leu Ala Ala Ala Phe Asn Ile Ser Val Leu Val Ala
   385                 390                 395                 400
   ggt ctg acg cca acg ctg gcg gcc tgg ctg gtc taa agctcgcaga           1246
   Gly Leu Thr Pro Thr Leu Ala Ala Trp Leu Val
                   405                 410
   atctgatgat gcctgcctat tacctgatgg tagtggcggt ggttggttta atcaccggcg 1306
   taaccatgaa agagacggca aatcgtccgt tgaaaggtgc gacaccggcg gcgtcagata 1366
   tacaggaagc gaaggaaatt ctcgtcgagc attacgataa tatcgagcag aaaatcgatg 1426
   atattgacca cgagattgcc gatttgcagg cgaaacgtac ccgcctggtg cagcaacatc 1486
   cgcgaattga tgaataa 1503
<210> 10
   <211> 411
   <212> PRT
   <213> Escherichia coli
<400> 10
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Tyr Leu Ser His
           275                 280                 285
   Asn Leu His Tyr Ser Glu Asp His Gly Val Leu Ile Ile Ile Ala Ile
       290                 295                 300
   Met Ile Gly Met Leu Phe Val Gln Pro Val Met Gly Leu Leu Ser Asp
   305                 310                 315                 320
   Arg Phe Gly Arg Arg Pro Phe Val Leu Leu Gly Ser Val Ala Leu Phe
                   325                 330                 335
   Val Leu Ala Ile Pro Ala Phe Ile Leu Ile Asn Ser Asn Val Ile Gly
               340                 345                 350
   Leu Ile Phe Ala Gly Leu Leu Met Leu Ala Val Ile Leu Asn Cys Phe
           355                 360                 365
   Thr Gly Val Met Ala Ser Thr Leu Pro Ala Met Phe Pro Thr His Ile
       370                 375                 380
   Arg Tyr Ser Ala Leu Ala Ala Ala Phe Asn Ile Ser Val Leu Val Ala
   385                 390                 395                 400
   Gly Leu Thr Pro Thr Leu Ala Ala Trp Leu Val
                   405                 410
<210> 11
   <211> 1504
   <212> DNA
   <213> Escherichia coli
<220>
   <221> CDS
   <222> (1)..(867)
   <223> Kodierregion proP M3-Allel (Insertion von C nach Nukleotidposition 842)
<400> 11
   atg ctg aaa agg aaa aaa gta aaa ccg att acc ctt cgt gat gtc acc        48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg        96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct       144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag       192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg       240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag       288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt       336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att       384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa       432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa       480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt       528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa       576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg       624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act       672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa       720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tac tat gcc gag tta ttt gtc gca        864
   Val Thr Tyr Tyr Met Leu Leu Thr Tyr Tyr Ala Glu Leu Phe Val Ala
           275                 280                 285
   taa cctgcattac tccgaagacc acggggtgct gattattatc gccattatga             917
   tcggtatgct gtttgtccag ccggtgatgg gcttgctgag tgaccgtttt ggccgtcgtc      977
   cgtttgtgct acttggtagt gttgccctgt ttgtgttggc gatcccggcg tttattctga     1037
   ttaacagtaa cgtcatcggc ctgatttttg ccgggttact gatgctggcg gtgatcctta     1097
   actgctttac gggcgttatg gcttctacct tgccagcgat gttcccgacg catatccgtt     1157
   acagcgcgct ggcggcggca tttaatattt cggtgctggt tgccggtctg acgccaacgc     1217
   tggcggcctg gctggtcgaa agctcgcaga atctgatgat gcctgcctat tacctgatgg     1277
   tagtggcggt ggttggttta atcaccggcg taaccatgaa agagacggca aatcgtccgt     1337
   tgaaaggtgc gacaccggcg gcgtcagata tacaggaagc gaaggaaatt ctcgtcgagc     1397
   attacgataa tatcgagcag aaaatcgatg atattgacca cgagattgcc gatttgcagg     1457
   cgaaacgtac ccgcctggtg cagcaacatc cgcgaattga tgaataa                   1504
<210> 12
   <211> 288
   <212> PRT
   <213> Escherichia coli
<400> 12
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Tyr Ala Glu Leu Phe Val Ala
           275                 280                 285
<210> 13
   <211> 1502
   <212> DNA
   <213> Escherichia coli
<220>
   <221> CDS
   <222> (1)..(897)
   <223> Kodierregion proP M4-Allel (Deletion A nach Nukleotidposition 854)
<400> 13
   atg ctg aaa agg aaa aaa gta aaa ccg att acc ctt cgt gat gtc acc         48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg         96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct        144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag        192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg        240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag        288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt        336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att        384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa        432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa        480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt        528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa        576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg        624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act        672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt ttt tgt cgc ata        864
   Val Thr Tyr Tyr Met Leu Leu Thr Tyr Met Pro Ser Phe Cys Arg Ile
           275                 280                 285
   acc tgc att act ccg aag acc acg ggg tgc tga ttattatcgc cattatgatc      917
   Thr Cys Ile Thr Pro Lys Thr Thr Gly Cys
       290                 295
   ggtatgctgt ttgtccagcc ggtgatgggc ttgctgagtg accgttttgg ccgtcgtccg      977
   tttgtgctac ttggtagtgt tgccctgttt gtgttggcga tcccggcgtt tattctgatt     1037
   aacagtaacg tcatcggcct gatttttgcc gggttactga tgctggcggt gatccttaac     1097
   tgctttacgg gcgttatggc ttctaccttg ccagcgatgt tcccgacgca tatccgttac     1157
   agcgcgctgg cggcggcatt taatatttcg gtgctggttg ccggtctgac gccaacgctg     1217
   gcggcctggc tggtcgaaag ctcgcagaat ctgatgatgc ctgcctatta cctgatggta     1277
   gtggcggtgg ttggtttaat caccggcgta accatgaaag agacggcaaa tcgtccgttg     1337
   aaaggtgcga caccggcggc gtcagatata caggaagcga aggaaattct cgtcgagcat     1397
   tacgataata tcgagcagaa aatcgatgat attgaccacg agattgccga tttgcaggcg     1457
   aaacgtaccc gcctggtgca gcaacatccg cgaattgatg aataa                     1502
<210> 14
   <211> 298
   <212> PRT
   <213> Escherichia coli
<400> 14
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
               115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Phe Cys Arg Ile
           275                 280                 285
   Thr Cys Ile Thr Pro Lys Thr Thr Gly Cys
       290                 295
<210> 15
   <211> 1503
   <212> DNA
   <213> Escherichia coli
<220>
   <221> CDS
   <222> (1)..(1503)
   <223> Kodierregion proP M11-Allel (Austausch t zu c an Position 1399
   bedingt AA-Austausch Y467H)
<400> 15
   atg ctg aaa agg aaa aaa gta aaa ccg att acc ctt cgt gat gtc acc        48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg        96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct        144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag        192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg        240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag        288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt        336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att        384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa        432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa        480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt        528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa        576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg        624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act        672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat        864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att      912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac      960
   Met Ile Gly Met Leu Phe Val Gln Pro Val Met Gly Leu Leu Ser Asp
   305                 310                 315                 320
   cgt ttt ggc cgt cgt ccg ttt gtg cta ctt ggt agt gtt gcc ctg ttt     1008
   Arg Phe Gly Arg Arg Pro Phe Val Leu Leu Gly Ser Val Ala Leu Phe
                   325                 330                 335
   gtg ttg gcg atc ccg gcg ttt att ctg att aac agt aac gtc atc ggc     1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt     1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc     1152
   Thr Gly Val Met Ala Ser Thr Leu Pro Ala Met Phe Pro Thr His Ile
       370                 375                 380
   cgt tac agc gcg ctg gcg gcg gca ttt aat att tcg gtg ctg gtt gcc     1200
   Arg Tyr Ser Ala Leu Ala Ala Ala Phe Asn Ile Ser Val Leu Val Ala
   385                 390                 395                 400
   ggt ctg acg cca acg ctg gcg gcc tgg ctg gtc gaa agc tcg cag aat     1248
   Gly Leu Thr Pro Thr Leu Ala Ala Trp Leu Val Glu Ser Ser Gln Asn
                   405                 410                 415
   ctg atg atg cct gcc tat tac ctg atg gta gtg gcg gtg gtt ggt tta     1296
   Leu Met Met Pro Ala Tyr Tyr Leu Met Val Val Ala Val Val Gly Leu
               420                 425                 430
   atc acc ggc gta acc atg aaa gag acg gca aat cgt ccg ttg aaa ggt     1344
   Ile Thr Gly Val Thr Met Lys Glu Thr Ala Asn Arg Pro Leu Lys Gly
           435                 440                 445
   gcg aca ccg gcg gcg tca gat ata cag gaa gcg aag gaa att ctc gtc     1392
   Ala Thr Pro Ala Ala Ser Asp Ile Gln Glu Ala Lys Glu Ile Leu Val
       450                 455                 460
   gag cat cac gat aat atc gag cag aaa atc gat gat att gac cac gag     1440
   Glu His His Asp Asn Ile Glu Gln Lys Ile Asp Asp Ile Asp His Glu
   465                 470                 475                 480
   att gcc gat ttg cag gcg aaa cgt acc cgc ctg gtg cag caa cat ccg     1488
   Ile Ala Asp Leu Gln Ala Lys Arg Thr Arg Leu Val Gln Gln His Pro
                   485                 490                 495
   cga att gat gaa taa                                                 1503
   Arg Ile Asp Glu
               500
<210> 16
   <211> 500
   <212> PRT
   <213> Escherichia coli
<400> 16
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Tyr Leu Ser His
           275                 280                 285
   Asn Leu His Tyr Ser Glu Asp His Gly Val Leu Ile Ile Ile Ala Ile
       290                 295                 300
   Met Ile Gly Met Leu Phe Val Gln Pro Val Met Gly Leu Leu Ser Asp
   305                 310                 315                 320
   Arg Phe Gly Arg Arg Pro Phe Val Leu Leu Gly Ser Val Ala Leu Phe
                   325                 330                 335
   Val Leu Ala Ile Pro Ala Phe Ile Leu Ile Asn Ser Asn Val Ile Gly
               340                 345                 350
   Leu Ile Phe Ala Gly Leu Leu Met Leu Ala Val Ile Leu Asn Cys Phe
           355                 360                 365
   Thr Gly Val Met Ala Ser Thr Leu Pro Ala Met Phe Pro Thr His Ile
       370                 375                 380
   Arg Tyr Ser Ala Leu Ala Ala Ala Phe Asn Ile Ser Val Leu Val Ala
   385                 390                 395                 400
   Gly Leu Thr Pro Thr Leu Ala Ala Trp Leu Val Glu Ser Ser Gln Asn
                   405                 410                 415
   Leu Met Met Pro Ala Tyr Tyr Leu Met Val Val Ala Val Val Gly Leu
               420                 425                 430
   Ile Thr Gly Val Thr Met Lys Glu Thr Ala Asn Arg Pro Leu Lys Gly
           435                 440                 445
   Ala Thr Pro Ala Ala Ser Asp Ile Gln Glu Ala Lys Glu Ile Leu Val
       450                 455                 460
   Glu His His Asp Asn Ile Glu Gln Lys Ile Asp Asp Ile Asp His Glu
   465                 470                 475                 480
   Ile Ala Asp Leu Gln Ala Lys Arg Thr Arg Leu Val Gln Gln His Pro
                   485                 490                 495
   Arg Ile Asp Glu
               500
<210> 17
   <211> 1452
   <212> DNA
   <213> Escherichia coli
<220>
   <221> CDS
   <222> (1)..(1452)
   <223> Kodierregion proP M7-Allel (Deletion von 51bp von Nukleotidposition 1173 bis 1223
<400> 17
   atg ctg aaa agg aaa aaa gta aaa ccg att acc ctt cgt gat gtc acc        48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg        96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct       144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag       192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg       240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag       288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt       336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att       384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa       432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa       480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt       528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa       576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg       624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act       672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat        864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att        912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac        960
   Met Ile Gly Met Leu Phe Val Gln Pro Val Met Gly Leu Leu Ser Asp
   305                 310                 315                 320
   cgt ttt ggc cgt cgt ccg ttt gtg cta ctt ggt agt gtt gcc ctg ttt       1008
   Arg Phe Gly Arg Arg Pro Phe Val Leu Leu Gly Ser Val Ala Leu Phe
                   325                 330                 335
   gtg ttg gcg atc ccg gcg ttt att ctg att aac agt aac gtc atc ggc       1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt       1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc       1152
   Thr Gly Val Met Ala Ser Thr Leu Pro Ala Met Phe Pro Thr His Ile
       370                 375                 380
   cgt tac agc gcg ctg gcg gcc tgg ctg gtc gaa agc tcg cag aat ctg       1200
   Arg Tyr Ser Ala Leu Ala Ala Trp Leu Val Glu Ser Ser Gln Asn Leu
   385                 390                 395                 400
   atg atg cct gcc tat tac ctg atg gta gtg gcg gtg gtt ggt tta atc       1248
   Met Met Pro Ala Tyr Tyr Leu Met Val Val Ala Val Val Gly Leu Ile
                   405                 410                 415
   acc ggc gta acc atg aaa gag acg gca aat cgt ccg ttg aaa ggt gcg       1296
   Thr Gly Val Thr Met Lys Glu Thr Ala Asn Arg Pro Leu Lys Gly Ala
               420                 425                 430
   aca ccg gcg gcg tca gat ata cag gaa gcg aag gaa att ctc gtc gag       1344
   Thr Pro Ala Ala Ser Asp Ile Gln Glu Ala Lys Glu Ile Leu Val Glu
           435                 440                 445
   cat tac gat aat atc gag cag aaa atc gat gat att gac cac gag att       1392
   His Tyr Asp Asn Ile Glu Gln Lys Ile Asp Asp Ile Asp His Glu Ile
       450                 455                 460
   gcc gat ttg cag gcg aaa cgt acc cgc ctg gtg cag caa cat ccg cga       1440
   Ala Asp Leu Gln Ala Lys Arg Thr Arg Leu Val Gln Gln His Pro Arg
   465                 470                 475                 480
   att gat gaa taa                                                     1452
   Ile Asp Glu
<210> 18
   <211> 483
   <212> PRT
   <213> Escherichia coli
<400> 18
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
   210 215 220
   Pro Ala Phe Gln Gln His Val Asp Lys Leu Glu Gln Gly Asp Arg Glu
   225 230 235 240
   Gly Leu Gln Asp Gly Pro Lys Val Ser Phe Lys Glu Ile Ala Thr Lys
   245 250 255
   Tyr Trp Arg Ser Leu Leu Thr Cys Ile Gly Leu Val Ile Ala Thr Asn
   260 265 270
   Val Thr Tyr Tyr Met Leu Leu Thr Tyr Met Pro Ser Tyr Leu Ser His
   275 280 285
   Asn Leu His Tyr Ser Glu Asp His Gly Val Leu Ile Ile Ile Ala Ile
   290 295 300
   Met Ile Gly Met Leu Phe Val Gln Pro Val Met Gly Leu Leu Ser Asp
   305 310 315 320
   Arg Phe Gly Arg Arg Pro Phe Val Leu Leu Gly Ser Val Ala Leu Phe
   325 330 335
   Val Leu Ala Ile Pro Ala Phe Ile Leu Ile Asn Ser Asn Val Ile Gly
   340 345 350
   Leu Ile Phe Ala Gly Leu Leu Met Leu Ala Val Ile Leu Asn Cys Phe
   355 360 365
   Thr Gly Val Met Ala Ser Thr Leu Pro Ala Met Phe Pro Thr His Ile
   370 375 380
   Arg Tyr Ser Ala Leu Ala Ala Trp Leu Val Glu Ser Ser Gln Asn Leu
   385 390 395 400
   Met Met Pro Ala Tyr Tyr Leu Met Val Val Ala Val Val Gly Leu Ile
   405 410 415
   Thr Gly Val Thr Met Lys Glu Thr Ala Asn Arg Pro Leu Lys Gly Ala
   420 425 430
   Thr Pro Ala Ala Ser Asp Ile Gln Glu Ala Lys Glu Ile Leu Val Glu
   435 440 445
   His Tyr Asp Asn Ile Glu Gln Lys Ile Asp Asp Ile Asp His Glu Ile
   450 455 460
   Ala Asp Leu Gln Ala Lys Arg Thr Arg Leu Val Gln Gln His Pro Arg
   465 470 475 480
   Ile Asp Glu
<210> 19
   <211> 1522
   <212> DNA
   <213> Escherichia coli
<220>
   <221> CDS
   <222> (1)..(1014)
   <223> Kodierregion proP M6-Allel (19bp Insertion nach Nukleotidposition 973)
<400> 19
   atg ctg aaa agg aaa aaa gta aaa ccg att acc ctt cgt gat gtc acc      48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg      96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct     144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag     192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg     240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag     288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt     336
   Lys Ile Leu Ala Ile Thr Ile Val Ile Met Ser Ile Ser Thr Phe Cys
               100                 105                 110
   att ggc tta ata ccg tcc tac gac acg att ggt att tgg gca ccg att     384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa     432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa     480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt     528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa        576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg        624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act        672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat        864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att        912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac        960
   Met Ile Gly Met Leu Phe Val Gln Pro Val Met Gly Leu Leu Ser Asp
   305                 310                 315                 320
   cgt ttt ggc cgt cgt ccg ttt gtg cta ctt gcg tcc gtt tgt gct act       1008
   Arg Phe Gly Arg Arg Pro Phe Val Leu Leu Ala Ser Val Cys Ala Thr
                   325                 330                 335
   tgg tag tgttgccctg tttgtgttgg cgatcccggc gtttattctg attaacagta        1064
   Trp
   acgtcatcgg cctgattttt gccgggttac tgatgctggc ggtgatcctt aactgcttta     1124
   cgggcgttat ggcttctacc ttgccagcga tgttcccgac gcatatccgt tacagcgcgc     1184
   tggcggcggc atttaatatt tcggtgctgg ttgccggtct gacgccaacg ctggcggcct     1244
   ggctggtcga aagctcgcag aatctgatga tgcctgccta ttacctgatg gtagtggcgg     1304
   tggttggttt aatcaccggc gtaaccatga aagagacggc aaatcgtccg ttgaaaggtg     1364
   cgacaccggc ggcgtcagat atacaggaag cgaaggaaat tctcgtcgag cattacgata     1424
   atatcgagca gaaaatcgat gatattgacc acgagattgc cgatttgcag gcgaaacgta     1484
   cccgcctggt gcagcaacat ccgcgaattg atgaataa                             1522
<210> 20
   <211> 337
   <212> PRT
   <213> Escherichia coli
<400> 20
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Tyr Leu Ser His
           275                 280                 285
   Asn Leu His Tyr Ser Glu Asp His Gly Val Leu Ile Ile Ile Ala Ile
       290                 295                 300
   Met Ile Gly Met Leu Phe Val Gln Pro Val Met Gly Leu Leu Ser Asp
   305                 310                 315                 320
   Arg Phe Gly Arg Arg Pro Phe Val Leu Leu Ala Ser Val Cys Ala Thr
                   325                 330                 335
   Trp
<210> 21
   <211> 1503
   <212> DNA
   <213> Escherichia coli
<220>
   <221> CDS
   <222> (1)..(1503)
   <223> Kodierregion proP M5-Allel (Austausch g zu t an Position 971 bedingt
   AA-Austausch R324L)
<400> 21
   atg ctg aaa agg aaa aaa gta aaa ccg att acc ctt cgt gat gtc acc          48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg          96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct         144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc agc gtg cag         192
   Tyr Ala Leu Gly Lys Val Phe Phe Pro Gly Ala Asp Pro Ser Val Gln
       50                  55                  60
   atg gtt gct gca ctt gcc act ttc tcc gtt ccc ttt ctg att cga ccg         240
   Met Val Ala Ala Leu Ala Thr Phe Ser Val Pro Phe Leu Ile Arg Pro
   65                  70                  75                  80
   ctt ggc gga ctc ttc ttt ggt atg ttg ggc gat aaa tat ggt cgc cag         288
   Leu Gly Gly Leu Phe Phe Gly Met Leu Gly Asp Lys Tyr Gly Arg Gln
                   85                  90                  95
   aag atc ctc gct atc act att gtg att atg tcg atc agt acg ttc tgt         336
   Lys Ile Leu Ala Ile Thr Ile Val Ile Met Ser Ile Ser Thr Phe Cys
                   100                 105                 110
   att ggc tta ata ccg tcc tac gac acg att ggt att tgg gca ccg att        384
   Ile Gly Leu Ile Pro Ser Tyr Asp Thr Ile Gly Ile Trp Ala Pro Ile
           115                 120                 125
   ctg ctg ttg atc tgt aag atg gca caa ggt ttc tcg gtc ggc ggt gaa        432
   Leu Leu Leu Ile Cys Lys Met Ala Gln Gly Phe Ser Val Gly Gly Glu
       130                 135                 140
   tat acc ggg gcg tcg ata ttt gtt gcg gaa tac tcc cct gac cgt aaa        480
   Tyr Thr Gly Ala Ser Ile Phe Val Ala Glu Tyr Ser Pro Asp Arg Lys
   145                 150                 155                 160
   cgt ggc ttt atg ggc agc tgg ctg gac ttc ggt tct att gcc ggg ttt        528
   Arg Gly Phe Met Gly Ser Trp Leu Asp Phe Gly Ser Ile Ala Gly Phe
                   165                 170                 175
   gtg ctg ggt gcg ggc gtg gtg gtg tta att tcg acc att gtc ggc gaa        576
   Val Leu Gly Ala Gly Val Val Val Leu Ile Ser Thr Ile Val Gly Glu
               180                 185                 190
   gcg aac ttc ctc gat tgg ggc tgg cgt att ccg ttc ttt atc gct ctg        624
   Ala Asn Phe Leu Asp Trp Gly Trp Arg Ile Pro Phe Phe Ile Ala Leu
           195                 200                 205
   ccg tta ggg att atc ggg ctt tac ctg cgc cat gcg ctg gaa gag act        672
   Pro Leu Gly Ile Ile Gly Leu Tyr Leu Arg His Ala Leu Glu Glu Thr
       210                 215                 220
   ccg gcg ttc cag cag cat gtc gat aaa ctg gaa cag ggc gac cgt gaa        720
   Pro Ala Phe Gln Gln His Val Asp Lys Leu Glu Gln Gly Asp Arg Glu
   225                 230                 235                 240
   ggt ttg cag gat ggc ccg aaa gtc tcg ttt aaa gag att gcc act aaa        768
   Gly Leu Gln Asp Gly Pro Lys Val Ser Phe Lys Glu Ile Ala Thr Lys
                   245                 250                 255
   tac tgg cgc agc ctg ttg aca tgt att ggt ctg gta att gcc acc aac        816
   Tyr Trp Arg Ser Leu Leu Thr Cys Ile Gly Leu Val Ile Ala Thr Asn
               260                 265                 270
   gtg act tac tac atg ttg ctg acc tat atg ccg agt tat ttg tcg cat        864
   Val Thr Tyr Tyr Met Leu Leu Thr Tyr Met Pro Ser Tyr Leu Ser His
           275                 280                 285
   aac ctg cat tac tcc gaa gac cac ggg gtg ctg att att atc gcc att        912
   Asn Leu His Tyr Ser Glu Asp His Gly Val Leu Ile Ile Ile Ala Ile
       290                 295                 300
   atg atc ggt atg ctg ttt gtc cag ccg gtg atg ggc ttg ctg agt gac        960
   Met Ile Gly Met Leu Phe Val Gln Pro Val Met Gly Leu Leu Ser Asp
   305                 310                 315                 320
   cgt ttt ggc ctt cgt ccg ttt gtg cta ctt ggt agt gtt gcc ctg ttt       1008
   Arg Phe Gly Leu Arg Pro Phe Val Leu Leu Gly Ser Val Ala Leu Phe
                   325                 330                 335
   gtg ttg gcg atc ccg gcg ttt att ctg att aac agt aac gtc atc ggc       1056
   Val Leu Ala Ile Pro Ala Phe Ile Leu Ile Asn Ser Asn Val Ile Gly
               340                 345                 350
   ctg att ttt gcc ggg tta ctg atg ctg gcg gtg atc ctt aac tgc ttt       1104
   Leu Ile Phe Ala Gly Leu Leu Met Leu Ala Val Ile Leu Asn Cys Phe
           355                 360                 365
   acg ggc gtt atg gct tct acc ttg cca gcg atg ttc ccg acg cat atc       1152
   Thr Gly Val Met Ala Ser Thr Leu Pro Ala Met Phe Pro Thr His Ile
       370                 375                 380
   cgt tac agc gcg ctg gcg gcg gca ttt aat att tcg gtg ctg gtt gcc       1200
   Arg Tyr Ser Ala Leu Ala Ala Ala Phe Asn Ile Ser Val Leu Val Ala
   385                 390                 395                 400
   ggt ctg acg cca acg ctg gcg gcc tgg ctg gtc gaa agc tcg cag aat       1248
   Gly Leu Thr Pro Thr Leu Ala Ala Trp Leu Val Glu Ser Ser Gln Asn
                   405                 410                 415
   ctg atg atg cct gcc tat tac ctg atg gta gtg gcg gtg gtt ggt tta       1296
   Leu Met Met Pro Ala Tyr Tyr Leu Met Val Val Ala Val Val Gly Leu
               420                 425                 430
   atc acc ggc gta acc atg aaa gag acg gca aat cgt ccg ttg aaa ggt       1344
   Ile Thr Gly Val Thr Met Lys Glu Thr Ala Asn Arg Pro Leu Lys Gly
           435                 440                 445
   gcg aca ccg gcg gcg tca gat ata cag gaa gcg aag gaa att ctc gtc       1392
   Ala Thr Pro Ala Ala Ser Asp Ile Gln Glu Ala Lys Glu Ile Leu Val
       450                 455                 460
   gag cat tac gat aat atc gag cag aaa atc gat gat att gac cac gag       1440
   Glu His Tyr Asp Asn Ile Glu Gln Lys Ile Asp Asp Ile Asp His Glu
   465                 470                 475                 480
   att gcc gat ttg cag gcg aaa cgt acc cgc ctg gtg cag caa cat ccg       1488
   Ile Ala Asp Leu Gln Ala Lys Arg Thr Arg Leu Val Gln Gln His Pro
                   485                 490                 495
   cga att gat gaa taa                                                   1503
   Arg Ile Asp Glu
               500
<210> 22
   <211> 500
   <212> PRT
   <213> Escherichia coli
<400> 22
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ser Val Gln
       50                  55                  60
   Met Val Ala Ala Leu Ala Thr Phe Ser Val Pro Phe Leu Ile Arg Pro
   65                  70                  75                  80
   Leu Gly Gly Leu Phe Phe Gly Met Leu Gly Asp Lys Tyr Gly Arg Gln
                   85                  90                  95
   Lys Ile Leu Ala Ile Thr Ile Val Ile Met Ser Ile Ser Thr Phe Cys
               100                 105                 110
   Ile Gly Leu Ile Pro Ser Tyr Asp Thr Ile Gly Ile Trp Ala Pro Ile
           115                 120                 125
   Leu Leu Leu Ile Cys Lys Met Ala Gln Gly Phe Ser Val Gly Gly Glu
       130                 135                 140
   Tyr Thr Gly Ala Ser Ile Phe Val Ala Glu Tyr Ser Pro Asp Arg Lys
   145                 150                 155                 160
   Arg Gly Phe Met Gly Ser Trp Leu Asp Phe Gly Ser Ile Ala Gly Phe
                   165                 170                 175
   Val Leu Gly Ala Gly Val Val Val Leu Ile Ser Thr Ile Val Gly Glu
               180                 185                 190
   Ala Asn Phe Leu Asp Trp Gly Trp Arg Ile Pro Phe Phe Ile Ala Leu
           195                 200                 205
   Pro Leu Gly Ile Ile Gly Leu Tyr Leu Arg His Ala Leu Glu Glu Thr
       210                 215                 220
   Pro Ala Phe Gln Gln His Val Asp Lys Leu Glu Gln Gly Asp Arg Glu
   225                 230                 235                 240
   Gly Leu Gln Asp Gly Pro Lys Val Ser Phe Lys Glu Ile Ala Thr Lys
                   245                 250                 255
   Tyr Trp Arg Ser Leu Leu Thr Cys Ile Gly Leu Val Ile Ala Thr Asn
               260                 265                 270
   Val Thr Tyr Tyr Met Leu Leu Thr Tyr Met Pro Ser Tyr Leu Ser His
           275                 280                 285
   Asn Leu His Tyr Ser Glu Asp His Gly Val Leu Ile Ile Ile Ala Ile
       290                 295                 300
   Met Ile Gly Met Leu Phe Val Gln Pro Val Met Gly Leu Leu Ser Asp
   305                 310                 315                 320
   Arg Phe Gly Leu Arg Pro Phe Val Leu Leu Gly Ser Val Ala Leu Phe
                   325                 330                 335
   Val Leu Ala Ile Pro Ala Phe Ile Leu Ile Asn Ser Asn Val Ile Gly
   340 345 350
   Leu Ile Phe Ala Gly Leu Leu Met Leu Ala Val Ile Leu Asn Cys Phe
   355 360 365
   Thr Gly Val Met Ala Ser Thr Leu Pro Ala Met Phe Pro Thr His Ile
   370 375 380
   Arg Tyr Ser Ala Leu Ala Ala Ala Phe Asn Ile Ser Val Leu Val Ala
   385 390 395 400
   Gly Leu Thr Pro Thr Leu Ala Ala Trp Leu Val Glu Ser Ser Gln Asn
   405 410 415
   Leu Met Met Pro Ala Tyr Tyr Leu Met Val Val Ala Val Val Gly Leu
   420 425 430
   Ile Thr Gly Val Thr Met Lys Glu Thr Ala Asn Arg Pro Leu Lys Gly
   435 440 445
   Ala Thr Pro Ala Ala Ser Asp Ile Gln Glu Ala Lys Glu Ile Leu Val
   450 455 460
   Glu His Tyr Asp Asn Ile Glu Gln Lys Ile Asp Asp Ile Asp His Glu
   465 470 475 480
   Ile Ala Asp Leu Gln Ala Lys Arg Thr Arg Leu Val Gln Gln His Pro
   485 490 495
   Arg Ile Asp Glu
   500
<210> 23
   <211> 2852
   <212> DNA
   <213> Escherichia coli
<220>
   <221> CDS
   <222> (1)..(243)
   <223> Kodierregion proP M2-Allel (1359bp Insertion nach Nukleotidposition 183)
<400> 23
   atg ctg aaa agg aaa aaa gta aaa ccg att acc ctt cgt gat gtc acc         48
   Met Leu Lys Arg Lys Lys Val Lys Pro Ile Thr Leu Arg Asp Val Thr
   1               5                   10                  15
   att att gat gac ggt aaa ctg cgt aaa gcc att acc gca gca tca ctg         96
   Ile Ile Asp Asp Gly Lys Leu Arg Lys Ala Ile Thr Ala Ala Ser Leu
               20                  25                  30
   ggt aat gca atg gaa tgg ttc gat ttt ggt gtt tat ggt ttt gtt gct        144
   Gly Asn Ala Met Glu Trp Phe Asp Phe Gly Val Tyr Gly Phe Val Ala
           35                  40                  45
   tac gca tta ggt aaa gtt ttt ttc ccg ggg gct gac ccc ata agc gct        192
   Tyr Ala Leu Gly Lys Val Phe Phe Pro Gly Ala Asp Pro Ile Ser Ala
       50                  55                  60
   aac tta agg gtt gaa cca tct gaa gaa tgc gac gcc tcg gtg cct cgt        240
   Asn Leu Arg Val Glu Pro Ser Glu Glu Cys Asp Ala Ser Val Pro Arg
   65                  70                  75                  80
   taa gacgatgcct cgcgttcttc aattgcgttt tgtaggctgt cagggatact            293
   gtcccacgaa tggccacctg taagctccag atgaccattt ttgttattct ccacaacgag     353
   ttagttcttc ttttcggatc cggcacttct gggggggaaa tccagcgatg gctggattat    413
   gtcgtcaatt aaaaatgcgg cgagtagatt agcaaatatc cacgctttcg cgagttcagg     473
   ttcctttgca cgcaaagcat ccaggtgcag caaacttttg agccgcttaa aagccagttc     533
   aatttgccat cgcagacggt aacaatcagc cacttgctct gctgaatatt catcttccgg     593
   taatgatgtt agcaatagca catggcccgc tgcttccagc gtttccgcct gaactactcg     653
   tccttttcga cgattctcgc tgagcagtcg ggttttactg attaatgctt tttcgggagg     713
   aagtgatacg gcaatgagac gtgccggaaa gggagctccg gcttttttat tacctgaatt    773
   gcctatcatt acagtggttt caccgttctt accgcaatcc agcccgcgca gaaaacccat     833
   catgtcaaag cgcattcctt ctgcagttaa ccagcgcaat cctcgccagt gaacccggac     893
   gatataatca gcttctccaa aagcaagtga gcggatacat tcgggacgcg aaccgaatcc     953
   ccggtcagca atgcgtatct cgtctgccgt ttgcgcaaat cggtccagcc gttcagcgtc    1013
   tctgctgtcg gttagctcaa aatcagtgaa ctgacaggta tgaggatcat atcccatatg    1073
   tagtcgccat tcagcgctgc cgcccccggg cgcactgatt gctgttccat cgacaagacg    1133
   caatctcttt ccgcttgtac aacccgtaac tgcggcgcgt acagcaagtg tttgtgcggc    1193
   aagtatgcca aaccagtcgg cggcattccg cagccgcttc aggagagcca cgtcagataa    1253
   tgttgcaacg tcatggagct gagcccatgc agtgacttca cgtaatgaca tccccccggg    1313
   gccgtaagcc agccccagac gtagcagagt tgcagcatca cgaatttcgc ggcggcgggt    1373
   tagagccccg gcattacgtg ccgaagtatc cagttcttcg ggcttaccaa tatgggccag    1433
   aattgctgac cagttatcgt gagagtaatt catcggcacg ttaaatcata tcaggcgtaa    1493
   taccacaacc cttaagttag cgcttatggg gctgacccca gcgtgcagat ggttgctgca    1553
   cttgccactt tctccgttcc ctttctgatt cgaccgcttg gcggactctt ctttggtatg    1613
   ttgggcgata aatatggtcg ccagaagatc ctcgctatca ctattgtgat tatgtcgatc    1673
   agtacgttct gtattggctt aataccgtcc tacgacacga ttggtatttg ggcaccgatt    1733
   ctgctgttga tctgtaagat ggcacaaggt ttctcggtcg gcggtgaata taccggggcg    1793
   tcgatatttg ttgcggaata ctcccctgac cgtaaacgtg gctttatggg cagctggctg    1853
   gacttcggtt ctattgccgg gtttgtgctg ggtgcgggcg tggtggtgtt aatttcgacc     1913
   attgtcggcg aagcgaactt cctcgattgg ggctggcgta ttccgttctt tatcgctctg     1973
   ccgttaggga ttatcgggct ttacctgcgc catgcgctgg aagagactcc ggcgttccag     2033
   cagcatgtcg ataaactgga acagggcgac cgtgaaggtt tgcaggatgg cccgaaagtc     2093
   tcgtttaaag agattgccac taaatactgg cgcagcctgt tgacatgtat tggtctggta     2153
   attgccacca acgtgactta ctacatgttg ctgacctata tgccgagtta tttgtcgcat     2213
   aacctgcatt actccgaaga ccacggggtg ctgattatta tcgccattat gatcggtatg     2273
   ctgtttgtcc agccggtgat gggcttgctg agtgaccgtt ttggccgtcg tccgtttgtg     2333
   ctacttggta gtgttgccct gtttgtgttg gcgatcccgg cgtttattct gattaacagt     2393
   aacgtcatcg gcctgatttt tgccgggtta ctgatgctgg cggtgatcct taactgcttt     2453
   acgggcgtta tggcttctac cttgccagcg atgttcccga cgcatatccg ttacagcgcg     2513
   ctggcggcgg catttaatat ttcggtgctg gttgccggtc tgacgccaac gctggcggcc     2573
   tggctggtcg aaagctcgca gaatctgatg atgcctgcct attacctgat ggtagtggcg     2633
   gtggttggtt taatcaccgg cgtaaccatg aaagagacgg caaatcgtcc gttgaaaggt     2693
   gcgacaccgg cggcgtcaga tatacaggaa gcgaaggaaa ttctcgtcga gcattacgat     2753
   aatatcgagc agaaaatcga tgatattgac cacgagattg ccgatttgca ggcgaaacgt     2813
   acccgcctgg tgcagcaaca tccgcgaatt gatgaataa                           2852
<210> 24
   <211> 80
   <212> PRT
   <213> Escherichia coli
<400> 24
   Met Leu Lys Arg Lys Lys Val Lys Pro Ile Thr Leu Arg Asp Val Thr
   1               5                   10                  15
   Ile Ile Asp Asp Gly Lys Leu Arg Lys Ala Ile Thr Ala Ala Ser Leu
               20                  25                  30
   Gly Asn Ala Met Glu Trp Phe Asp Phe Gly Val Tyr Gly Phe Val Ala
           35                  40                  45
   Tyr Ala Leu Gly Lys Val Phe Phe Pro Gly Ala Asp Pro Ile Ser Ala
       50                  55                  60
   Asn Leu Arg Val Glu Pro Ser Glu Glu Cys Asp Ala Ser Val Pro Arg
   65                  70                  75                  80
<210> 25
   <211> 38
   <212> DNA
   <213> Escherichia coli
<220>
   <221> misc_feature
   <222> (1)..(38)
   <223> Primer serCF(XbaI)
<400> 25
   aggtgctcta gagtccgcgc tgtgcaaatc cagaatgg 38
<210> 26
   <211> 37
   <212> DNA
   <213> Escherichia coli
<220>
   <221> misc_feature
   <222> (1)..(37)
   <223> Primer serCR(HindIII)
<400> 26
   tacaccaagc ttaactctct acaacagaaa taaaaac 37
<210> 27
   <211> 33
   <212> DNA
   <213> Escherichia coli
<220>
   <221> misc_feature
   <222> (1)..(33)
   <223> Primer serAF(XbaI)
<400> 27
   ctgtagtcta gattagtaca gcagacgggc gcg 33
<210> 28
   <211> 68
   <212> DNA
   <213> Escherichia coli
<220>
   <221> misc_feature
   <222> (1)..(68)
   <223> Primer serAR(SHSSNB)
<400> 28
   caagagctca agcttgcatg cgattcccgg gcggccgcaa taagatctcc gtcagggcgt 60
   ggtgaccg 68
<210> 29
   <211> 34
   <212> DNA
   <213> Escherichia coli
<220>
   <221> misc_feature
   <222> (1)..(34)
   <223> Primer serB(SphI)
<400> 29
   ccatgcgcat gcccaccctt tgaaaatttg agac 34
<210> 30
   <211> 75
   <212> DNA
   <213> Escherichia coli
<220>
   <221> misc_feature
   <222> (1)..(75)
   <223> Primer serB(SmaI)
<400> 30
   ccgcatgtcg acatcccggg gcagaaaggc ccacccgaag gtgagccagt gtgattactt 60
   ctgattcagg ctgcc 75
<210> 31
   <211> 35
   <212> DNA
   <213> Escherichia coli
<220>
   <221> misc_feature
   <222> (1)..(35)
   <223> Primer glyA-downstream
<400> 31
   atctaaagat ctgttacgac agatttgatg gcgcg 35
<210> 32
   <211> 33
   <212> DNA
   <213> Escherichia coli
<220>
   <221> misc_feature
   <222> (1)..(33)
   <223> glyA-upstream
<400> 32
   ttcatcgcgg ccgcgaaaga atgtgatgaa gtg 33
<210> 33
   <211> 30
   <212> DNA
   <213> Escherichia coli
<220>
   <221> misc_feature
   <222> (1)..(30)
   <223> Primer proPmut1(HindIII)
<400> 33
   gtcaaagctt atatggtcgc cagaagatcc 30
<210> 34
   <211> 30
   <212> DNA
   <213> Escherichia coli
<220>
   <221> misc_feature
   <222> (1)..(30)
   <223> Primer proPmut2(BamHI)
<400> 34
   gtcaggatcc tcagccgcat tacacagttg 30
<210> 35
   <211> 1564
   <212> DNA
   <213> Escherichia coli
<220>
   <221> misc_feature
   <222> (5)..(10)
   <223> Erkennungssequenz HindIII
<220>
   <221> mutation
   <222> (969)..(969)
   <223> M8-Mutation: Austausch a zu t an Position 1234, bedingt E412*
<220>
   <221> misc_feature
   <222> (1534)..(1539)
   <223> Erkennungssequenz BamHI
<400> 35
   gtcaaagctt atatggtcgc cagaagatcc tcgctatcac tattgtgatt atgtcgatca      60
   gtacgttctg tattggctta ataccgtcct acgacacgat tggtatttgg gcaccgattc     120
   tgctgttgat ctgtaagatg gcacaaggtt tctcggtcgg cggtgaatat accggggcgt     180
   cgatatttgt tgcggaatac tcccctgacc gtaaacgtgg ctttatgggc agctggctgg     240
   acttcggttc tattgccggg tttgtgctgg gtgcgggcgt ggtggtgtta atttcgacca     300
   ttgtcggcga agcgaacttc ctcgattggg gctggcgtat tccgttcttt atcgctctgc     360
   cgttagggat tatcgggctt tacctgcgcc atgcgctgga agagactccg gcgttccagc     420
   agcatgtcga taaactggaa cagggcgacc gtgaaggttt gcaggatggc ccgaaagtct     480
   cgtttaaaga gattgccact aaatactggc gcagcctgtt gacatgtatt ggtctggtaa     540
   ttgccaccaa cgtgacttac tacatgttgc tgacctatat gccgagttat ttgtcgcata     600
   acctgcatta ctccgaagac cacggggtgc tgattattat cgccattatg atcggtatgc     660
   tgtttgtcca gccggtgatg ggcttgctga gtgaccgttt tggccgtcgt ccgtttgtgc     720
   tacttgcgtc cgtttgtgct acttggtagt gttgccctgt ttgtgttggc gatcccggcg     780
   tttattctga ttaacagtaa cgtcatcggc ctgatttttg ccgggttact gatgctggcg     840
   gtgatcctta actgctttac gggcgttatg gcttctacct tgccagcgat gttcccgacg     900
   catatccgtt acagcgcgct ggcggcggca tttaatattt cggtgctggt tgccggtctg     960
   acgccaacgc tggcggcctg gctggtcgaa agctcgcaga atctgatgat gcctgcctat    1020
   tacctgatgg tagtggcggt ggttggttta atcaccggcg taaccatgaa agagacggca    1080
   aatcgtccgt tgaaaggtgc gacaccggcg gcgtcagata tacaggaagc gaaggaaatt    1140
   ctcgtcgagc attacgataa tatcgagcag aaaatcgatg atattgacca cgagattgcc    1200
   gatttgcagg cgaaacgtac ccgcctggtg cagcaacatc cgcgaattga tgaataagct    1260
   gaaacggatg gcctgatgtg acgctgtctt atcaggccaa ttgaactctt aaggttcact    1320
   taatctctga cgcgcatact ctcctccagg ttaacggagg agagtgcaat gaaaaaccgt    1380
   gtttatgaaa gtttaactac cgtgttcagc gtgctggtgg tcagcagctt tctttatatc    1440
   tggtttgcca cgtactgatc tttcttcagc cgtacccagg cccgcgtgcc ggaagtctct    1500
   tgccggtttt gcaggaaaaa ctgcccgtga tgcaactgtg taatgcggct gagcggccgc    1560
   tcag                                                                1564
<210> 36
   <211> 1542
   <212> DNA
   <213> Escherichia coli
<220>
   <221> misc_feature
   <222> (5)..(10)
   <223> Erkennungssequenz HindIII
<220>
   <221> mutation
   <222> (588)..(589)
   <223> M4-Mutation: Deletion eines A nach Nukleotidposition 854
<220>
   <221> misc_feature
   <222> (1533)..(538)
   <223> Erkennungssequenz BamHI
<400> 36
   gtcaaagctt atatggtcgc cagaagatcc tcgctatcac tattgtgatt atgtcgatca 60
   gtacgttctg tattggctta ataccgtcct acgacacgat tggtatttgg gcaccgattc 120
   tgctgttgat ctgtaagatg gcacaaggtt tctcggtcgg cggtgaatat accggggcgt 180
   cgatatttgt tgcggaatac tcccctgacc gtaaacgtgg ctttatgggc agctggctgg 240
   acttcggttc tattgccggg tttgtgctgg gtgcgggcgt ggtggtgtta atttcgacca 300
   ttgtcggcga agcgaacttc ctcgattggg gctggcgtat tccgttcttt atcgctctgc 360
   cgttagggat tatcgggctt tacctgcgcc atgcgctgga agagactccg gcgttccagc 420
   agcatgtcga taaactggaa cagggcgacc gtgaaggttt gcaggatggc ccgaaagtct 480
   cgtttaaaga gattgccact aaatactggc gcagcctgtt gacatgtatt ggtctggtaa 540
   ttgccaccaa cgtgacttac tacatgttgc tgacctatat gccgagtttt tgtcgcataa 600
   cctgcattac tccgaagacc acggggtgct gattattatc gccattatga tcggtatgct 660
   gtttgtccag ccggtgatgg gcttgctgag tgaccgtttt ggccgtcgtc cgtttgtgct 720
   acttggtagt gttgccctgt ttgtgttggc gatcccggcg tttattctga ttaacagtaa 780
   cgtcatcggc ctgatttttg ccgggttact gatgctggcg gtgatcctta actgctttac 840
   gggcgttatg gcttctacct tgccagcgat gttcccgacg catatccgtt acagcgcgct 900
   ggcggcggca tttaatattt cggtgctggt tgccggtctg acgccaacgc tggcggcctg 960
   gctggtcgaa agctcgcaga atctgatgat gcctgcctat tacctgatgg tagtggcggt 1020
   ggttggttta atcaccggcg taaccatgaa agagacggca aatcgtccgt tgaaaggtgc 1080
   gacaccggcg gcgtcagata tacaggaagc gaaggaaatt ctcgtcgagc attacgataa 1140
   tatcgagcag aaaatcgatg atattgacca cgagattgcc gatttgcagg cgaaacgtac 1200
   ccgcctggtg cagcaacatc cgcgaattga tgaataagct gaaacggatg gcctgatgtg 1260
   acgctgtctt atcaggccaa ttgaactctt aaggttcact taatctctga cgcgcatact 1320
   ctcctccagg ttaacggagg agagtgcaat gaaaaaccgt gtttatgaaa gtttaactac 1380
   cgtgttcagc gtgctggtgg tcagcagctt tctttatatc tggtttgcca cgtactgatc 1440
   tttcttcagc cgtacccagg cccgcgtgcc ggaagtctct tgccggtttt gcaggaaaaa 1500
   ctgcccgtga tgcaactgtg taatgcggct gaggatcctg ac 1542
<210> 37
   <211> 1563
   <212> DNA
   <213> Escherichia coli
<220>
   <221> misc_feature
   <222> (5)..(10)
   <223> Erkennungssequenz HindIII
<220>
   <221> mutation
   <222> (722)..(740)
   <223> M6-Mutation: 19bp Insertion nach Nukleotidposition 973
<220>
   <221> misc_feature
   <222> (1553)..(1558)
   <223> Erkennungssequenz BamHI
<400> 37
   gtcaaagctt atatggtcgc cagaagatcc tcgctatcac tattgtgatt atgtcgatca      60
   gtacgttctg tattggctta ataccgtcct acgacacgat tggtatttgg gcaccgattc     120
   tgctgttgat ctgtaagatg gcacaaggtt tctcggtcgg cggtgaatat accggggcgt     180
   cgatatttgt tgcggaatac tcccctgacc gtaaacgtgg ctttatgggc agctggctgg     240
   acttcggttc tattgccggg tttgtgctgg gtgcgggcgt ggtggtgtta atttcgacca     300
   ttgtcggcga agcgaacttc ctcgattggg gctggcgtat tccgttcttt atcgctctgc     360
   cgttagggat tatcgggctt tacctgcgcc atgcgctgga agagactccg gcgttccagc     420
   agcatgtcga taaactggaa cagggcgacc gtgaaggttt gcaggatggc ccgaaagtct     480
   cgtttaaaga gattgccact aaatactggc gcagcctgtt gacatgtatt ggtctggtaa     540
   ttgccaccaa cgtgacttac tacatgttgc tgacctatat gccgagttat ttgtcgcata     600
   acctgcatta ctccgaagac cacggggtgc tgattattat cgccattatg atcggtatgc     660
   tgtttgtcca gccggtgatg ggcttgctga gtgaccgttt tggccgtcgt ccgtttgtgc     720
   tacttgcgtc cgtttgtgct acttggtagt gttgccctgt ttgtgttggc gatcccggcg     780
   tttattctga ttaacagtaa cgtcatcggc ctgatttttg ccgggttact gatgctggcg     840
   gtgatcctta actgctttac gggcgttatg gcttctacct tgccagcgat gttcccgacg     900
   catatccgtt acagcgcgct ggcggcggca tttaatattt cggtgctggt tgccggtctg     960
   acgccaacgc tggcggcctg gctggtcgaa agctcgcaga atctgatgat gcctgcctat    1020
   tacctgatgg tagtggcggt ggttggttta atcaccggcg taaccatgaa agagacggca    1080
   aatcgtccgt tgaaaggtgc gacaccggcg gcgtcagata tacaggaagc gaaggaaatt    1140
   ctcgtcgagc attacgataa tatcgagcag aaaatcgatg atattgacca cgagattgcc    1200
   gatttgcagg cgaaacgtac ccgcctggtg cagcaacatc cgcgaattga tgaataagct    1260
   gaaacggatg gcctgatgtg acgctgtctt atcaggccaa ttgaactctt aaggttcact    1320
   taatctctga cgcgcatact ctcctccagg ttaacggagg agagtgcaat gaaaaaccgt    1380
   gtttatgaaa gtttaactac cgtgttcagc gtgctggtgg tcagcagctt tctttatatc    1440
   tggtttgcca cgtactgatc tttcttcagc cgtacccagg cccgcgtgcc ggaagtctct    1500
   tgccggtttt gcaggaaaaa ctgcccgtga tgcaactgtg taatgcggct gagggatcct    1560
   cag                                                                  1563
<210> 38
   <211> 3503
   <212> DNA
   <213> Escherichia coli
<220>
   <221> gene
   <222> (1001)..(2503)
   <223> Kodierregion proP
<400> 38
   ttctttcgcg tgtgcagctt cagacatgga cgaacggcgg catgttaaat gcgccgctta      60
   gcctgcgtct gacattggtg gaaaaactgg cgtcgatgct ggatcccggt catctggcac     120
   tgacgcagat cgcgcagcat ctggcgctgc tgcaaaaaat ggatcaccgc cagcactctg     180
   ctttcccgga gctcccccag caaattgccg ccttgtatga gtggttttca gcccgttgtc     240
   gctggaagga aaaggcgtta acgcaacgag gcctactggt gcaggcaggt gatcagagcg     300
   agcaaatttt tacccgctgg cgtgctgggg cgtataacgc ctggtcgttg cctgggcgct     360
   gttttatcgt tctggaggag ttgcgctggg gggcatttgg cgatgcctgc cgtctgggaa     420
   gcccgcaagc ggtggcgttg ttgctgggtg atttgctcga gaaagcgaca caacatctgg     480
   cagagagtat caatgcggca ccgaccacgc gtcactatta ccatcagtgg tttgcctctt     540
   cgaccgttcc gacgggcggg gagcatgctg attttttaag ttggctggga aagtggacca     600
   cggcagataa acaacccgtt tgctggtcag tgacccaacg ctggcaaact gtcgcgctgg     660
   ggatgccacg actctgttca gcgcagcgtc tggcgggggc aatgctcgag gaaatcttct     720
   ctgtaaattt ggcgtaaata atcagttaca tcaatgagtc ctaaacgaaa tccatgtgtg     780
   aagttgatca caaatttaaa cactggtagg gtaaaaaggt cattaactgc ccaattcagg     840
   cgtcaactgg tttgattgta cattccttaa ccggagggtg taagcaaacc cgctacgctt     900
   gttacagaga ttgcatcctg caattcccgc tccccttttg cggccgtcgc gctgattttt      960
   ctggcgtttg cggaaatggg ccaactctgc gaggaaagct atgctgaaaa ggaaaaaagt    1020
   aaaaccgatt acccttcgtg atgtcaccat tattgatgac ggtaaactgc gtaaagccat    1080
   taccgcagca tcactgggta atgcaatgga atggttcgat tttggtgttt atggttttgt    1140
   tgcttacgca ttaggtaaag tttttttccc gggggctgac cccagcgtgc agatggttgc    1200
   tgcacttgcc actttctccg ttccctttct gattcgaccg cttggcggac tcttctttgg    1260
   tatgttgggc gataaatatg gtcgccagaa gatcctcgct atcactattg tgattatgtc    1320
   gatcagtacg ttctgtattg gcttaatacc gtcctacgac acgattggta tttgggcacc    1380
   gattctgctg ttgatctgta agatggcaca aggtttctcg gtcggcggtg aatataccgg    1440
   ggcgtcgata tttgttgcgg aatactcccc tgaccgtaaa cgtggcttta tgggcagctg    1500
   gctggacttc ggttctattg ccgggtttgt gctgggtgcg ggcgtggtgg tgttaatttc    1560
   gaccattgtc ggcgaagcga acttcctcga ttggggctgg cgtattccgt tctttatcgc    1620
   tctgccgtta gggattatcg ggctttacct gcgccatgcg ctggaagaga ctccggcgtt    1680
   ccagcagcat gtcgataaac tggaacaggg cgaccgtgaa ggtttgcagg atggcccgaa    1740
   agtctcgttt aaagagattg ccactaaata ctggcgcagc ctgttgacat gtattggtct    1800
   ggtaattgcc accaacgtga cttactacat gttgctgacc tatatgccga gttatttgtc    1860
   gcataacctg cattactccg aagaccacgg ggtgctgatt attatcgcca ttatgatcgg    1920
   tatgctgttt gtccagccgg tgatgggctt gctgagtgac cgttttggcc gtcgtccgtt    1980
   tgtgctactt ggtagtgttg ccctgtttgt gttggcgatc ccggcgttta ttctgattaa    2040
   cagtaacgtc atcggcctga tttttgccgg gttactgatg ctggcggtga tccttaactg    2100
   ctttacgggc gttatggctt ctaccttgcc agcgatgttc ccgacgcata tccgttacag    2160
   cgcgctggcg gcggcattta atatttcggt gctggttgcc ggtctgacgc caacgctggc    2220
   ggcctggctg gtcgaaagct cgcagaatct gatgatgcct gcctattacc tgatggtagt    2280
   ggcggtggtt ggtttaatca ccggcgtaac catgaaagag acggcaaatc gtccgttgaa    2340
   aggtgcgaca ccggcggcgt cagatataca ggaagcgaag gaaattctcg tcgagcatta    2400
   cgataatatc gagcagaaaa tcgatgatat tgaccacgag attgccgatt tgcaggcgaa    2460
   acgtacccgc ctggtgcagc aacatccgcg aattgatgaa taagctgaaa cggatggcct    2520
   gatgtgacgc tgtcttatca ggccaattga actcttaagg ttcacttaat ctctgacgcg    2580
   catactctcc tccaggttaa cggaggagag tgcaatgaaa aaccgtgttt atgaaagttt    2640
   aactaccgtg ttcagcgtgc tggtggtcag cagctttctt tatatctggt ttgccacgta    2700
   ctgatctttc ttcagccgta cccaggcccg cgtgccggaa gtctcttgcc ggttttgcag    2760
   gaaaaactgc ccgtgatgca actgtgtaat gcggctgaca atacttaacc ccagaccaat    2820
   cccgccataa cggctgtcca tacgtacaaa cgctttactc aactccccgc atttactctc    2880
   atcaatacct ggtccttcat cttcaactgc catgaccgct ccgtcatctt cttgcagctt    2940
   aatcataatg ttgctgcctt gcgggctgta acgatgggcg ttttctacca ggtttcgcaa    3000
   taacatccgc agcagggttg catcaccctg aacggtgatg tcggcggcgc tctctggcaa    3060
   tagcagggtt tgctgtcgct ggtcgagcat ggtactgagt tcgtcatacg aggggagaat    3120
   gacatcttcc agcagtttta catgttgata attaccggaa gaaaatgact gtccggcacg    3180
   cgccagttgc agcagctggg agacgctctc catcatctga tcaagccgtg ccactaacgg    3240
   tgctacatca atgtgatgcg ttttcgccag cagttccaga tgcaaacgca cccccgccag    3300
   tggcgttcgc agttcgtgcg cgacgtcagc ggtaaacaac ctttcgttat ccagcgtgct    3360
   ggtcaggcga ctgaccagat cgtttaacgc cgaaaccacc gcttcgattt cgagggtggc    3420
   gctgtgaatg gcaatgggcg ttaagttgtc ggcggtgcgc gcttccagct ctttttgcag    3480
   ctccgccagc gggcgggtga tgc                                            3503